(19) Europäisches Patentamt
European Patent Office
Office européen des brevets

(11) EP 2 808 815 A2

(12) EUROPEAN PATENT APPLICATION

(43) Date of publication:
**03.12.2014 Bulletin 2014/49**

(51) Int Cl.:
***G06F 19/24** (2011.01)* ***G06F 19/20** (2011.01)*

(21) Application number: **14174952.3**

(22) Date of filing: **10.03.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.03.2009 US 158948 P**

(62) Document number(s) of the earlier application(s) in accordance with Art. 76 EPC:
**10751103.2 / 2 406 730**

(71) Applicant: **Agency for Science, Technology And Research**
**Singapore 138632 (SG)**

(72) Inventors:
- **Kuznetsov, Vladimir A.**
  **Singapore 138671 (SG)**
- **Motakis, Efthimios**
  **Singapore 138671 (SG)**
- **Ivshina, Anna V.**
  **Singapore 138671 (SG)**

(74) Representative: **Fleck, Barbara**
**Marks & Clerk LLP**
**62-68 Hills Road**
**Cambridge CB2 1LA (GB)**

Remarks:
This application was filed on 30-06-2014 as a divisional application to the application mentioned under INID code 62.

(54) **Identification of biologically and clinically essential genes and gene pairs, and methods employing the identified genes and gene pairs**

(57) A method of obtaining cut-off expression values should be selected so as to maximise the separation of the respective survival curves of the two groups of patients. Pairs of genes are statistically significant genes are generated by generating a plurality of models, each of which represents a way of partitioning a set of subjects based on the optimal cut-off expression values of the pair of genes. Those gene pairs are identified for which one of the models has a high prognostic significance. Novel survival significant gene sets forming functional modules which could be used to develop specific prognostic and predictive tests are derived.

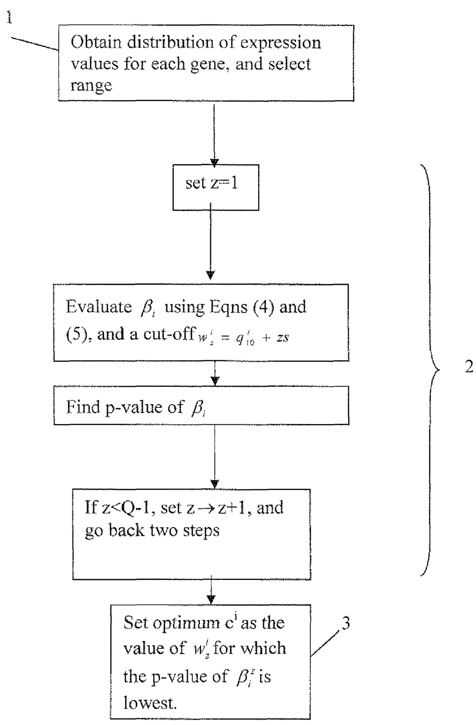


Figure 2

EP 2 808 815 A2

## Description

Related applications

The present application is related to US61/158,948 from which it claims priority, and also to Singapore patent application number 200901682-5, which has the same filing date as US61/158,948.

Field of the invention

The present invention relates to identification of clinically distinct sub-groups of patients and corresponding genes and/or pairs of genes for which the respective gene expression values in a subject and clinical status are statistically significant in relation to a medical condition, for example cancer progression, or more particularly breast cancer patient's survival. The gene expression values may for example be indicative of the susceptibility of the individual subject to the medical condition (in context of time survival or/and disease progression), or the prognosis of a subject who exhibits the medical condition. The invention further relates to methods employing the identified patient survival significant genes and gene pairs.

Background of the invention

Global gene expression profiles of subjects are often used to obtain information about those subjects, such as their susceptibility to certain medical condition, or, in the case of subjects exhibiting medical conditions, their prognosis. For example, having determined that a particular gene is important, the level in which that gene is expressed in a subject can be used to classify the individual into one of a plurality of classes, each class being associated with a different susceptibility or prognosis. The class comparison analysis leads to a better understanding of the disease process by identifying gene expression in primary tumours associated with subject survival outcomes (Kuznetsov *et al.,* 2006).

1. The theory of survival analysis

First we will describe briefly the background theory of survival analysis. We denote by $T$ the patient's *survival time.* $T$ is a continuous non-negative random variable which can take values $t$, $t \in [0, \infty)$. $T$ has density function $f(t)$ and cumulative distribution function $F(t) = P(T \le t)$. $F(t) = \int_0^t f(t')dt'$. We are primarily interested in estimating two quantities:

The survival function:

$$S(t) = P(T > t) = 1 - F(t)$$

The hazard function:

$$h(t) = \frac{f(t)}{S(t)} = \lim_{\Delta t \to 0} \frac{P(t \le T < (t + \Delta t) | T \ge t)}{\Delta t}$$

The survival function expresses the probability of a patient to be alive at time t. It is often presented in the form $S(t) = \exp(-H(t))$, where $H(t) = \int_0^t h(u)du$ denotes the cumulative hazard. The hazard function assesses the instantaneous risk of death at time t, conditional on survival up to that time.

Notice that the hazard function is expressed in terms of the survival function. To this extent, survival distributions and hazard functions can be generated for any distribution defined for $t \in [0,\infty)$. By considering a random variable W, distributed in $(\infty,-\infty)$, we can generate a family of survival distributions by introducing location ($\alpha$) and scale ($\sigma$) changes of the form $\log T = \alpha + \sigma W$.

Alternatively, we can express the relationship of the survival distribution to covariates by means of a parametric model. The parametric model employs a "regressor" variable x. Take for example a model based on the exponential distribution and write: log(h(t)) = $\alpha + \beta x$, or equivalently, $h(t) = \exp(\alpha + \beta x)$.

This is a linear model for the log-hazard, or, equivalently, a multiplicative model for the hazard. The constant $\alpha$ represents the log-baseline hazard (the hazard when the regressor x = 0) and the slope parameter $\beta$ gives the change in hazard rate as x varies. This is an easy example of how survival models can be obtained from simple distributional assumptions. In the next paragraphs we will see more specific examples.

2. Cox proportional hazards model

One of the most popular survival models is the Cox proportional hazards model (Cox, 1972):

$$\log \quad h(t) = \alpha(t) + \beta x \tag{1}$$

where, as before, $t$ is the survival time, $h(t)$ represents the hazard function, $\alpha(t)$ is the baseline hazard, $\beta$ is the slope parameter of the model and x is the regressor. The popularity of this model lies in the fact that it leaves the baseline hazard function $\alpha(t)$ (which we may alternatively designate as log $h_0(t)$) unspecified (no distribution assumed). It can be estimated iteratively by the method of partial likelihood of Cox (1972). The Cox proportional hazards model is semi-parametric because while the baseline hazard can take any form, the covariates enter the model linearly.

Cox (1972) showed that the $\beta$ coefficient can be estimated efficiently by the Cox partial likelihood function. Suppose that for each of a plurality of K subjects (labelled by $k$=1,...,K), we observe at corresponding time $t_k$ a certain nominal (i.e. yes/no) clinical event has occurred (e.g. whether there has been metastasis). This knowledge is denoted $e_k$. For example $e_k$ may be 0 if the event has not occurred by time $t_k$ (e.g. no tumour metastasis at time $t_k$) and 1 if the event has occurred (e.g. tumour metastasis at time $t_k$). Cox (1972) showed that the $\beta$ coefficient can be estimated efficiently by the Cox partial likelihood function, estimated as:

$$L(\beta_i) = \prod_{k=1}^{K} \left\{ \frac{\exp(\beta \; x_k)}{\sum_{j \in R(t_k)} \exp(\beta \; x_j)} \right\}^{e_k} \tag{2}$$

where $R(t_k) = \{j: t_j \geq t_k\}$ is the risk set at time $t_k$. Typically, e is a binary variable taking value 0 = non-occurrence of the event until time $t$ or 1 = occurrence of the event at time t. Later we will discuss a particular case of clinical event we consider in the work, without limiting though our model to this specific case.

The likelihood (2) is minimized by Newton-Raphson optimization method for finding successively better approximations to the zeroes (or roots) of a real-valued function (Press et al., 1992), with a very simple elimination algorithm to invert and solve the simultaneous equations.

3. The goodness-of-split Measure of Survival and Selection of Prognostic Significant Genes

Assume a microarray experiment with i = 1, 2, ..., N genes, whose intensities are measured for k = 1, 2, ..., $K$ breast cancer patients. The log-transformed intensities of gene i and patient $k$ are denoted as $y_{i,k}$. Log-transformation serves for data "Gaussianization" and variance stabilization purposes, although other approaches, such as the log-linear hybrid transformation of Holder et al. (2001), the generalized logarithm transform of Durbin et al. (2002) and the data-driven Haar-Fisz transform of Motakis et al. (2006), have also been considered in the literature.

Associated with each patient k are a disease free survival time $t_k$ (in this work DFS time), a nominal clinical event $e_k$ taking values 0 in the absence of an event until DFS time $t_k$ or 1 in the presence of the event at DFS time $t_k$ (DFS event) and a discrete gradual characteristic (histologic grade). Note that in this particular work the events correspond to the presence or absence of tumor metastasis for each of the k patients. Other types of events and/or survival times are possible to be analyzed by the model we will discuss below.

Additional information, which is not utilized in this work, includes patients' age (continuous variable ranging from 28 to 93 years old), tumor size (in milimeters), breast cancer subtype (Basal, ERBB2, Luminal A, Luminal B, No subtype, normal-like), patients' ER status (ER+ and ER-) and distant metastasis (a binary variable indicating the presence or absence of distant metastasis).

Assuming, without loss of generality, that the K clinical outcomes are negatively correlated with the vector of expression signal intensity $y_i$ of gene i, patient $k$ can be assigned to the high-risk or the low-risk group according to:

$$x_k^i = \begin{cases} 1 \ (high-risk), if\ y_{i,k} > c^i \\ 0 \ (low-risk), if\ y_{i,k} \le c^i \end{cases} \quad (3)$$

where $c^i$ denotes the predefined cut-off of the *i*th gene's intensity level. In the case of positive correlation between the K clinical outcomes and $y_i$, patient *k* is simply assigned to one of the two groups according to:

$$x_k^i = \begin{cases} 1 \ (high-risk), if\ y_{i,k} \le c^i \\ 0 \ (low-risk), if\ y_{i,k} > c^i \end{cases}$$

After specifying $x_k^i$, the DFS times and events are subsequently fitted to the patients' groups by the Cox proportional hazard regression model (Cox, 1972):

$$\log h_k^i(t_k | x_k^i, \beta_i) = \alpha_i(t_k) + \beta_i x_k^i \quad (4)$$

where, as before, $h^i_k$ is the hazard function and $\alpha_i(t_k) = \log h^i_0(t_k)$ represents the unspecified log-baseline hazard function for gene *i*; $\beta_i$ is the ith element of the vector $\vec{\beta}$ of the model regression parameters to be estimated; and $t_k$ is the patients' survival time. To assess the ability of each gene to discriminate the patients into two distinct genetic classes, the Wald statistic (W) (Cox and Oakes, 1984) of the $\beta_i$ coefficient of model (4) is estimated by minimizing the univariate Cox partial likelihood function for each gene *i*:

$$L(\beta_i) = \prod_{k=1}^{K} \left\{ \frac{\exp(\beta_i^T x_k^i)}{\sum_{j \in R(t_k)} \exp(\beta_i^T x_j^i)} \right\}^{e_k} \quad (5)$$

where $R(t_k) = \{j: t_j \ge t_k\}$ is the risk set at time $t_k$ and $e_k$ is the clinical event at time $t_k$. The actual fitting of model (4) is conducted by the survival package in R (http://cran.r-project.org/web/packages/survival/index.html). The genes with the largest $\beta_i$ Wald statistics ($W_i$'s) or the lowest $\beta_i$ Wald P values are assumed to have better group discrimination ability and thus called *highly survival significant genes.* These genes are selected for further confirmatory analysis or for inclusion in a prospective gene signature set. Note that given $\beta_i$, one derives the Wald statistic, W, as:

$$W = \frac{\beta_i^{\,2}}{var(\beta_i)}$$

where $var(\beta_i) = \frac{1}{I(MLE)}$ and I denotes the Fisher information matrix of the $\beta_i$ parameter. Estimating the Wald P value, simply requires evaluation of the probability:

$$p - value = \Pr\left(\frac{\beta_i^{\,2}}{var(\beta_i)} > \chi_v^2\right) \quad (5)$$

where $\chi^2_{v}$ denotes the chi-square distribution with *v* degrees of freedom. Typically, *v* is the number of parameters of the Cox proportional hazards model and in our case *v* = 1. Expression (5) can be derived from the proper statistical tables of the chi-square distribution.

From Eqn. (3) notice that the selection of prognostic significant genes relies on the predefined cut-off value $c^i$ that separates the low-risk from the high-risk patients. The simplest cut-off basis is the mean of the individual gene expression values within samples (Kuznetsov, 2006), although other choices (e.g. median, trimmed mean, etc) could be also applied. Two problems, associated with such cut-offs, are: 1) they are suboptimal cut-off values that often provide low classification accuracy or even miss existing groups; 2) the search for prognostic significance is carried out for each gene independently, thus ignoring the significance and the impact of genes' co-expression on the patient' survival.

Summary of the invention

In a first aspect, the present invention proposes in general terms that a cut-off expression value should be selected so as to maximise the separation of the respective survival curves of the two groups of patients. From another point of view, this means that the cut-off expression value is selected such that the partition of subjects which it implies is of maximal statistical significance. This overcomes a possible problem in the known method described above: that if the cut-off expression values are not well-chosen they may provide low classification accuracy even for genes which are very statistically significant for certain ranges of expression value.

A specific expression of the first aspect of the invention is a computerized method for optimising, for each gene i of a set of N genes, a corresponding cut-off expression value $c^i$.
for partitioning subjects according to the expression level of the corresponding gene,
the method employing medical data which, for each subject k of a set *of* $K^*$ subjects suffering from the medical condition, indicates (i) the survival time of subject k, and (ii) for each gene i, a corresponding gene expression value $y_{i,k}$ of subject k;
the method comprising, for each gene i,

(i) for each of a plurality of a trial values of $c^i$:

(a) identifying a subset of the $K^*$ subjects such that $y_{i,k}$ is above the trial value of $c^i$;
(b) computationally fitting the corresponding survival times of the subjects to the Cox proportional hazard regression model, said fitting using, for subjects within the subset, a regression parameter $\beta_i$ corresponding to the gene *i*; and
(c) obtaining from the regression parameter $\beta_i$, a significance value indicative of prognostic significance of the gene;

(ii) identifying the trial cut-off expression value for which the corresponding significance value indicates the highest prognostic significance for the gene *i*.

The cut-off expression value $y_{i,k}$ here may be a logarithm of the measured expression intensity of gene *i* in patient *k* (as in the background section above, and in following description of specific embodiments). However, the expression value $y_{i,k}$ may alternatively be any other transformation of the measured expression insensity, or indeed the raw intensity itself.

In a second aspect, the invention proposes in general terms that pairs of genes are selected. For each gene pair, we generate a plurality of models, each of which represents a way of partitioning a set of subjects based on the expression values of the pair of genes. We then identify those gene pairs for which one of the models has a high prognostic significance. This overcomes a problem of the known method described above that the search for prognostic significance is carried out for each gene independently, thus neglecting any significance of genes' co-expression on patient survival.

A specific expression of the second aspect of the invention is a computerized method for identifying one or more pairs of genes, selected from a set of *N* genes, which are statistically associated with prognosis of a potentially-fatal medical condition,
the method employing medical data which, for each subject k of a set of $K^*$ subjects suffering from the medical condition, indicates (i) the survival time of subject k, and (ii) for each gene i, a corresponding gene expression value $y_{i,k}$ of subject *k*;
the method comprising:

(i) for each of the *N* genes obtaining a corresponding cut-off expression value;

(ii) forming a plurality of pairs of the identified genes (i, *j* with $i \neq j$), and for each pair of genes:

(1) forming a plurality of models $m_{i,j}$, each model $m_{i,j}$ including a comparison of the corresponding cut-off expression values $c^i$ and $c^j$ of the respective levels of expression $y_{i,k}, y_{j,k}$ of the genes $i$ ,$j$ in the set of $K^*$ subjects;

(2) for each model determining, a respective subset of the $K^*$ subjects using the model;

(3) computationally fitting the corresponding survival times of the subjects to the Cox proportional hazard regression model, said fitting using, for subjects within each of the subsets, a corresponding regression parameter $\beta_{ij}^{m}$ corresponding to the model $m_{i,j}$; and

(4) obtaining from the regression parameters $\beta_{ij}^{m}$, a significance value indicative of prognostic significance of the model; and

(iii) identifying one or more of said pairs of genes $i,j$ for which the corresponding significance values for one of the models have the highest prognostic significance.

The first and second aspects of the invention may be used in combination. That is, cut-off expression values for individual genes derived according to the first aspect of the invention may be employed in a method according to the second aspect of the invention.

Note that the "expression values" referred to in the specific expressions of the invention may be the direct outputs of expression value measurements, but more preferably are the logarithms (e.g. natural logarithms) of such measurements, and optionally may have been subject to a normalisation operation.

In either of the above aspects of the invention, the medical data preferably includes nominal data which indicates for each patient, whether one or more clinical events have occurred. For example, the nominal data may indicate whether tumour metastasis had occurred by the survival time. The significance value may be calculated using a formula which incorporates the nominal data. Alternatively or additionally, it can be used to select a subset of the patients, such that the clinical data for that subset of patients is used in the method.

In either of the above aspects of the invention, the survival time may be an actual survival time (i.e. a time taken to die) or a time spent in a certain state associated with the medical condition, e.g. a time until metastasis of a cancer occurs.

Furthermore, in either of the above aspects of the invention the $K^*$ subjects may be a subset of a larger dataset of $K$ ($K > K^*$) subjects. For example, the data for $K^*$ subjects can be used as training data, and the rest used for validation.

Alternatively, a plurality of subsets of the $K$ subjects can be defined, and the method defined above is carried out independently for each of the subsets. Each of these subsets of the $K$ subjects is a respective "cohort" of the subjects; if the cohorts do not overlap, they are independent training datasets. Note that each time the method is performed for a certain cohort, $K^*$ denotes the number of subjects in that cohort, which may be different from the number of subjects in other of the cohorts. After this, there is a step of discovering which pairs of genes were found to be significant for all the cohorts.

Once one or more genes, or pairs of significant genes, are identified by a method according to either of the above aspects of the invention, they can be used to obtain useful information in relation to a certain subject (typically not one of the cohort(s) of subjects) using a statistical model which takes as an input the ratio(s) of the expression values of the corresponding identified pair(s) of genes. The information may for example be susceptibility to the medical condition, the or prognosis (e.g. relating to recurrence or death) of a subject suffering from the condition.

In a third aspect the invention proposes a kit, such as a microarray, for detecting the expression level of a set of genes, the set having no more than 1000 members, or no more than 100 members, or no more than 20 members, and including

(a) at least one of BRRN1; FLJ11029; C6orf173; STK6; MELK; and/or
(b) at least one of the pairs: (i) SPAG5-ERCC6L, (ii) CENPE-CCNE2, (iii) CDCA8-CLDN5, (iv) CCNA2-PTPRT, (v) Megalin (LRP2) and integrin alpha 7 (ITGA7), (vi) NUDT1 and NMU genes, and (vii) HN1 and CACNA1D.

The invention may be expressed, as above, in terms of a method implemented using a computer, or alternatively as a computer system programmed to implement the method, or alternatively as a computer program product (e.g. embodied in a tangible storage medium) including program instructions which are operable by the computer to perform the method. The computer system may in principle be any general computer, such as a personal computer, although in practice it is more likely typically to be a workstation or a mainframe supercomputer.

Brief description of the figures

Figure 1 shows a histogram of Disease Free Survival (DFS) times of (A) Stockholm and (B) Uppsala cohort for subjects with tumour metastasis (Event = 1) and without tumour metastasis (Event = 0). The dotted lines indicate the DFS threshold values.

Figure 2 is a flow diagram of a first method according to the invention.

Figure 3 shows a plot of log p-values against cut-off expression value levels for the LRP2 and ITGA7 prognostic genes in (a) Stockholm cohort and (b) Uppsala cohort. The dashed line indicates the log (p-value) corresponding to the Data driven grouping (DDg) cut-off expression value obtained by the method of Fig. 2. This cut-off expression value is marked by a cross on the dashed line. The dotted line indicates the log (p-value) corresponding to the Mean Based grouping (MBg) value, which is marked by a cross on the dotted line.

Figure 4 illustrates the four possible ways in which the expression values of a pair of genes i *and j* can be realized in relation to respective cut-off expression values $c^i$ and $c^i$.

Figure 5 is a flow diagram of a second method according to the invention.

Figure 6, which is composed of Fig. 6A to 6G, shows experimental data comparing a known method, and the methods of Figs. 1 and 4" using clinical data from the for two gene pairs for the "Stockholm" cohort of patients.

Figure 7, which is composed of Fig. 7A to 7E, shows experimental data for comparing the known method (Fig. 7B and 7D) and the method of Fig. 4 (Fig. 7A and 7C), and numerical data (Fig. 7E), for the same gene pair but using the "Uppsala" cohort of patients.

Figure 8 shows the Kaplan-Meier plot for survival of the Uppsala patients in groups of grades 1 and 1-like versus grades 3 and 3-like, based on a 5 gene signature.

Definitions

"Array" or "microarray," as used herein, comprises a surface with an array, preferably an ordered array, of putative binding (e.g., by hybridization) sites for a sample which often has undetermined characteristics. An array can provide a medium for matching known and unknown nucleic acid molecules based on base-pairing rules and automating the process of identifying the unknowns. An array experiment can make use of common assay systems such as microplates or standard blotting membranes, and can be worked manually, or make use of robotics to deposit the sample. The array can be a macro-array (containing nucleic acid spots of about 250 microns or larger) or a micro-array (typically containing nucleic acid spots of less than about 250 microns).

The term "cut-off expression value" represented by c (followed with the proper superscript; e.g. $c^i$) refers to a value of the expression level of a particular nucleic acid molecule (or gene) in a subject. The cut-off expression value is used to partition the subjects into classes, according to whether the expression level of the corresponding gene is below or above the cut-off expression value. Note that it makes no difference whether all subjects for which the expression value is actually equal to the cut-off value are classified as being in one class or alternatively in the other.

The term "gene" refers to a nucleic acid molecule that encodes, and/or expresses in a detectable manner, a discrete product, whether RNA or protein. It is appreciated that more than one nucleic acid molecule may be capable of encoding a discrete product. The term includes alleles and polymorphisms of a gene that encodes the same product or an analog thereof.

There are various methods for detecting gene expression levels. Examples include Reverse-Transcription PCR, RNAse protection, Northern hybridisation, Western hybridisation, Real-Time PCR and microarray analysis. The gene expression level may be determined at the transcript level, or at the protein level, or both. The nucleic acid molecule or probe may be immobilized on a support, for example, on an array or a microarray. The detection may be manual or automated. Standard molecular biology techniques known in the art and not specifically described may be employed as described in Sambrook and Russel, Molecular Cloning: A Laboratory Manual, Cold Springs Harbor Laboratory, New York (2001).

Gene expression may be determined from sample(s) isolated from the subject(s).

Detailed description of the embodiments

Embodiments of the methods will now be explained with reference to the figures, and experimental results are presented which were generated using two cohorts of subjects: the Uppsala and Stockholm cohorts.

1. Subjects and Tumour Specimens used in experiments

The clinical characteristics of the subjects and the tumour samples of Uppsala and Stockholm cohorts have been summarised in Ivshina *et al.,* 2006. The Stockholm cohort comprised $K_s$ = 159 subjects with breast cancer, operated on in Karolinska Hospital from January 1, 1994, through December 31, 1996, and identified in the Stockholm-Gotland breast Cancer registry. The Uppsala cohort involved $K_u$ = 251 subjects representing approximately 60% of all breast cancers resections in Uppsala County, Sweden, from January 1, 1987, to December 31, 1989. Information on the subjects' disease free survival (DFS) times/events and the expression patterns of approximately 30000 gene transcripts (representing $N$ = 44928 probe sets on Affymetrix U133A and U133b arrays) in 315 primary breast tumours were obtained from NCBI Gene Expression Omnibus (GEO) (Stockholm data set label is GSE4922; Uppsala data set label is GSE1456). The microarray intensities were calibrated (RMA) and the probe set signal intensities were log-transformed and scaled by adjusting the mean signal to a target value of log500 (Ivshina *et al.,* 2006). In this study, Affymetrix U133A and 133b probesets (232 gene signatures) was used to provide classification of the low- and high-aggressive cancer sub-types described in Ivshina *et al.,* 2006. For each of these patients, there was date specifiying the disease free survival time (DFS) of an event (tumor metastasis). and the actual occurrence of this event (a binary variable taking the values 1 = occurrence of the event, 0 = no occurrence of the event).

2 Training data

Figure 1 shows the distribution of DFS survival time (below, we refer to the DFS survival time for the k-th subject as $t_k$) for the Stockholm cohort (Fig. 1A) and the Uppasla cohort (Fig. 1 B). Each of Fig. 1A and Fig. 1B includes a separate histogram for each of two categories of patients: those for whom tumour metastasis had occurred (this possibility is referred to in Figure 1 as "Event = 1", and below we refer to this as $e_k$=1) and those for whom it had not (this possibility is referred to in Figure 1 as "Event = 0", and below we refer to this as $e_k$=0).

As may be seen in Figure 1, most of the patients are "typical responders" by which we mean that those for whom Event=1 have short survival times, and those for whom Event = 0 have long survival times. In other words we noticed that tumor metastasis typically occurs at a short time (in years) after the beginning of the experiment, whereas the frequency of the occurrence decreases as time increases. To this extent, Figure 1 shows that our data consists of two different distribution of survival times (one for patients without tumor metastasis and one with patients with metastasis). The data that do not agree with this observation are considered as outliers or data from non-typical patients.

The present embodiments used only the "typical" data as training data. In other words, there was a pre-processing of the data to identify only typical patients: data from subjects who satisfy the above Event and $t_k$ relationship. Then we apply our methods on the typical (or training) data only (data from responders that satisfy the above Event and $t_k$ relationship), estimate a cutoff value for each gene by (1) and use these estimates to the whole set of patients to infer about prognostic significance by (2).

Based on visual inspection of Figure 1, the inventors considered that the part of the Stockholm cohort which should be used as training data is the data from "typical" subjects with $t_k$> 5 years and $e_k$=0, or $t_k \leq$ 5 years and $e_k$=1. The 5 year cut-off is shown by the dashed line in Figure 1A. This resulted in 148 Stockholm training set subjects. Following the same procedure for the Uppsala data, the threshold was set at 8 years (see the dashed line in Figure 1B), which resulted in 212 Uppsala training set subjects.

3 Nomenclature

The terminology explained in the background section of this document is followed in the following explanation of the embodiments and some comparative examples. In these embodiments and comparative examples, the number of patients in the training set is denoted $K^*$ which is less than $K$, the total number of patients about whom data existed. The subjects of the training set are labelled $k$=1,....,$K^*$. The DFS survival time for the $k$-th subject is referred to as $t_k$, and whether or not tumour metastatis has occurred is referred to as $e_k$. Each of the embodiments, and the comparative example, involve fitting a set of survival times to an equation such as Eqn. (4). The fitting to Eqn. (4) was conducted by the survival package which can be found at the following website: http://cran.r-project.org/web/packages/survival/index.html. See also the references Cox, D.R and Snell, E. J (1968) and Cox, D.R. and Oakes, D (1984). This made it possible to find a Wald statistic for each gene/gene-pair in the matter explained in the background section of this document.

4. Finding cut-off values and identifying single genes: A first embodiment and a comparative example

We now discuss estimation of a cut-off expression value for each gene. This is done first using the prior art method discussed above (Mean based grouping - "MBg") and using a embodiment of the invention illustrated in Fig. 1 (Data driven grouping-"DDg").

4.1 Comparative Example 1: Mean Based Grouping (MBg)

For each gene i of the training set, the respective mean $\mu_i$ of the values $y_{i,k}$ of the subjects in the training set was found using the $K^*$ training set patients. Note that $K^* < K$ and that in Stockholm $K_s = 159$ and $K_{s^*} = 148$ while in Uppsala $K_u = 251$ and $K_{u^*} = 212$ (for simplicity we drop the *s* and *u* subscripts in the following paragraphs). The subjects of the training set were then grouped according to whether their values of $y_{i,k}$ were above or below a cut-off $c^i = \mu_i$. Equation (3) was used to generate a set of values $\{x_k^i\}$, and from this a set of corresponding values $\beta_i$ was generated by fitting Equation (4). The prognostic significance of gene *i* was evaluated by reporting the p-value of the estimated $\beta_i$. The genes with significantly small p-values ($p < 0.05$ or $p < 0.01$) were selected.

Schematically, the steps we follow are:

1. For gene i = 1, estimate the mean expression signal $\mu_i$ from the training set of $K^*$ patients and set the grouping cutoff $c^i = \mu_i$. Alternatively, one could estimate the median or the trimmed-mean expression signal from the set of $K^*$ patients and set accordingly the grouping cutoff equal to these values (these are the median- and trimmed-mean based grouping methods that do not discussed further here because they lead to similar results to the ones of mean-based method).
2. Group the $k = 1, 2, ..., K$ patients according to

$$x_k^i = \begin{cases} 1 \ (\boldsymbol{high} - risk), if\ y_{i,k} > c^i \\ 0 \ (low - risk), if\ y_{i,k} \le c^i \end{cases}$$

or

$$x_k^i = \begin{cases} 1 \ (\boldsymbol{high} - risk), if\ y_{i,k} \le c^i \\ 0 \ (low - risk), if\ y_{i,k} > c^i \end{cases}$$

with $c^i = \mu_i$.

3. Evaluate the prognostic significance of gene i by reporting the P value of the estimated $\beta_i$ from model

$$\log \boldsymbol{h}_k^i(t_k | x_k^i, \beta_i) = \alpha_i(t_k) + \beta_i x_k^i$$

4. Iterate for all genes i = 2, ..., *N.*
5. Select as prognostic significant the genes with significantly small P values ($p <$ *alpha* = 1%).

4.2 First embodiment: Data-Driven Grouping (DDg)

The first embodiment is explained with reference to Fig. 2. For each gene i, the distribution of the $K^*$ signal intensity values $y_{i,k}$ was computed, and 10th quantile $(q_{10}^i)$ and the 90th quantile $(q_{90}^i)$ were derived (step 1). Within a range $(q_{10}^i, q_{90}^i)$, a search was performed for the value that most successfully discriminates the two unknown genetic classes, which corresponds to the minimum $\beta_i^z$ p-value (here z = 1, ..., Q). In step 2 of Fig. 2, the following sub-steps were performed:

1. Form the candidate cut-offs vector of dimension 1 x Q, $\vec{w}^i = y_{i,k}^*$, where $y_{i,k}^*$ is the log-transformed intensities within $(q_{10}^i, q_{90}^i)$ and Q is the number of elements in $\underline{w}^i$. Each element of the $\vec{w}^i$ is a trial cut-off value. For $i$ = 1 and $c^i = \vec{w}_z^i$, z = 1, ..., Q use

$$x_k^i = \begin{cases} 1\ (high-risk) & if \quad y_{i,k} > c^i \\ 0\ (low-risk) & if \quad y_{i,k} \leq c^i \end{cases}$$

or

$$x_k^i = \begin{cases} 1\ (high-risk) & if \quad y_{i,k} \leq c^i \\ 0\ (low-risk) & if \quad y_{i,k} > c^i \end{cases}$$

to separate the $K^*$ subjects with $c^i$.

For z=1 (the first element in $\vec{w}^i$) evaluate the prognostic significance of gene $i$ given cut-off $\vec{w}_z^i$ by estimating the $\beta_i^z$ from $\log\ h_k^i(t_k|x_k^i, \beta_i^z) = \alpha_i(t_k) + \beta_i^z x_k^i$; and

$$L(\beta_i^z) = \prod_{k=1}^{K} \left\{ \frac{\exp(\beta_i^z x_k^i)}{\sum_{j\in R(t_k)} \exp(\beta_i^z x_k^i)} \right\}^{e_k}.$$

3. Iterate for z = 2, ..., Q to estimate the Q p-values (corresponding to Q distinct cut-off expression value levels) for each i. The "optimal" cut-off expression value $c^i$ for each i is the taken as the one with the minimum $\beta_i^z$ p-value, provided that the sample size of each group is sufficiently large (formally above 30) and Cox proportional hazards model is plausible.

1. Using this cut-off, evaluate the prognostic significance of gene i by estimating the $\beta_i$ from Eqn. (4) and Eqn (5) for the full set of patients.

2. Iterate steps 1 to 4 for i = 2, ..., $N$.

In step 3, the "optimal" cut-off expression value for each i is the taken as the one with the minimum $\beta_i^z$ p-value, provided that the sample size of each group is sufficiently large (formally above 30) and model defined by Eqn. (4) is plausible. Note that here $\beta_i$ is substituted by $\beta_i^z$ indicating that the search was for the $\beta_i$ that leads to the best cut-off expression value.

In order to validate the significance of the findings (in terms of the estimated p-values), the Stockholm and Uppsala samples were bootstrapped and the 99% confidence intervals for the $\beta_i$ coefficients of Eqn. (4) were estimated. We use the non-parametric residuals bootstrap for the proportional hazards model of Loughin (1995) using the *boot* package in R. Specifically, the algorithm works as follows sequentially for each gene $i$:

1. Estimate $\beta_i$ of model:

$$\log h_k^i\left(t_k \middle| x_k^i, \beta_i\right) = \alpha_i(t_k) + \beta_i x_k^i$$

by maximizing the likelihood:

$$L(\beta_i) = \prod_{k=1}^{K} \left\{ \frac{\exp(\beta_i^T x_k^i)}{\sum_{j \in R(t_k)} \exp\left(\beta_i^T x_j^i\right)} \right\}^{e_k}$$

2. Calculate the independent and identically (Uniform in [0,1]) distributed generalized residuals, calculated in Cox and Snell (1968) and Loughin (1995) by the "probability scale data"

$$u_k = \left[1 - F_0(t)\right]^{\exp\left(\beta^T x_k^i\right)}, \quad k = 1, 2, \ldots, K,$$

where $F_0(t) = P\left(T \leq t \middle| \exp\left(\beta_i^T x^i\right) = 1\right)$ denotes the baseline failure time distribution. Typically, $\hat{F}_\alpha(t)$ is a step function with jumps at the observed failure times (estimated automatically in the *survival* package), which does not affect the Uniformity of the generalized residuals (Loughin, 1995)

3. Consider the pairs $\{(u_1,e_1),\ldots,(u_K,e_K)\}$ and resample with replacement $B$ pairs of observations ($B$ bootstrap samples) $\left\{\left(u_1^{(b)}, e_1^{(b)}\right), \ldots, \left(u_K^{(b)}, e_K^{(b)}\right)\right\}$,, b = 1, ..., B (a typical bootstrap step)

4. Calculate the probability scale survival times

$$t_k^{(b)} = 1 - \left[u_k^{(b)}\right]^{1/\exp(\beta_i^T x_k^i)}$$

$$t_k^{(b)} = 1 - \left[u_k^{(b)}\right]^{1/\exp\left(\beta_i^T x_k^i\right)}$$

and estimate the bootstrap coefficients $\beta_i^{(1)}$, $\beta_i^{(2)}$, ,..., $\beta_i^{(B)}$ by numerically maximizing the partial likelihood:

$$L^{(b)}(\beta_i) = \prod_{k=1}^{K} \left\{ \frac{exp\left(\beta_i^T x_k^i\right)}{\sum_{j \in R^{(b)}(t_k)} exp\left(\beta_i^T x_j^i\right)} \right\}^{e_i^{(b)}} \qquad b = 1, \ldots, B$$

Based on these coefficients, we estimate and report the Bias-Corrected accelerated (BCa) bootstrap confidence intervals for each $\beta_i$ coefficient that correct the simple quantile intervals of $\beta_i$ for bias and skewness in their distribution (Efron and Tibshirani, 1994). A detailed discussion (theory and applications) on BCa intervals is given in Efron and Tibshirani (1994). Here the 99% BCa were estimated by the *boot* package in R. Bootstrap test p-values based on quantile method gave similar results using the Wald test.

4.3 Identification of genes

The gene expression profiles were correlated with clinical outcome (disease free survival time; DFS) in the two cohorts with the intention of identifying specific genes that predict survival. It was found that a large fraction of the genes had some correlation with survival, and could be used to predict survival.

Figure 3 shows the results of the comparative example and first embodiment for the LRP2 and ITGA7 prognostic genes in (a) Stockholm cohort and (b) Uppsala cohort. In each of the four graphs, the dashed line indicates the log (p-value) corresponding to the Data driven grouping (DDg) cut-off expression value obtained by the method of Fig. 2. This cut-off expression value is marked by a cross on the dashed line. The dotted line in each of the graphs indicates the log (p-value) corresponding to a MBg cut-off found by method of section 4.1, and this is cut-off is marked by a cross on the dotted line. Figure 3 suggests that the data-driven grouping improves prediction of subjects' survival compared to the mean based approach.

4.4 Survival Significance of Genes of Genetic Grade Signature for the Stockholm Cohort

The mean-based grouping and the data-driven grouping with the estimated cut-off expression value were repeated for the full set of 159 subjects of the Stockholm Cohort to estimate the Wald p-values for each of the 264 probe sets (representing 232-gene genetic grade signature (Ivshina *et al.,* 2006). At 1% significance level, MBg identified 151 probesets (148 prognostic gene signatures), while DDg identified 195 probesets (192 prognostic gene signatures). 82 of the 100 top-level survival related probesets of the two approaches are common. For the genes with p-values lower than 0.1%, the methods are highly reproducible. In this case, ~99.0% of the MBg probesets was also present in the DDg list.

Bias-Corrected accelerated confidence intervals (BCa CI's) were used to confirm the Wald statistic estimates. By estimating the 99% BCa CI's, 118 and 145 probesets were predicted by MBg and DDg methods, respectively, as survival significant probesets. As a comparison between Wald statistic and BCa, 52 probesets (Wald-only positives) with significant Wald p-values (at *alpha* = 1%) were not significant by BCa bootstrapping; while 2 genes (Wald-only negatives) for which the p-values were not significant were significant with BCa bootstrapping for the DDg selected group set. For the MBg selected probesets, the 99% BCa CI's found 118 significant probesets with 38 Wald-only positives and 2 Wald-only negatives.

5.1 Identifying synergetic Gene pairs: a second embodiment

A second embodiment of the invention is explained with reference to Figures 4 and 5. The approach resembles the idea of Statistically Weighted Syndromes algorithm (Kuznetsov *et al.,* 2006). For a given gene pair $i, j, i \neq j$, and respective individual cut-off expression values $c^i$ and $c^j$, we define seven "models", each of which is a possible way in which the expression levels of the two genes might be significant. Then we test the data to see if any of the seven models are in fact statistically significant. The models are defined using the concept of Figure 4, which shows how a 2-D area having $y_{i,k}$, $y_{j,k}$ as axes. The 2-D area is divided into four regions A, B, C and D, defined as follows:

$$\begin{aligned} &\text{A: } y_{i,k} < c^i \text{ and } y_{j,k} < c^j \\ &\text{B: } y_{i,k} \geq c^i \text{ and } y_{j,k} < c^j \\ &\text{C: } y_{i,k} < c^i \text{ and } y_{j,k} \geq c^j \\ &\text{D: } y_{i,k} \geq c^i \text{ and } y_{j,k} \geq c^j \end{aligned} \qquad (6)$$

Each of the seven models is then defined as a respective selection from among the four regions:

Model 1 is that a subject's prognosis is correlated with whether the subject's patient's expression levels are within regions A or D, rather than B or C.

Model 2 is that a subject's prognosis is correlated with whether the subject's patient's expression levels are within regions A, B or C, rather than D.

Model 3 is that a subject's prognosis is correlated with whether the subject's patient's expression levels are within regions A, C or D, rather than B.

Model 4 is that a subject's prognosis is correlated with whether the subject's patient's expression levels are within regions B, C or D, rather than A.

Model 5 is that a subject's prognosis is correlated with whether the subject's patient's expression levels are within regions A, B or D, rather than C.

Model 6 is that a subject's prognosis is correlated with whether the subject's patient's expression levels are within regions A or C, rather than B or D.

Model 7 is that a subject's prognosis is correlated with whether the subject's patient's expression levels are within regions A or B, rather than C or D.

Note that model 6 is equivalent to asking only whether the subject's expression level of gene 1 is below of above $c^1$ (i.e. it assumes that the expression value of gene 2 is not important). Model 7 is equivalent to asking only whether the subject's expression for gene 2 is above or below $c^2$ (it assumes that the expression value of gene 1 is not important). Thus, models 1-5 are referred to as "synergetic" (1 - 5), and the models 6 and 7 as "independent".

The algorithm of the second embodiment evaluates the significance of all possible gene pairs *i*, *j* as follows (see Figure 5):

1. Set *i*=1 and *j*=2 (step 11).

2. For each of the 7 models, obtain the respective sub-set of the subjects whose expression values for genes i and *j* obey the respective set of the conditions (6). For example, for model 1, we obtain the subset of the *K** subjects whose expression values obey conditions A, B or C. This is the subset of the subjects for which $y_{i,k} < c^i$ and/or $y_{j,k} < c^j$ (i.e. the set of subjects which for which condition D is not obeyed). Let us define a parameter $x^m_{i,j,k}$, where $x^m_{i,j,k} = 1$ if and only if, for genes i and *j,* and model *m* (*m*=1,...7), the expression levels $y_{i,k}$ and $y_{j,k}$ meet the conditions of model m.

3. Fit the survival values to:

$$\log h^{i,j}{}_k(t_k \mid x^m_{i,j,k}, \beta^m_{i,j}) = \alpha_{i,j}(t_k) + \beta^m_{i,j} \cdot x^m_{i,j,k}, \qquad (7)$$

4. Estimate the seven Wald p-values $\beta^m_{i,j}$. Provided that the respective groups sample sizes are sufficiently large and the assumptions of the Cox regression model are satisfied, the model is the one with the smallest $\beta^m_{i,j}$ p-value.

5. Iterate steps 12 to 14 for all pairs of genes.

The algorithm above was then applied to the data set in order to examine whether gene pairing could improve the prognostic outcome for certain survival significant genes. All the possible 34716 probeset pairs (0.5 x 264 x 263) of the study were considered. Figure 6 presents the synergetic grouping results for two selected gene pairs: LRP2 - ITGA7 and CCNA2 - PTPRT. These pairs had been chosen based on two criteria; *Criterion 1:* their synergy (as indicated by the p-values) was highly significant; *Criterion 2:* criterion 1 was satisfied in both cohorts.

Fig. 6A and 6C respectively show clinical data for the LRP2-ITAG7 combination, the crosses representing subjects with DFS time < 3 (indicator of "high-risk") and the circles representing subjects with DFS time > 3 (indicator of "low-risk"). The horizontal and vertical lines within the graph indicate the respective cut-off expression values for LRP2 and ITAG7 selected by MBg (Fig. 6A) and DDg (Fig. 6B). Thus, for example, for the LRP2 gene, MMg gives a cut-off expression value of about 6.61 with a p-value of $9.8 \times 10^{-4}$, whereas DDg (the method of Fig. 1) gives a cut-off expression value of about 6.38. The ITGA7 cut-off expression value was 7.7 with a p-value of $1.9 \times 10^{-3}$ (7.6 in MBg). Similarly for the other pair, the CCNA2 DDg cut-off expression value was 5.95 with a p-value of $1.8 \times 10^{-5}$ (6.04 in MBg) and the PTPRT cut-off expression value was 7.25 with a p-value of $1.4 \times 10^{-5}$ (7.52 in MBg).

As shown in the table of Fig. 6G, the method of Fig. 5, when practiced for the gene pair LRP2-ITAG7 using the cut-off expression values for LRP2 and ITGA7 obtained by MBg, selected model 4, and then produced a p-value for the synergy of $1.6 \times 10^{-4}$. By contrast, when the method of Fig. 4 was practiced with the optimized cut-off expression values for LRP2 and ITGA7 from the method of Fig. 1 (e.g. for gene LRP2, the cut-off expression value of 6.38), the method still selected model 4, but the consequent p-value was $2.5 \times 10^{-6}$. Whichever of the sets of cut-off expression values is used, the subjects in A region are identified as "low-risk" subjects, whereas the B, C and D subjects ("high-risk" subjects) while for the CCNA2-PTPRT case the A, C and D ("high-risk") subjects were separated from the B subjects ("low-risk").

For the LRP2-ITGA7 case, the subjects at A region (identified as "low-risk" subjects) were separated from the B, C and D subjects ("high-risk" subjects) while for the CCNA2-PTPRT case the A, C and D ("high-risk") subjects were separated from the B subjects ("low-risk").

Fig. 6B and 6D are corresponding Kaplan-Meier curves (Kaplan and Meier, 1958). The solid lines correspond to "low-risk" subjects and the dotted lines to "high-risk" subjects. Fig. 6B shows the two Kaplan-Meier curves for each of (i) for LRP2 using the cut-offs from MBg, (ii) for ITGA7 using the cut-offs from MBg, and (iii) for model 4 using Eqn. (7) and the two cut-off values from MBg.. 6D shows the two Kaplan-Meier curves for each of (i) for LRP2 using the cut-offs from DDg, (ii) for ITGA7 using the cut-offs from DDg, and (iii) for model 4 using Eqn. (7) and the two cut-off values from DDg.

For the gene pair CCNA2-PTPRT, the corresponding results for performing the method of Fig. 4 using cut-off expression values from MBg was $3.9\times10^{-5}$, and using the optimized cut-off expression values from the method of Fig. 1 (i.e. DDg) was $4.0\times10^{-8}$. In both cases, model 3 was selected as the most significant. It is evident that using the optimized cut-off expression values gives much greater statistical significance. The large difference in the estimated DDg and MBg p-values was due to the different cut-off expression values estimated. The Kaplan-Meier curves are shown in Fig. 6E (using the cut-off expression values from DDg) and 6F (using the cut-off expression values from MBg). As in Figs. 6B and 6D, each of these curves shows separately the two curves for each of the two individual genes, and the two curves derived from Eqn. (7) with m=3 which benefit from the synergy of the genes.

For both pairs of genes, the DDg method separates the "low-risk" from the "high-risk" subjects more accurately as shown by the respective Kaplan-Meier curves.

<u>6. Comparison of the results in sections 4 and 5 above with those obtained using the Uppsala cohort</u>

As mentioned above, in the case of the Uppsala cohort, the training set of "typical" patients consisted of $K_u^*$ = 212 subjects. The the "mean-based" and "data-driven" cut-off expression values for each of the $N$ = 264 probesets were derived according to the comparative example and the first embodiment. The the Wald p-values were derived by Eqns. (4) and (5). At 1% significance, DDg found 195 probesets (191 prognostic gene signatures) and MBg found 131 probesets (127 prognostic gene signatures). There was almost perfect reproducibility for the top level MBg probesets (probesets with p-values lower than 0.1 %) in the DDg list, while 82% of the top 100 MBg-DDG probesets were common. The 99% BCa CI's showed results similar to the Stockholm results. For DDg, 168 significant genes (157 by MBg) were found with 28 Wald-only positives (26 by MBg) and 2 Wald-only negatives (1 by MBg). Across the two cohorts, the Wald statistic discovered 165 common probe sets (~85% of the DDg significant set) while the bootstrap method found 123 common probe sets (~73% of the bootstrap DDg significant set).

Figure 7 presents the individual and synergetic prognostic results for the LRP2-ITGA7 and CCNA2-PTPRT genes pairs. The significance of Figs. 7A to 7E corresponds to that of Figs. 6D, 6B, 6E, 6F and 6G respectively. Gene pairing with DDg was clearly the best choice in our analysis, since the two "synergy" curves of Fig. 7A and 7C are by far the best separated. High reproducibility on the findings of the two cohorts, not only for these particular pairs but also for the whole set of important gene pairs identified in Stockholm and Uppsala was observed.

Recently, we discovered a 5-gene signature (6 probesets) which re-classifies tumours with histologic grade 2 into two sub-types, 1-like grade and 3-like grade tumours (Ivshina *et al.,* 2006) that show similar genetic features with grade 1 and grade 3 breast cancer tumours, respectively. Here, we find that all 6 probesets of this 5-gene signature are survival-significant genes identified by the DDg method (BRRN1 (212949_at, p=1.4E-03); FLJ11029 (228273_at, p=1.7E-04); C6orf173 (226936_at, p=6.2E-04); STK6 (208079_s_at, p=3.4E-04; 204092_s_at, p=6.4E-04), MELK (204825_at, p=1.2E-05)), while combinations of these genes with other genes produce synergetic survival effects, suggesting that these genes are representative and robust members of quite a diverse gene regulatory network.

Figure 8 shows the capability of the 5-genes signature of Ivshina *et al.,* 2006 for predicting subjects' survival outcome for the Uppsala cohort. This figure shows that the survival curves for subjects of joined grade 1 and grade 1-like group are significantly different from the survival curve of subjects of joined grade 3 and grade 3-like group. Using this 5-gene signature we applied the SWS classification method Kuznetsov *et al.,* 2006 and found that the two groups could be discriminated with <7% errors, which suggests that the grouped tumours are different biological entities. Indeed, we observed that the tumours in grade 1&1-like group exhibit mostly "normal-like" and "luminal-A" sub-types, while tumours in 3&3-like group exhibit "luminal-B", "ERBB2+" and "basal" subtypes. For Stockholm cohort the both SWS signature and clinical sub-typing provide similar classification. In Figure 8, the survival curve of group of grades 1 & 1-like subjects contains a fraction of subjects with DFS of less than 5 years. This observation suggests the existence of a distinct subgroup of subjects with relatively poor clinical outcome. Using DDg independently and in combination with standard unsupervised techniques (e.g., hierarchical clustering) for grade 1 &1-like group we discovered a set of genes for which expression values could be associated with poor prognosis.

7. Functional significance and reproducibility of the Genes Associated with Patient Survival

The search for common genes across two independent studies may lead to the identification of the most reliable genes for further analysis. Accordingly, the functional significance and functional reproducibility of the results in the two cohorts were investigated further.

In order to compare the specificity of DDg and MBg in terms of the gene functions they identify, GO (gene ontology) analyses of the top 100 genes of each method were conducted in Panther (Protein Analysis through Evolutionary Relationships) software from the website (pantherdb.org). This grouped the genes into pathways and/or biological processes. Significant enrichment in p53 (DDg p-value = 2.4E-03; MBg p-value = 2.4E-03) and ubiquitin proteasome (DDg p-value = 5.1E-02; MBg p-value = 5.2E-02) pathways were identified. Further, significant biological processes include: cell cycle (DDg p-value = 1.2E-23; MBg p-value = 3.4E-23) and mitosis (DDg p-value = 2.4E-11; MBg p-value = 6.0E-11). Significant molecular functions include: Microtubule family Cytoskeletal protein (DDg p-value = 9.9E-10; MBg p-value = 3.0E-10) and protein kinase (DDg p-value - 1.5E-04; MBg p-value = 6.6E-05).

Similar GO analysis results were observed for the Uppsala data and the findings were well supported by the results of previous studies (Ivshina *et al.,* 2006 and Pawitan *et al.,* 2005). Importantly, the GO analysis produced results very similar to those of the top 1000 survival highly significant genes identified by DDg method from the entire list of 44928 probesets for both the Stockholm and Uppsala cohorts. Table 1 shows the GO analysis of the best gene synergy results according to Criteria 1 and 2. 100 highly significant synergetic pairs represent 44 unique DDg genes and 49 unique MBg genes were identified

**Table 1.** GO analysis of top reproducible 100 gene pairs in Stockholm and Uppsala cohorts with DDg and MBg methods. T = Total number of genes with given GO annotation, P = p-value for significance of GO term enrichment, E = Expected number of genes with given GO annotation, O = Observed number of genes with given GO annotation.

| | | DDg | | | MBg | | |
|---|---|---|---|---|---|---|---|
| | T | P | E | O | P | E | O |
| **Pathways** | | | | | | | |
| Cell cycle | 29 | 7.94E-06 | 0.12 | 4 | 9.05E-05 | 0.08 | 3 |
| p53 pathway | 136 | 2.66E-05 | 0.57 | 6 | 5.18E-05 | 0.4 | 5 |
| p53 pathway feedback loops 2 | 66 | 1.89E-04 | 0.28 | 4 | 9.87E-04 | 0.19 | 3 |
| DNA replication | 25 | 5.12E-03 | 0.11 | 2 | 2.49E-03 | 0.07 | 2 |
| Folate biosynthesis | 7 | 2.90E-02 | 0.03 | 1 | 2.02E-02 | 0.02 | 1 |
| Formyltetrahydroformate biosynthesis | 10 | 4.12E-02 | 0.04 | 1 | 2.87E-02 | 0.03 | 1 |
| Ubiquitin proteasome pathway | 89 | 5.00E-02 | 0.37 | 2 | 2.80E-02 | 0.26 | 2 |
| Parkinson disease | 106 | 7.40E-02 | 0.45 | 2 | 3.85E-02 | 0.31 | 2 |
| **Biological Process** | | | | | | | |
| Cell cycle | 1009 | 2.56E-28 | 4.25 | 40 | 7.58E-23 | 2.94 | 30 |
| Mitosis | 382 | 4.92E-14 | 1.61 | 18 | 3.54E-13 | 1.11 | 15 |
| Cell cycle control | 418 | 3.13E-10 | 1.76 | 15 | 3.69E-06 | 1.22 | 9 |
| Chromosome segregation | 121 | 2.95E-09 | 0.51 | 9 | 2.99E-09 | 0.35 | 8 |
| Cell proliferation and differentiation | 1028 | 3.04E-07 | 4.33 | 18 | 1.43E-06 | 2.99 | 14 |
| DNA metabolism | 360 | 4.07E-07 | 1.51 | 11 | 1.02E-07 | 1.05 | 10 |
| DNA replication | 155 | 4.78E-06 | 0.65 | 7 | 6.66E-06 | 0.45 | 6 |
| Protein phosphorylation | 660 | 1.13E-04 | 2.78 | 11 | 6.76E-04 | 1.92 | 8 |

(continued)

| | | | | | | | |
|---|---|---|---|---|---|---|---|
| **Biological Process** | | | | | | | |
| Meiosis | 84 | 4.68E-04 | 0.35 | 4 | 1.96E-03 | 0.24 | 3 |
| DNA repair | 169 | 7.86E-04 | 0.71 | 5 | 1.55E-03 | 0.49 | 4 |
| Protein modification | 1157 | 1.18E-03 | 4.87 | 13 | 1.89E-03 | 3.37 | 10 |
| Cytokinesis | 115 | 1.49E-03 | 0.48 | 4 | 4.72E-03 | 0.33 | 3 |
| Embryogenesis | 141 | 3.10E-03 | 0.59 | 4 | 7.98E-04 | 0.41 | 4 |
| Developmental processes | 2152 | 3.76E-03 | 9.05 | 18 | 8.82E-03 | 6.26 | 13 |
| Oncogenesis | 472 | 3.94E-03 | 1.99 | 7 | 4.91 E-02 | 1.37 | 4 |
| Cell structure | 687 | 8.69E-03 | 2.89 | 8 | 5.01E-02 | 2 | 5 |
| DNA recombination | 44 | 1.51 E-02 | 0.19 | 2 | 3.06E-04 | 0.13 | 3 |
| Protein targeting and localization | 253 | 2.25E-02 | 1.06 | 4 | 6.49E-03 | 0.74 | 4 |
| Cell structure and motility | 1148 | 2.31E-02 | 4.83 | 10 | 1.17E-01 | 3.34 | 6 |
| Mesoderm development | 551 | 2.94E-02 | 2.32 | 6 | 7.71E-02 | 1.6 | 4 |
| Other cell cycle process | 9 | 3.72E-02 | 0.04 | 1 | 2.59E-02 | 0.03 | 1 |
| Lipid, fatty acid and steroid metabolism | 770 | 3.73E-02 | 3.24 | 0 | 1.03E-01 | 2.24 | 0 |
| Nucleoside, nucleotide and nucleic acid metabolism | 3343 | 3.81 E-02 | 14.07 | 21 | 2.94E-02 | 9.73 | 16 |
| **Molecular function** | | | | | | | |
| Microtubule binding motor protein | 68 | 5.19E-13 | 0.29 | 10 | 3.37E-11 | 0.2 | 8 |
| Microtubule family cytoskeletal protein | 235 | 4.27E-10 | 0.99 | 12 | 1.90E-09 | 0.68 | 10 |
| Kinase activator | 62 | 7.45E-06 | 0.26 | 5 | 3.55E-05 | 0.18 | 4 |
| DNA helicase | 76 | 1.97E-05 | 0.32 | 5 | 1.48E-03 | 0.22 | 3 |
| Non-receptor serine/threonine protein kinase | 303 | 4.65E-05 | 1.27 | 8 | 1.96E-03 | 0.88 | 5 |
| Cytoskeletal protein | 878 | 8.62E-05 | 3.69 | 13 | 2.29E-04 | 2.55 | 10 |
| Kinase modulator | 175 | 1.06E-04 | 0.74 | 6 | 1.76E-03 | 0.51 | 4 |
| Protein kinase | 529 | 4.18E-04 | 2.23 | 9 | 8.97E-04 | 1.54 | 7 |
| Helicase | 173 | 8.72E-04 | 0.73 | 5 | 1.43E-02 | 0.5 | 3 |
| Exodeoxyribonuclease | 15 | 1.89E-03 | 0.06 | 2 | 9.13E-04 | 0.04 | 2 |
| Kinase | 684 | 2.47E-03 | 2.88 | 9 | 3.80E-03 | 1.99 | 7 |
| Nucleic acid binding | 2850 | 7.47E-03 | 11.99 | 21 | 1.62E-02 | 8.29 | 15 |
| DNA topoisomerase | 6 | 2.49E-02 | 0.03 | 1 | 1.73E-02 | 0.02 | 1 |
| DNA strand-pairing protein | 6 | 2.49E-02 | 0.03 | 1 | 1.73E-02 | 0.02 | 1 |

(continued)

| Molecular function | | | | | | | |
|---|---|---|---|---|---|---|---|
| Select regulatory molecule | 1190 | 2.87E-02 | 5.01 | 10 | 5.79E-02 | 3.46 | 7 |
| Non-motor microtubule binding protein | 74 | 3.93E-02 | 0.31 | 2 | 1.99E-02 | 0.22 | 2 |
| Nuclease | 189 | 4.61E-02 | 0.8 | 3 | 1.80E-02 | 0.55 | 3 |

8. A Large Number of Gene Pairs can exhibit a significantly high synergetic survival effect

In order to compare specificity and sensitivity of the methods in the 2-D case (i.e. using pairs of genes), 34716 pairs of genetic grade signature pairs were considered and the numbers of pairs which provide a significant p-value by the Wald statistic of survival curves < 0.01 were counted. The DDg method was more specific and more sensitive than the MBg method. For example using the Stockholm cohort, MBg identified 11778 significant probeset pairs. In comparison, the DDg method identified 16489 significant pairs (~1.4 times the MBg method), resulting in 4711 DDg pairs unique to DDg. The large difference in the number of significant genes identified by the two methods shows that the DDg method can find interesting genes not located by the MBg method (or any other grouping method based on a single point estimate of $y_i$ expression levels). This feature indicates that the DDg method may be particularly appealing for prognostic gene identification.

Using the more stringent p-value of 0.005, it was found that ~39% of the gene pairs common to both DDg and MBg were false positives. In addition, at a significance level *alpha* = 1% (equivalent to p-value of 0.01), 40% of the unique DDg gene pairs were false positives. In order to reduce Type I errors due to multiple testing (false positives), Bonferroni correction was applied on the Wald test p-values to base the inference on a more stringent significance level. This method identified 1180 significant DDg gene pairs in the Stockholm cohort and 1465 significant DDg gene pairs in the Uppsala cohort, with 53 common pairs in the two cohorts. The respective values derived using MBg approach were 85 significant gene pairs in the Stockholm cohort and 75 significant gene pairs in the Upsala cohort, with no common gene pairs between the two cohorts. For the individual gene analysis, DDg with Bonferroni correction found 97 and 88 significant genes in the Stockholm and Uppsala cohorts, respectively, with 58 common genes between the two cohorts, while the corresponding numbers for MBg were 35 and 36 significant genes (10 common).

The grouping scheme analysis was repeated for the full data set of 44928 probesets. In the Stockholm cohort, the DDg method identified 7473 prognostic significant genes by the Wald test. With Bonferroni correction, the number was 90 prognostic significant genes. In the Uppasala cohort, the respective numbers were 5545 by the Wald test and 55 after Bonferroni correction. Between the two cohorts, 3152 common prognostic genes were identified by the Wald-based DDg test while the MBg method identified 559 (~18% of DDg). This further supports that the DDg method is able to identify many statistically significant and biologically meaningful prognostic and predictive genes.

Table 2 presents the top 7 gene pairs in terms of the Criteria 1 and 2. These pairs exhibit high synergetic effect in both cohorts and their synergy produces significantly stronger effect than individual grouping (as indicated by the respective p-values in Table 2).

**Table 2.** Top 7 gene pairs in Stockholm and Uppsala cohorts. P-values of common synergetic genes (and model) of Stockholm (S) and Uppsala (U) cohorts (columns 3-4); P-values of independent grouping (columns 5-8). ** Breast cancer associated genes; * cancer associated genes.

| $Gene^1$ | $Gene^2$ | $P_S$ (model) | $P_U$ (model) | $P^1_S$ | $P^1_U$ | $P^2_S$ | $P^2_U$ |
|---|---|---|---|---|---|---|---|
| LRP2** | ITGA7** | 2.5E-06 (4) | 3.1E-07 (4) | 9.8E-04 | 1.0E-02 | 1.9e-03 | 3.5e-03 |
| CCNA2** | PTPRT** | 4.0E-08 (3) | 1.4E-06 (3) | 1.8E-05 | 5.7E-04 | 1.4E-05 | 2.2e-02 |
| NUDT1** | NMU* | 1.7E-06 (2) | 1.1E-06 (2) | 1.1e-04 | 3.9e-03 | 9.5E-03 | 1.5E-04 |
| CCNE2** | CENPE** | 1.2E-06 (2) | 6.2E-06 (2) | 9.2E-05 | 2.1E-03 | 7.0E-05 | 1.1e-03 |
| CDCA8** | CLDN5** | 1.9E-06 (3) | 7.8E-08 (3) | 5.6E-04 | 1.4E-05 | 1.1e-04 | 1.7e-02 |
| HN1* | CACNA1D | 7.3E-06 (3) | 8.5E-06 (3) | 5.9E-04 | 3.8E-04 | 2.1e-03 | 4.1e-03 |
| SPAG5** | FLJ20105** | 2.0E-06 (2) | 4.7E-07 (4) | 6.1E-05 | 5.9E-04 | 9.9E-05 | 3.7e-05 |

Survival significant gene are summarized in the table 3.

**Table 3.** Nineteen Survival Significant Genes. Top 7 gene pairs in Stockholm and Uppsala cohorts. P-values of common synergetic genes (and model) of Stockholm (S) and Uppsala (U) cohorts (columns 3-4). 5-gene genetic grading signature genes. "2D" refers to selecting pairs of genes, "1D" refers to selecting individual genes.

| **Gene** | **$P_S$ (model)** | **$P_U$ (model)** | **Method** |
|---|---|---|---|
| LRP2 | 2.5E-06 (4) | 3.1E-07 (4) | 2D |
| ITGA7 | 2.5E-06 (4) | 3.1E-07 (4) | 2D |
| CCNA2 | 4.0E-08 (3) | 1.4E-06 (3) | 2D |
| PTPRT | 4.0E-08 (3) | 1.4E-06 (3) | 2D |
| CCNE2 | 1.2E-06 (2) | 6.2E-06 (2) | 2D |
| CENPE | 1.2E-06 (2) | 6.2E-06 (2) | 2D |
| CDCA8/Borealin | 1.9E-06 (3) | 7.8E-08 (3) | 2D |
| CLDN5 | 1.9E-06 (3) | 7.8E-08 (3) | 2D |
| SPAG5 | 2.0E-06 (2) | 4.7E-07 (4) | 2D |
| FLJ20105/ERCC6L | 2.0E-06 (2) | 4.7E-07 (4) | 2D |
| NUDT1 | 1.7E-06 (2) | 1.1E-06 (2) | 2D |
| NMU | 1.7E-06 (2) | 1.1E-06 (2) | 2D |
| HN1 | 7.3E-06 (3) | 8.5E-06 (3) | 2D |
| CACNA1D | 7.3E-06 (3) | 8.5E-06 (3) | 2D |
| **5-gene signature** | 5.20E-06 | NA | |
| BRRN1 | 1.40E-03 | NA | 1D |
| FLJ11029/228273_at | 1.70E-04 | NA | 1D |
| C6orf173/CENPW | 6.20E-04 | NA | 1D |
| STK6 | 3.40E-04 | NA | 1D |
| MELK | 1.20E-05 | NA | 1D |

Discussion

In the embodiments, the semi-parametric Cox proportional hazard regression model was used to estimate predictive significance of genes for disease outcome as indicated by patient survival times. For a given gene, the optimal partition (cut-off expression value) of its expression domain was estimated by maximising the separation of survival curves related to the high- and low-risk of disease behaviour, as indicated by the Wald statistic derived from the corresponding univariate Cox partial likelihood function. The top-level genes having the largest Wald statistic were selected for further confirmatory GO- analysis and inclusion into gene signatures.

A similar selection procedure was also developed in order to construct two-genes signatures exhibiting synergetic influence on patient survival. This approach was applied to analyse Affymetrix U133 data sets of two large breast cancer cohorts to identify genes and genes pairs related to genetic breast cancer grade signature (Ivshina *et al.,* 2006). The genes that were most significantly correlated with the disease free survival time of breast cancer patients were selected. These genes could be subsequently used as an input in reconstruction analysis of biological programs/pathways associated with aggressiveness of breast cancer (Ivshina *et al.,* 2006).

All genes of 5-gene genetic grading signature are survival significant. Additionally, they are co-regulated in primary breast cancer samples (data not present) and could functionally related to each other._BRRN1 encodes a member of the barr gene family and a regulatory subunit of the condensin complex. This complex is required for the conversion of interphase chromatin into condensed chromosomes. The protein encoded by this gene is associated with mitotic chromosomes, except during the early phase of chromosome condensation. During interphase, the protein has a distinct punctate nucleolar localization. There is a SSB-specific response of condensin I through PARP-1 and a role for condensin in SSB. repair. The protein encoded by STK6 is a cell cycle-regulated kinase that appears to be involved in microtubule formation and/or stabilization at the spindle pole during chromosome segregation. The encoded protein is found at the centrosome in interphase cells and at the spindle poles in mitosis. This gene may play a role in tumor development and progression. A processed pseudogene of this gene has been found on chromosome 1, and an unprocessed pseudogene has been found on chromosome 10. Multiple transcript variants encoding the same protein have been found for this gene. MELK is known to have a critical role in the proliferation of brain tumors, including their stem cells, and suggest that MELK may be a compelling molecular target for treatment of high-grade brain tumors. 2. Maternal embryonic leucine zipper kinase transcript abundance correlates with malignancy grade in astrocytomas 3. the kinase activity of MELK is likely to affect mammary carcinogenesis through inhibition of the pro-apoptotic function of Bcl-GL 4. analysis of MELK substrate specificity and activity regulation 5. pEg3 is a potential regulator of the G2/M progression and may act antagonistically to the CDC25B phosphatase,pEg3 kinase is able to specifically phosphorylate CDC25B in vitro. One phosphorylation site was identified and corresponded to serine 323.

For our work that available information is important in context of validation of our method of identification of patient survival significant genes. In particular, these 3 genes are included in the genetic grade signature which classifies breast cancer patients according to aggressiveness of the cancer disease (Ivshina et al, 2006) and have also used as important clinical markers of breast cancer and other human cancers. MELK is used now as a target for adjuvant therapy in clinical trials. All these genes are survival significant by our DDg estimates.

The biological importance of the DDg survival genes was also demonstrated by the proliferative pattern of the 5-gene grade signature (Figure 8).

Thus, these genes itself or in pairs or in larger number groups with other genes represent biologically and clinically important survival significant gene signature. We could claim, that such genes and their combinations could be used as " a positive control" for reliable selection of poorly defined/unknown genes which could be promising as novel and important components of critical biological pathways in cancer cells, and novel markers for prognosis and prediction of cancer patients.

In particular, C6orf173 (226936_at, 1 D DDg: p=6.2E-04) which is a member of our 5-gene genetic grading signature (Ivshina et al, 2006) and this gene is also a survival significant by our analysis (Kuznetsov *et al,* 2006; Motakis *et al,* 2009). It was reported a a cancer up-regulated gene (CUG2) (Lee *et al,* 2007; Kim *et al,* 2009). CUG2 was recently renamed CENPW based on the new findings that it is a component of the centromeric complex playing a crucial role in the assembly of functional kinetochore complex during mitosis (Hori et al, 2008).

FLJ11029 (228273_at, p=1.7E-04) is an unknown gene. We found that with very high expression in many cancer tissues and cell lines, however the functions of this gene are unknown. Due to moderate level of evolution conservation and lost of ORF structures (data not shown), FLJ11029 (228273_at) gene could be considered as a novel long non-protein coding gene. In Uppsala and Stockholm cohorts, CUG2(CENPW) and FLJ11029 are strongly positively correlated to each other (Kendal correlation; p<0.0001) and simultaneously with the expression levels of BRRN1, STK6, and MELK (Kendal correlation p<0.0001). Our results of survival analysis suggest that all 5 genes are survival significant and could form essential functional module associated with mitosis phase of breast cancer cells.

Base on these findings we suggest that CUG2, BRRN1, STK6, MELK and can be involved in same regulatory pathway (or sub-network) which is associated with mitotic chromosomes, chromosome condensation and their segregation.

Thus, we suggest a function of genetic grade 5-gene signature genes, related to their biological attributes to coordination and co-regulation dynamics of mitotic chromosomes; these genes provide a synergetic effect on survival of the breast cancer patients and could be used to identify distinct subtypes of breast cancers.

A large number of synergetic gene pairs that were significantly associated with survival of breast cancer patients had been identified and biologically meaningful information about these survival-significant genes may also be postulated. This DDg approach was able to identify interesting targets that were not picked up by other methods, e.g. MBg approach. In order to further test the effectiveness of the DDg approach on the whole gene population of the study, the two methods were applied to the 44928 probesets and the DDg prognostic genes list was found to be twice as large and all genes identified by MBg were also present in the DDg list.

Most of the top-level survival significant genes were related to the cell cycle, and more specifically to the shortest phase of the cell cycle, mitosis (Table 1, Table 2). Figure 6E and 6F show an example of a highly significant partition of breast cancer patients by survival (DFS) based on one of cell cycle gene pairs - CCNA2-PTPRT. CCNA2 (Cyclin-A2 or Cyclin A) is an essential gene for the control of the cell cycle at the G1/S (start) and G2/M (mitosis) transitions and it is accumulated steadily during G2 and is abruptly destroyed at mitosis. The paired partner of CCNA2 is a receptor protein tyrosine phosphatise T gene, PRPRT, which regulates cell growth, proliferation and may be important for the STAT3 signalling pathway and development of some cancers. The present survival analysis suggests that this pair of regulatory genes could be involved with other cell cycle genes (see, for example, Table 2) in the control of breast cancer cell development. CCNA2 has second synergistic gene partner, it is CENPE.

CENPE (Centrosome-associated protein E) is a kinesin-like motor protein that accumulates in the G2 phase of the cell cycle. Unlike other centrosome-associated proteins, it is not present during interphase and first appears at the centromere region of chromosomes during prometaphase. CENPE is proposed to be one of the motors responsible for mammalian chromosome movement and/or spindle elongation. CDCA8(synonym Borealin) is a synergestic survival significant partner of CENPE. CDCA8 is a component of a chromosomal passenger complex required for stability of the bipolar mitotic spindle. CDCA8 play regulatory role in SUMO pathway including RanBP2 and SENP3. Mitotic regulator Survivin binds as a monomer to its functional interactor Borealin. Thus the both genes CENPE and CDCA8 could be physically co-localized in vicinity of centromeric and spindle regions of mitotic cells and form a functional module controlling key biological processes of cell mitosis. Our goodness-off split survival (DDg) analysis suggests that the both genes could be essential for survival of breast cancer patients.

An other survival significant gene SPAG5 encodes a protein associated with the mitotic spindle apparatus (http://www.genecards.org/cgi-bin/carddisp.pl?gene=SPAG5&search=SPAG5). By the literature, SPAG5 encoded protein may be involved in the functional and dynamic regulation of mitotic spindles. FLJ20105 ((ERCC6L); http://www.genecards.org/cgi-bin/carddisp.pl?gene=ERCC6L&search=ERCC6L) is a partner of synergistic survival significant pair of SPAG5. ERCC6L is DNA helicase that acts as an essential component of the spindle assembly checkpoint. Contributes to the mitotic checkpoint by recruiting MAD2 to kinetochores and monitoring tension on centromeric chromatin. Acts as a tension sensor that associates with catenated DNA which is stretched under tension until it is resolved during anaphase. Thus, the both genes of that pair are co-localized (centromeric region) and involved in the same molecular machinery (mitotic spindle assembly).

Thus, based on our analysis, we claim that gene pairs SPAG5-ERCC6L, CENPE-CCNE2, CDCA8-CLDN5 consist of a novel breast cancer associated synergistic survival significant gene pairs playing a important role in dynamics of chromosomes during cell mitosis. Together with the 5-gene genetic grade signature genes they form an integrative functional module essential for breast cancer prognosis and prediction.

Compared to single survival significant genes, gene pairs showed highly significant p-values of Wald statistic ($\sim 10^{-8}$ vs $\sim 10^{-5}$). This result implied that the pairing procedure itself should be considered as a unique statistical tool for identification of patients with very poor/good prognosis. Interestingly, some of the gene pairs are not directly related to the cell cycle.

Some of the pairs, for example, megalin (LRP2) and itnergrin alpha 7 (ITGA7) have not been discussed for breast cancer classification or patients' survival in the literature. The megalin gene contributes to the endocytic uptake of 25(OH)D3-DBP and activation of the vitamin D receptor (VDR) pathway. The VDR pathway is normally expressed in mammary gland, where it functions to oppose estrogen-driven proliferation and maintain differentiation. LRP2 can be highly expressed in some breast cancer cells. The above suggest that LRP2 participates in negative growth regulation of mammary epithelial cells. Associations of expression of integrin alpha 7 with breast cancer and breast cancer patient survival have not been reported. Megalin and integrin alpha 7 have also not been reported as molecular partners in anti-cancer regulation.

In the present study, several other gene pairs can be related to strong survival significance and produce an interaction effect influencing the likelihood of survival of breast cancer patients (Results, Table 2). These gene pairs, discovered by the present approach, might be grouped into survival significant interactome sub-networks and be an important source for the discovery of new anti-cancer drugs.

HN1-CACNA1D pair. Hematological and neurological expressed 1 protein is a protein that in humans is encoded by the *HN1* gene. CACNA1D is a calcium channel, voltage-dependent, L type, alpha 1 D subunit. The roles of these genes in breast cancer have been not studied yet.

Mis-incorporation of oxidized nucleoside triphosphates into DNA/RNA during replication and transcription can cause mutations that may result in carcinogenesis or neurodegeneration. The protein encoded by NUDT1 (synonym MTH1) is an enzyme that hydrolyzes oxidized purine nucleoside triphosphates, such as 8-oxo-dGTP, 8-oxo-dATP, 2-hydroxy-dATP, and 2-hydroxy rATP, to monophosphates, thereby preventing mis-incorporation. The encoded protein is localized mainly in the cytoplasm, with some in the mitochondria, suggesting that it is involved in the sanitization of nucleotide pools both for nuclear and mitochondrial genomes. Several alternatively spliced transcript variants, some of which encode distinct isoforms, have been identified. Additional variants have been observed, but their full-length natures have not been determined. A single-nucleotide polymorphism that results in the production of an additional, longer isoform (p26) has been described. NUDT1 plays an important role in protecting cells against H(2)O(2)-induced apoptosis via a Noxa- and caspase-3/7-mediated signaling pathway. Elevated levels of NUDT1 protein is associated non-smal cell lung carcinomas. This gene provides synergistic survival effect together with gene neuromedin U (NMU). NMU may be involved in the HGF-c-Met paracrine loop regulating cell migration, invasiveness and dissemination of pancreatic ductal adenocarcinoma. NMU and its cancer-specific receptors, as well as its target genes, are frequently overexpressed in clinical samples of lung cancer and in cell lines, and that those gene products play indispensable roles in the growth and progression of lung cancer cells. NmU expression is related to Myb and that the NmU/NMU1R axis constitutes a previously unknown growth-promoting autocrine loop in myeloid leukemia cells. NMU plays a role in feeding behavior and catabolic functions via corticotropin-releasing hormone. Amino acid variants in NMU associate with overweight and obesity, suggesting that NMU is involved in energy regulation in humans. Overexpression of neuromedin U is associated with bladder tumor formation, lung metastasis and cancer cachexia. Our results suggest important role of NUDT1 and NMU genes in breast cancer progression and the breast patient's survival. Thus, this pair could be considered as novel prognostic and predictive biomarkers for breast cancer.

Thus, our data-driven approach allows the discovery of novel mechanistically important individual genes and small gene signatures that predict cancer patient survival. In doing so, the method can lead to the identification of new potential targets for anti-cancer drugs and facilitate the development of alternative approaches for cancer treatment.

The methods can be extended up to any number of genes. We can check only the "all possible pairs" of the statistically significant individual genes or generally all possible pairs of genes in our study. The number of genes to be used is purely a matter of the researcher's choice.

**Table 4** indicates the SEQ ID NO. and the corresponding gene and GenBank reference in the sequence listing. Where more than one transcript variant exists, the invention may be practiced with any one of the transcript variants, any of several of the transcript variants or all of the transcript variants.

| SEQ ID NO: | Gene | GenBank Reterence |
|---|---|---|
| 1 | LRP2 | LRP2 |
| 2 | ITGA7 transcript variant 2 | ITGA7 transcript variant 2 |
| 3 | CCNA2 | CCNA2 |
| 4 | PTPRT transcript variant 1 | PTPRT transcript variant 1 |
| 5 | FLJ11029 228273 at | FLJ11029 228273_at |
| 6 | C6orf173/CENPW/CUG2 | C6orf173/CENPW/CUG2 |
| 7 | MELK | MELK |
| 8 | NUDT1/MTH1 transcript variant 4A | NUDT1/MTH1 transcript variant 4A |
| 9 | NMU | NMU |
| 10 | CCNE2 | CCNE2 |
| 11 | CENPE | CENPE |
| 12 | CDCA8/Borealin | CDCA8/Borealin |
| 13 | CLDN5 transcript variant 2 | CLDN5 transcript variant 2 |
| 14 | HN1 transcript variant 2 | HN1 transcript variant 2 |
| 15 | HN1 transcript variant 3 | HN1 transcript variant 3 |
| 16 | CACNA1 D transcript variant 1 | CACNA1 D transcript variant 1 |
| 17 | SPAG5 | SPAG5 |
| 18 | FLJ20105/ERCC6L | NM_017669.2 |
| 19 | BBRN1/ NCAPH | NM_015341.3 |
| 20 | STK6/ AURKA transcript variant 1 | NM_198433.1 |

References

Breslow, N.E., "Covariance analysis of censored survival data", Biometrics, vol. 30, pp 89-99, 1974.

Cox DR: Regression Models and Life Tables (with Discussion). Journal of the Royal Statistical Society Series B 1972, 34: 187-220.

Cox, D.R. and Snell, E.J., "A general definition of residuals (with discussion)", Journal of the Royal Statistical Society, Series B, vol. 30, pp 248-265, 1968.

Cox R.D. and Oakes, D., Analysis of Survival Data. London: Chapman and Hall, 1984.

Efron, B. and Tibshirani, R.J., An Introduction to the Bootstrap. New York: Chapman and Hall, 1994.

Hori T, Amano M, Suzuki A, Backer CB, Welburn JP, Dong Y, McEwen BF, Shang WH, Suzuki E, Okawa K, Cheeseman IM, Fukagawa T. CCAN makes multiple contacts with centromeric DNA to provide distinct pathways to the outer kinetochore. Cell. 2008 Dec 12;135(6):1039-52.PMID: 19070575

Ivshina, A.V., George, J., Senko, O et al, "Genetic reclassification of histologic grade delineates new clinical subtypes of breast cancer", Cancer Research, vol. 66, pp 10292-10301, 2006.

Kaplan, E. L. and Meier, P., "Nonparametric estimation from incomplete observations". JASA, vol. 53, 457-48, 1958.

Kim H, Lee M, Lee S, Park B, Koh W, Lee DJ, Lim DS, Lee S. Cancer-upregulated gene 2 (CUG2), a new component of centromere complex, is required for kinetochore function. Mol Cells. 2009 Jun;27(6):697-701. Epub 2009 Jun 12.PMID: 19533040

Kuznetsov, V.A., Senko, O.V., Miller, L.D. and Ivshina, A., "Statistically Weighted Voting Analysis of Microarrays for Molecular Pattern Selection and Discovery Cancer Genotypes", International Journal of Computer Science and Network Security, vol. 6, pp 73-83, 2006.

Lee S, Gang J, Jeon SB, Choo SH, Lee B, Kim YG, Lee YS, Jung J, Song SY, Koh SS. Molecular cloning and functional analysis of a novel oncogene, cancer-upregulated gene 2 (CUG2). Biochem Biophys Res Commun. 2007 Aug 31;360(3):633-9. Epub 2007 Jun 28.PMID: 17610844

Loughin, T.M., "A residual bootstrap for regression parameters in proportional hazards model", J. of Statistical and Computational Simulations, vol. 52, pp 367-384, 1995.

Motakis, E., Nason, G.P., Fryzlewicz, P. and Rutter, G.A., "Variance stabilization and normalization for one-color microarray data using a data-driven multiscale approach", Bioinformatics, vol. 22, pp 2547-2553, 2006.

Motakis E, Ivshina AV, Kuznetsov VA. Data-driven approach to predict survival of cancer patients: estimation of microarray genes' prediction significance by Cox proportional hazard regression model. IEEE Eng Med Biol Mag. 2009 Jul-Aug;28(4):58-66.

Millenaar, F. F., Okyere, J., May, S.T., van Zanten, M., Voesenek, L.A.C.J. and Peters, A.J.M., "How to decide? Different methods of calculating gene expression from short oligonucleotide array data will give different results", BMC Bioinformatics, vol. 7, no. 137, 2006.

Pawitan, Y., Bjohle, J., Amler, L., at al, "Gene expression profiling spares early breast cancer patients from adjuvant therapy: derived and validated in two population-based cohorts", Breast Cancer Research, vol. 7, pp R953-R964, 2005.

Press, W.H., Flannery, B.P., Teukolsky, S.A and Vetterling, W.T., "Numerical Recipes in C: The Art of Scientific Computing", Cambridge University Press, 1992. Sambrook and Russel, Molecular Cloning: A Laboratory Manual, Cold Springs Harbor Laboratory, New York (2001).

The invention is further described in the following clauses:

1. A computerized method for optimising, for each gene i of a set of N genes, a corresponding cut-off expression value $c^i$ for partitioning subjects according to the expression level of the corresponding gene,
the method employing medical data which, for each *subject k* of a set *of K** subjects suffering from the medical condition, indicates (i) the survival time of subject k, and (ii) for each gene i, a corresponding gene expression value $y_{i,k}$ of subject *k*;
the method comprising, for each gene i,

(i) for each of a plurality of a trial values of $c^i$:

(a) identifying a subset of the $K^*$ subjects such that $y_{i,k}$ is above the trial value of $c^i$;
(b) computationally fitting the corresponding survival times of the subjects to the Cox proportional hazard regression model, said fitting using, for subjects within the subset, a regression parameter $\beta_i$ corresponding to the gene $i$; and
(c) obtaining from the regression parameter $\beta_i$, a significance value indicative of prognostic significance of the gene;

(ii) identifying the trial cut-off expression value for which the corresponding significance value indicates the highest prognostic significance for the gene i.

2. A computerized method according to clause 1 in which, in step (c), the significance value is a Wald statistic of the Cox proportional hazard regression model.

3. A computerized method according to clause 1 further including a preliminary step of:

measuring, for each gene i, the distribution of the corresponding expression values $y_{i,k}$ of the $K^*$ subjects;
for each gene i, selecting a range of the corresponding distribution; and
generating the plurality of trial values of $c^i$ as values within the range,
said optimization of the cut-off expression value $c^i$ for each gene i being performed by performing said steps (a) and (b) for each trial value of $c^i$, and step (ii) comprising selecting the trial value of $c^i$ for which the corresponding regression parameter $\beta_i$ indicates the highest prognostic significance for the gene i.

4. A computerized method according to clause 3 in which the trial values of $c^i$ are the elements of the vector of dimension 1 x Q, $\vec{w^i}\, y_{\bar{i}}$, where $y_{\bar{i}}$, is the log-transformed intensities within $(q_{10}^i, q_{90}^i)$ and Q is the number of elements in $\vec{w^i}$.

5. A computerized method according to clause 4 in which, for each value of i and z:

step (a) includes generating a parameter

$$x_k^i = \begin{cases} 1\ (first-group) & if \quad y_{i,k} > \underline{w_z^i} = c^i \\ 0\ (second\ group) & if \quad y_{i,k} \leq \underline{w_z^i} = c^i \end{cases}$$

step (b) is performed using the Cox proportional hazard regression model in the form:

$$\log\ h^i{}_k(t_k \mid x_k^i, \beta_i) = \alpha_i(t_k) + \beta_i \cdot x_k^i,$$

where $h^i{}_k$ is a hazard function, $t_k$ denotes the survival time of the $k$-th subject, and $\alpha_i(t_k)$ represents an unspecified log-baseline hazard function; and
in step (c), the significance value is given by the univariate Cox partial likelihood function:

$$L(\beta_i) = \prod_{k=1}^{K} \left\{ \frac{\exp(\beta_i^T x_k^i)}{\sum_{j \in R(t_k)} \exp(\beta_i^T x_j^i)} \right\}^{e_k}$$

where $R(t_k) = \{j: t_j \geq t_k\}$ is the risk set at time $t_k$, $\beta_i^T$ is the transposed 1 x N regression parameters vector and $e_k$ indicates whether the patient has experienced a specific clinical event.

6. A computerized method of identifying, within a set of N genes, one or more genes which are statistically associated with prognosis of a potentially-fatal medical condition, the method including:

(i) for each gene i of the set of genes, optimising a corresponding cut-off expression value $c^i$ , by a method according to clause 1; and

(ii) identifying one or more genes i for which the corresponding significance values for the optimised cut-off expression value $c^i$ have the highest prognostic significance.

7. A computerized method according to clause 6 including a step, after step (ii) of rejecting identified genes having a prognostic significance below a threshold.

8. A computerized method of obtaining information about a first subject in relation to a potentially-fatal medical condition, the method including:

(i) identifying, by a method according to clause 6, from among a set of N genes, one or more genes which are statistically associated with prognosis of the medical condition, and the corresponding optimized cut-off expression values;

(ii) for each of the one or more identified genes obtaining a corresponding gene expression value $y_i$ of the first subject; and

(iii) obtaining said information using the obtained gene expression values and the optimized cut-off expression values.

9. A computerized method according to clause 8 in which said information is a prognosis for the first subject who is suffering from the medical condition, a susceptibility of the first subject to the medical condition, a prediction of the recurrence of the medical condition, or a recommended treatment for the medical condition.

10. A computerized method according to clause 1 wherein the medical condition is cancer, and the expression levels are from samples of respective tumours in the $K^*$ subjects.

11. A computerized method according to clause 10 wherein the medical condition is breast cancer.

12. A computerized method for identifying one or more pairs of genes, selected from a set of N genes, which are statistically associated with prognosis of a potentially-fatal medical condition, the method comprising:

(i) for each of the *N* genes obtaining a corresponding optimized cut-off expression value by a method according to clause 1;

(ii) forming a plurality of pairs of the identified genes (i, *j* with $i \neq j$), and for each pair of genes:

(1) forming a plurality of models $m_{i,j}$, each model $m_{i,j}$ including a comparison with $c^i$ and $c^j$ of the respective levels of expression $y_{i,k}$ ,$y_{j,k}$ of the genes *i* ,*j* in the set of $K^*$ subjects;

(2) for each model determining, a respective subset of the $K^*$ subjects using the model;

(3) computationally fitting the corresponding survival times of the subjects to the Cox proportional hazard regression model, said fitting using, for subjects within each of the subsets, a corresponding regression parameter $\beta_{ij}^{m2}$ corresponding to the test $m_{i,j}$; and

(4) obtaining from the regression parameters $\beta_{ij}^{m2}$, a significance value indicative of prognostic significance of the model; and

(iii) identifying one or more of said pairs of genes *i,j* for which the corresponding significance values for one of the models have the highest prognostic significance.

13. A computerized method according to clause 12, wherein said models for gene pair (i, *j*) are selected from the group consisting of:

(1) either $y_{i,k} > c^i$ and $y_{j,k} > c^j$ *or* $y_{i,k} < c^i$ and $y_{j,k} < c^j$;
*(2)* $Y_{i,k} > c^i$ and $y_{j,k} > c^j$;
(3) $y_{i,k} > c^i$ and $y_{j,k} < c^j$;
(4) $y_{i,k} < c^i$ and $y_{j,k} < c^j$;
*(5)* $y_{i,k} < c^i$ and $y_{j,k} > c^j$;
*(6)* $y_{i,k} > c^i$; and
(7) $y_{j,k} > c^j$.

14. A computerized method of obtaining information about a first subject in relation to a potentially-fatal medical condition, the method including:

(i) identifying, by a method according to clause 12, from among a set of N genes, one or more pairs of genes which are statistically associated with prognosis of the medical condition, and the corresponding optimized cut-off expression values;

(ii) for each of the one or more identified pairs of genes *i, j* obtaining corresponding gene expression values $y_i$ and $y_j$ of the first subject; and

(iii) obtaining said information using the obtained gene expression values and the optimized cut-off expression values.

15. A computerized method according to clause 14 in which said information is a prognosis for the first subject who is suffering from the medical condition, a susceptibility of the first subject to the medical condition, a prediction of the recurrence of the medical condition, or a recommended treatment for the medical condition.

16. A computerized method for identifying one or more pairs of genes, selected from a set of N genes, which are statistically associated with prognosis of a potentially-fatal medical condition,
the method employing medical data which, for each subject k of a set *of* $K^*$ subjects suffering from the medical condition, indicates (i) the survival time of subject k, and (ii) for each gene i, a corresponding gene expression value $y_{i,k}$ of subject k; the method comprising:

(i) for each of the N genes obtaining a corresponding cut-off expression value;

(ii) forming a plurality of pairs of the identified genes ($i, j$ with $i \neq j$), and for each pair of genes:

(1) forming a plurality of models $m_{i,j}$, each model $m_{i,j}$ including a comparison of the corresponding cut-off expression values $c^i$ and $c^j$ of the respective levels of expression $y_{i,k}$ ,$y_{j,k}$ of the genes $i$ ,$j$ in the set of $K^*$ subjects;

(2) for each model determining, a respective subset of the $K^*$ subjects using the model;

(3) computationally fitting the corresponding survival times of the subjects to the Cox proportional hazard regression model, said fitting using, for subjects within each of the subsets, a corresponding regression parameter $\beta_{ij}^{m}$ corresponding to the model $m_{i,j}$; and

(4) obtaining from the regression parameters $\beta_{ij}^{m}$ , a significance value indicative of prognostic significance of the model; and

(iii) identifying one or more of said pairs of genes $i,j$ for which the corresponding significance values for one of the models have the highest prognostic significance.

17. A computer program product, such as a tangible recording medium, carrying program instructions to cause a computer system to perform a method according to any of clauses 1 to 16.

18. A computer system arranged to perform a method according to any of clauses 1 to 16.

19. A method according to clause 8 in which the genes include any one or more, and preferably all, of: BRRN1; FLJ11029; C6orf173; STK6; MELK.

20. A method according to clause 14 in which the identified gene pairs include any one or more of SPAG5-ERCC6L, CENPE-CCNE2, CDCA8-CLDN5, and CCNA2-PTPRT and/or any one of more of (i) Megalin (LRP2) and itnergrin alpha 7 (ITGA7), (ii) NUDT1 and NMU genes and (iii) HN1 and CACNA1D.

21. A kit, such as a microarray, for detecting the expression level of a set of genes, the set having no more than 1000 member, or no more than 100 members, or no more than 20 members, and including

(a) at least one of BRRN1; FLJ11029; C6orf173; STK6; MELK; and/or
(b) at least one of the pairs: (i) SPAG5-ERCC6L, (ii) CENPE-CCNE2, (iii) CDCA8-CLDN5, (iv) CCNA2-PTPRT, (v) Megalin (LRP2) and itnergrin alpha 7 (ITGA7), (vi) NUDT1 and NMU genes, and (vii) HN1 and CACNA1D.

22. A novel concept of survival significant genes, gene pairs and gene signatures is developed based on the DDg method which allows discovering many novel hidden subgroups of the subjects and simultaneously to identify clinically essential biomarkers of diseases, including breast cancer. Method might be used for analysis of any medical conditions associated with clinical laboratory measures and time dynamics of the condition in the subjects (patients).

23. Compared to single survival significant genes, gene pairs showed highly significant p-values of Wald statistic ($\sim 10^{-8}$ vs $\sim 10^{-5}$). This result implied that the pairing procedure itself should be considered as a unique statistical tool for identification of patients with poor and good prognoses.

24. Strong enrichment of mitotic cetromere and chromosome segregation genes among top-level survival significant genes allows us to suggest novel prognostic and diagnostic panel of expressed genes associated with breast cancer (see Table 3 and Discussion). All of these genes are significant in context of most critical end-point of any disease-survival of the patients.

25. Gene pairs SPAG5-ERCC6L, CENPE-CCNE2, CDCA8-CLDN5, CCNA2-PTPRT playing an important role in dynamics of chromosomes during cell mitosis. Thus, these pairs consist of a novel type of gene signature, which member are synergistically associated in pairs and in combination provide reliable partition of the breast cancer patients onto low- and high-risk disease relapse sub-groups.

26. Together with the 5-gene genetic grade signature genes they form an integrative functional module. This module integrated with pairs SPAG5-ERCC6L, CENPE-CCNE2, CDCA8-CLDN5, CCNA2-PTPRT consists a set for more reliable grouping of the patients. Our data strongly suggest that this functional module could be essential for breast cancer prognosis and prediction and target genes/products for drug discovery.

27. DDg method reveals that genes of the 5-gene genetic breast cancer grading signature ((BRRN1 (212949_at, p=1.4E-03); FLJ11029 (2s28273_at, p=1.7E-04); C6orf173 (226936_at, p=6.2E-04); STK6 (208079_s_at, p=3.4E-04; 204092_s_at, p=6.4E-04), MELK (204825_at, p=1.2E-05)) along of in combination with other survival significant genes could consist a cancer-specific functional module(s) of prognosis of survival significance of the breast cancer patients.

28. FLJ11029 (2s28273_at) is a novel non-coding gene which is strongly survival significant in breast cancer, which could be used as novel biomarker in clinical application and a target for anti-gene therapy.

29. Megalin (LRP2) and itnergrin alpha 7 (ITGA7) could be considered as molecular partners in anti-cancer regulation and discussed as novel markers of breast cancer prognosis and classification.

30. Our results suggest important role of NUDT1 and NMU genes in breast cancer progression and the breast patient's survival. This pair could be considered as novel prognostic and predictive pair of biomarkers of breast cancer.

31. HN1 and CACNA1 D are the genes which importance for breast cancer is predited by 2D DDg method.

32. Our final step of goodness-off split (DDg) analysis involves determining the patients' composite group based on the final list of survival significant genes and synergistic survival significant gene pairs identified by our patient' grouping algorithms. In other words, we combine the information from all significant genes of Tables 3 into a final set of genes which could be used to test is there a given patient identified as a member of the two biologically and clinically distinct patients' groups.

## Claims

**1.** A kit, such as a microarray, for detecting the expression level of a set of genes, the set having no more than 1000 members, or no more than 100 members, or no more than 20 members, and including

(a) at least one of BRRN1; FLJ11029; C6orf173; STK6; MELK; and/or
(b) at least one of the pairs: (i) SPAG5-ERCC6L, (ii) CENPE-CCNE2, (iii) CDCA8-CLDN5, (iv) CCNA2-PTPRT, (v) Megalin (LRP2) and integrin alpha 7 (ITGA7), (vi) NUDT1 and NMU genes, and (vii) HN1 and CACNA1 D.

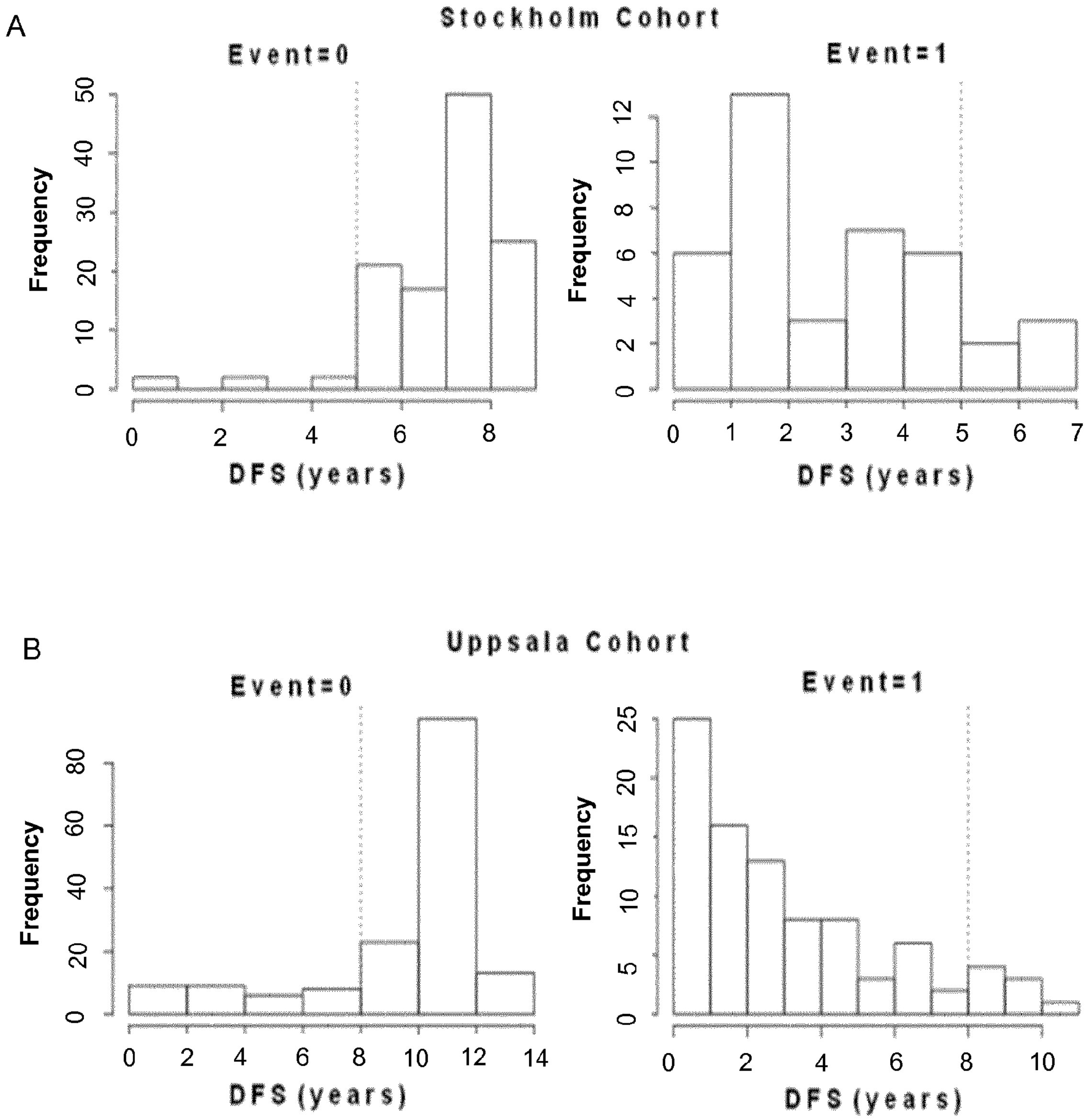
A
Stockholm Cohort
Event=0
Frequency
0
10
20
30
40
50
0
2
4
6
8
DFS (years)
Event=1
Frequency
0
2
4
6
8
10
12
0
1
2
3
4
5
6
7
DFS (years)
B
Uppsala Cohort
Event=0
Frequency
0
20
40
60
80
0
2
4
6
8
10
12
14
DFS (years)
Event=1
Frequency
0
5
10
15
20
25
0
2
4
6
8
10
DFS (years)

Figure 1

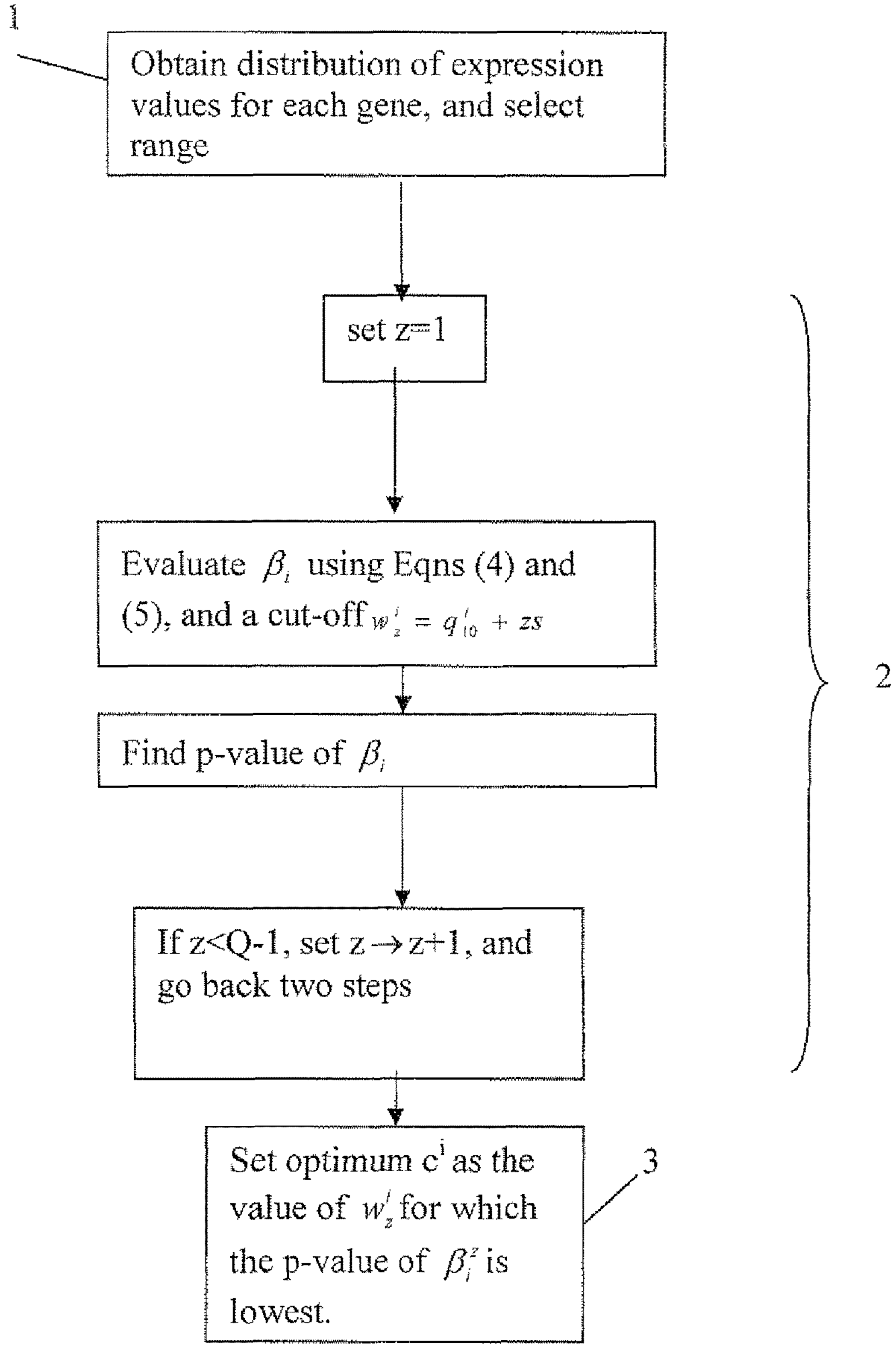
1
Obtain distribution of expression values for each gene, and select range
set z=1
Evaluate $\beta_i$ using Eqns (4) and (5), and a cut-off $w_z^i = q_{10}^i + zs$
Find p-value of $\beta_i$
If z<Q-1, set z→z+1, and go back two steps
2
Set optimum $c^i$ as the value of $w_z^i$ for which the p-value of $\beta_i^z$ is lowest.
3

Figure 2

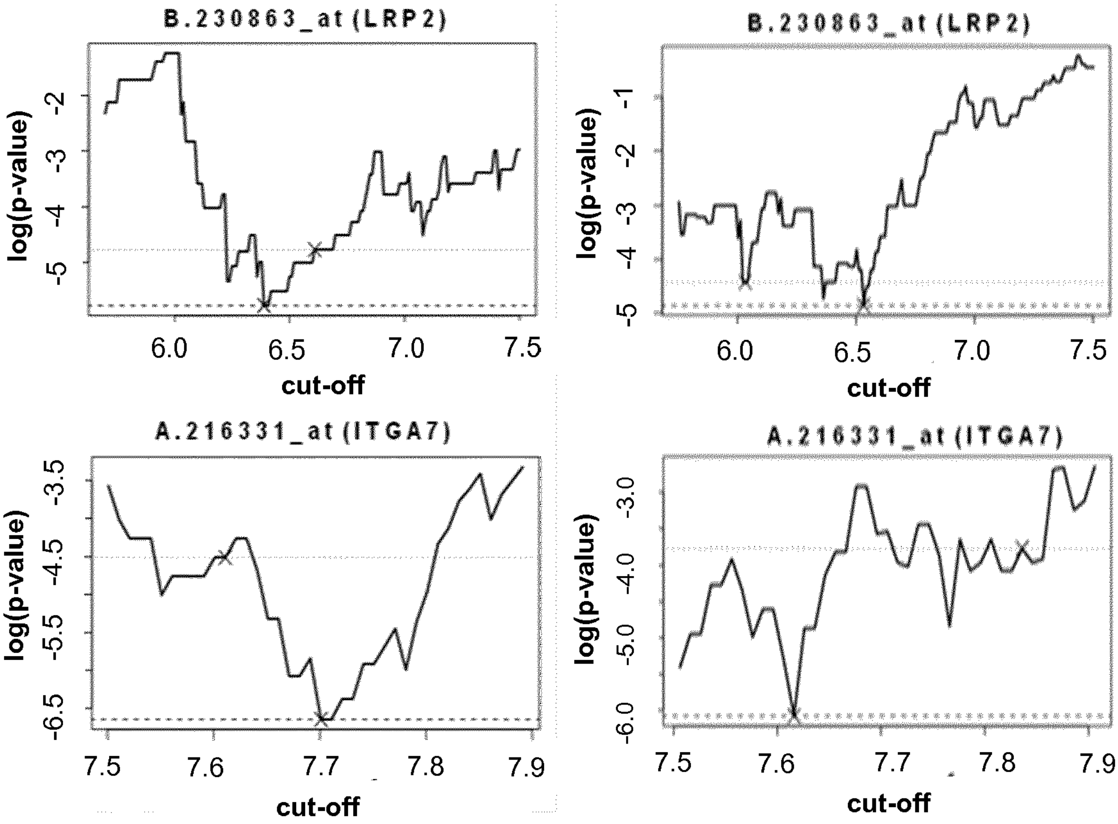


(a) (b)

Figure 3

$c^i$

| Gene j | High | C | D | $c^j$ |
|---|---|---|---|---|
| | Low | A | B | |
| | Signal | Low | High | |

Gene i

Figure 4

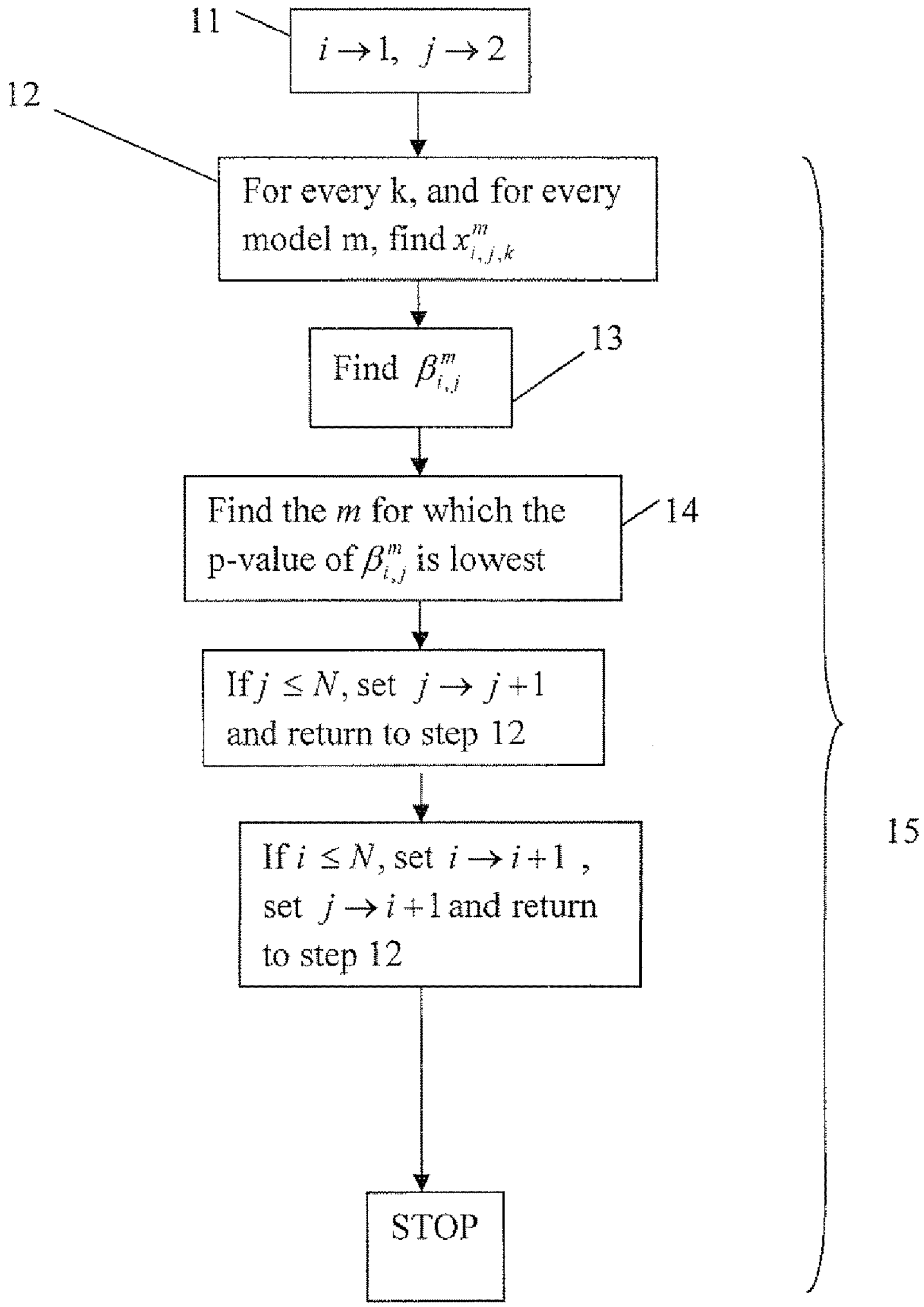
11
$i \to 1, \ j \to 2$
12
For every k, and for every model m, find $x^m_{i,j,k}$
Find $\beta^m_{i,j}$
13
Find the *m* for which the p-value of $\beta^m_{i,j}$ is lowest
14
If $j \le N$, set $j \to j+1$ and return to step 12
If $i \le N$, set $i \to i+1$ , set $j \to i+1$ and return to step 12
15
STOP

Figure 5

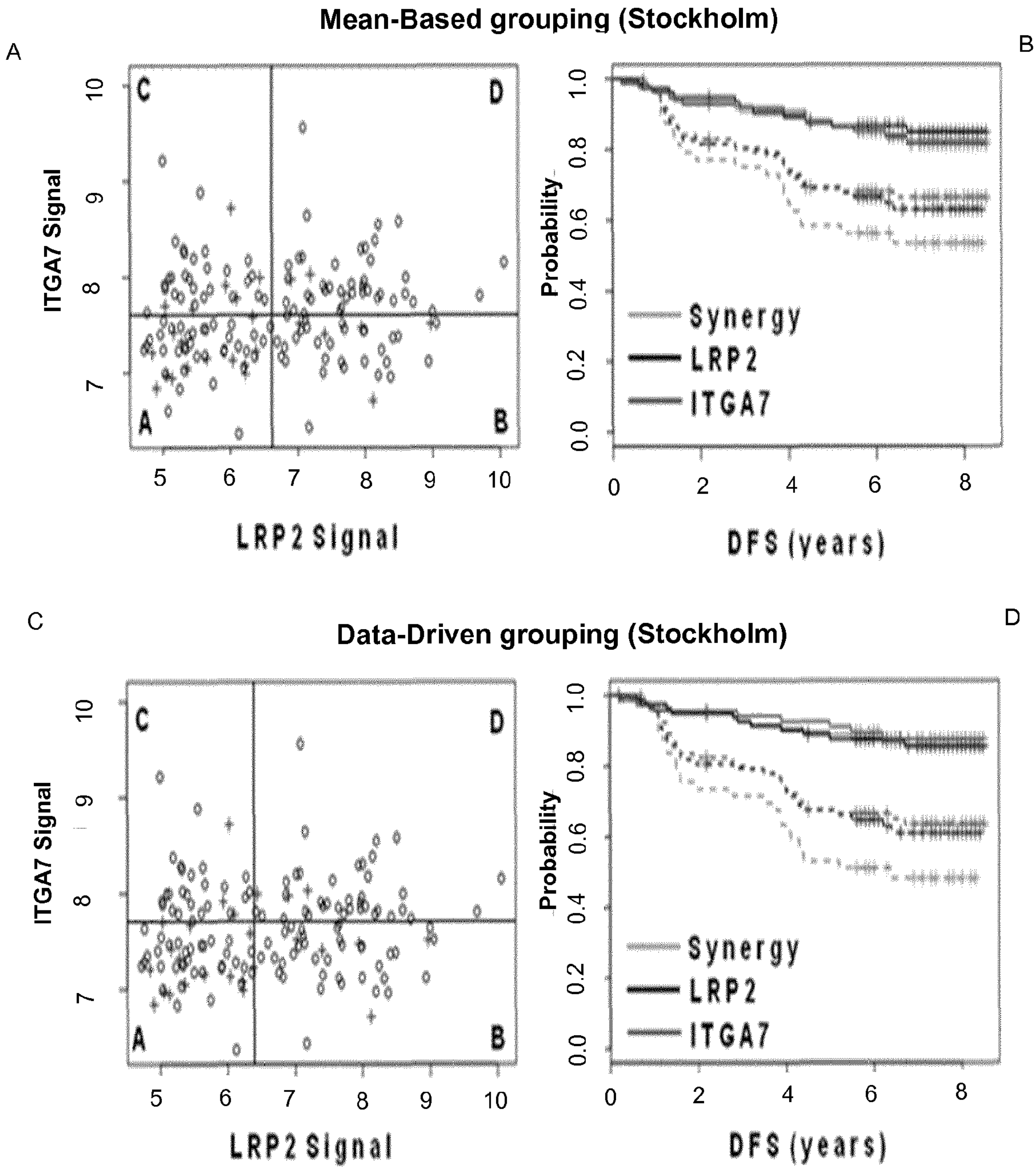
Mean-Based grouping (Stockholm)
A
B
ITGA7 Signal
LRP2 Signal
Probability
DFS (years)
Synergy
LRP2
ITGA7
C
D
Data-Driven grouping (Stockholm)

Figure 6

E

Data-Driven grouping (Stockholm)

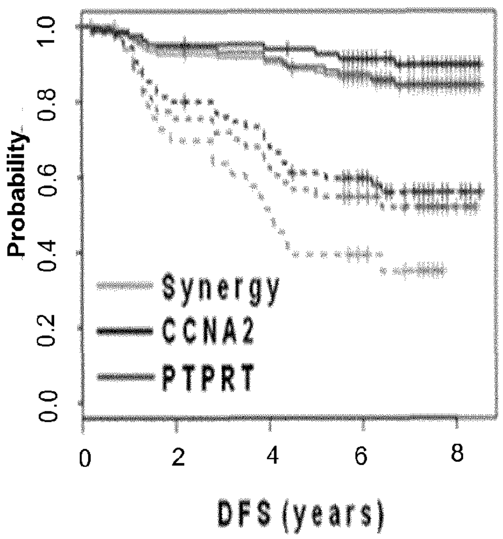


F

Mean-Based grouping (Stockholm)

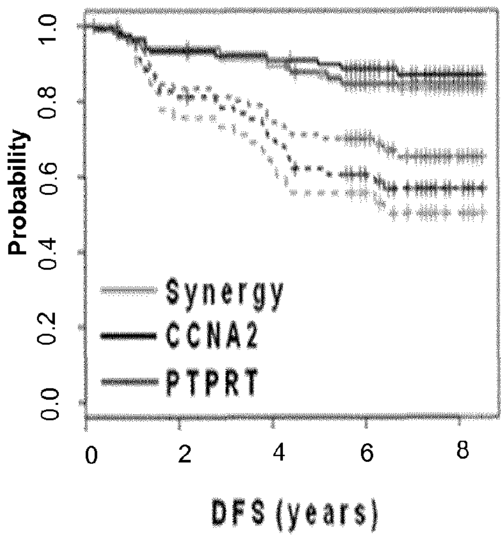


G

| Affy ID[1](gene name) | Affy ID[2](gene name) | $P_{DD}$(group) | $P_{DD}$ (ID[1]) | $P_{DD}$ (ID[2]) | $P_{MB}$(group) |
|---|---|---|---|---|---|
| B.230863_at(LRP2) | A.216331_at(ITGA7) | 2.5E-06(4) | 9.8E-04 | 1.9E-03 | 1.6E-04(4) |
| A.203418_at(CCNA2) | A.205948_at(PTPRT) | 4.0E-08(3) | 1.8E-05 | 1.4E-05 | 3.9E-05(3) |

Figure 6 (continued)

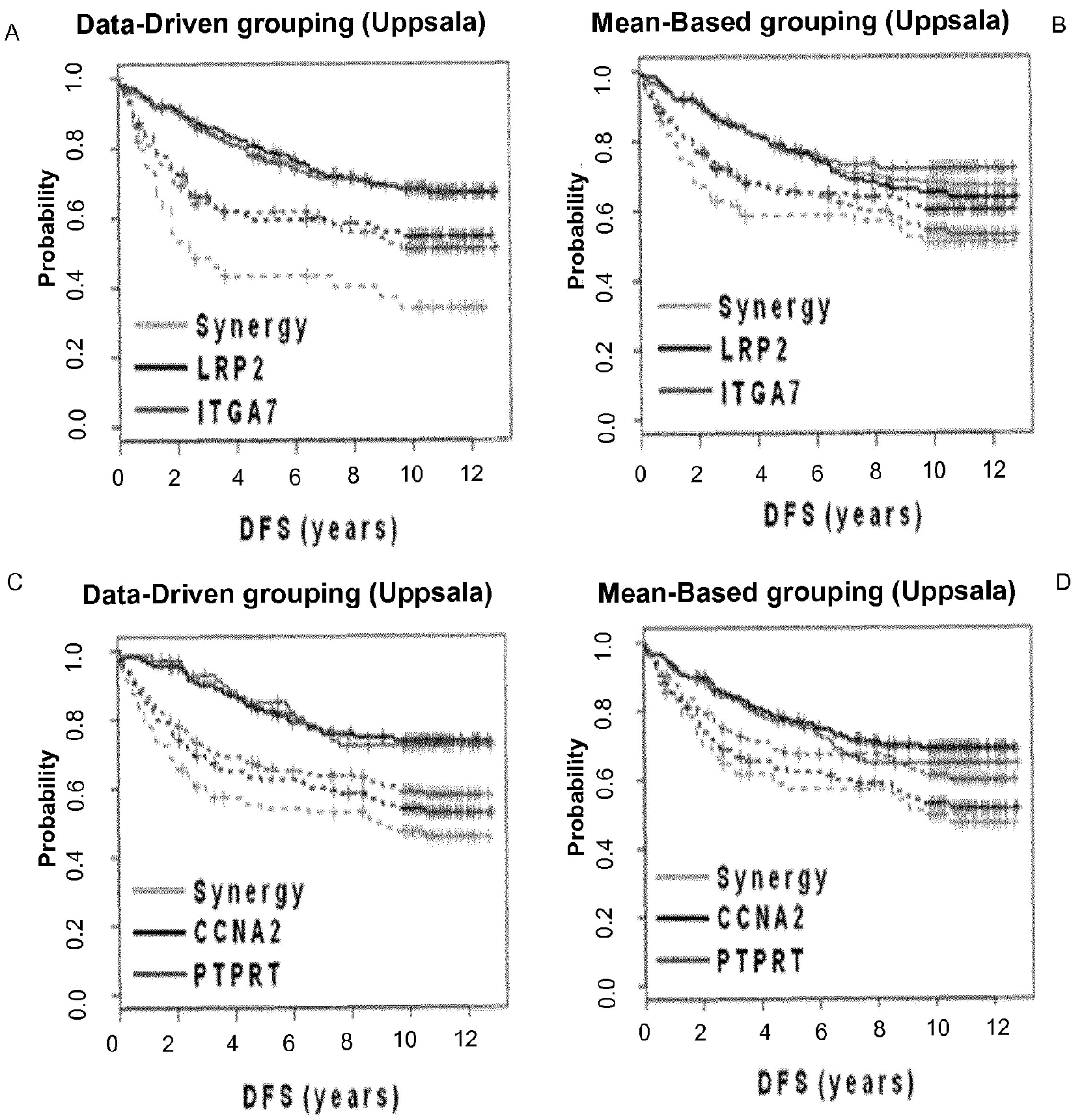


E

| Affy ID[1] (gene name) | Affy ID[2] (gene name) | $P_{DD}$(group) | $P_{DD}$ (ID[1]) | $P_{DD}$(ID[2]) | $P_{MB}$(group) |
|---|---|---|---|---|---|
| B.230863_at(LRP2) | A.216331_at(ITGA7) | 3.0E-07(4) | 1.0e-02 | 3.5E-03 | 1.6E-03(4) |
| A.203418_at(CCNA2) | A.205948_at(PTPRT) | 1.4E-06(3) | 5.7E-04 | 2.2E-02 | 1.4E-03(3) |

Figure 7

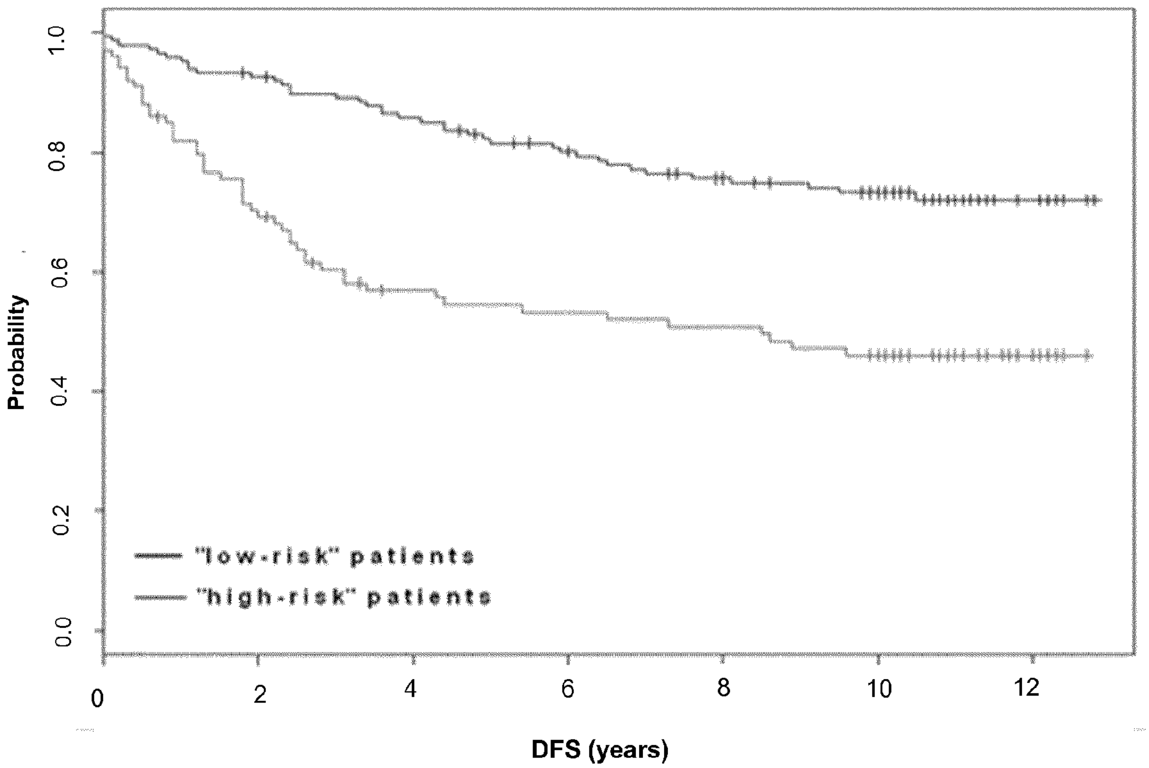
1.0
0.8
0.6
0.4
0.2
0.0
Probability
"low-risk" patients
"high-risk" patients
0
2
4
6
8
10
12
DFS (years)

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61158948 B
- SG 2009016825


### Non-patent literature cited in the description


- **BRESLOW, N.E.** Covariance analysis of censored survival data. *Biometrics,* 1974, vol. 30, 89-99
- **COX DR.** Regression Models and Life Tables (with Discussion). *Journal of the Royal Statistical Society Series B,* 1972, vol. 34, 187-220
- **COX, D.R. ; SNELL, E.J.** A general definition of residuals (with discussion). *Journal of the Royal Statistical Society, Series B,* 1968, vol. 30, 248-265
- **COX R.D. ; OAKES, D.** Analysis of Survival Data. Chapman and Hall, 1984
- **EFRON, B. ; TIBSHIRANI, R.J.** An Introduction to the Bootstrap. Chapman and Hall, 1994
- **HORI T ; AMANO M ; SUZUKI A ; BACKER CB ; WELBURN JP ; DONG Y ; MCEWEN BF ; SHANG WH ; SUZUKI E ; OKAWA K.** CCAN makes multiple contacts with centromeric DNA to provide distinct pathways to the outer kinetochore. *Cell,* 12 December 2008, vol. 135 (6), 1039-52
- **IVSHINA, A.V. ; GEORGE, J. ; SENKO, O et al.** Genetic reclassification of histologic grade delineates new clinical subtypes of breast cancer. *Cancer Research,* 2006, vol. 66, 10292-10301
- **KAPLAN, E. L. ; MEIER, P.** Nonparametric estimation from incomplete observations. *JASA,* 1958, vol. 53, 457-48
- **KIM H ; LEE M ; LEE S ; PARK B ; KOH W ; LEE DJ ; LIM DS ; LEE S.** Cancer-upregulated gene 2 (CUG2), a new component of centromere complex, is required for kinetochore function. *Mol Cells. 2009 Jun,* 12 June 2009, vol. 27 (6), 697-701
- **KUZNETSOV, V.A. ; SENKO, O.V. ; MILLER, L.D. ; IVSHINA, A.** Statistically Weighted Voting Analysis of Microarrays for Molecular Pattern Selection and Discovery Cancer Genotypes. *International Journal of Computer Science and Network Security,* 2006, vol. 6, 73-83
- **LEE S ; GANG J ; JEON SB ; CHOO SH ; LEE B ; KIM YG ; LEE YS ; JUNG J ; SONG SY ; KOH SS.** Molecular cloning and functional analysis of a novel oncogene, cancer-upregulated gene 2 (CUG2). *Biochem Biophys Res Commun. 2007 Aug 31,* 28 June 2007, vol. 360 (3), 633-9
- **LOUGHIN, T.M.** A residual bootstrap for regression parameters in proportional hazards model. *J. of Statistical and Computational Simulations,* 1995, vol. 52, 367-384
- **MOTAKIS, E. ; NASON, G.P. ; FRYZLEWICZ, P. ; RUTTER, G.A.** Variance stabilization and normalization for one-color microarray data using a data-driven multiscale approach. *Bioinformatics,* 2006, vol. 22, 2547-2553
- **MOTAKIS E ; IVSHINA AV ; KUZNETSOV VA.** Data-driven approach to predict survival of cancer patients: estimation of microarray genes' prediction significance by Cox proportional hazard regression model. *IEEE Eng Med Biol Mag,* July 2009, vol. 28 (4), 58-66
- **MILLENAAR, F. F. ; OKYERE, J. ; MAY, S.T. ; VAN ZANTEN, M. ; VOESENEK, L.A.C.J. ; PETERS, A.J.M.** How to decide? Different methods of calculating gene expression from short oligonucleotide array data will give different results. *BMC Bioinformatics,* 2006, vol. 7 (137
- **PAWITAN, Y. ; BJOHLE, J. ; AMLER, L.** Gene expression profiling spares early breast cancer patients from adjuvant therapy: derived and validated in two population-based cohorts. *Breast Cancer Research,* 2005, vol. 7, R953-R964
- **PRESS, W.H. ; FLANNERY, B.P. ; TEUKOLSKY, S.A ; VETTERLING, W.T.** Numerical Recipes in C: The Art of Scientific Computing. Cambridge University Press, 1992
- **SAMBROOK ; RUSSEL.** Molecular Cloning: A Laboratory Manual. Cold Springs Harbor Laboratory, 2001

SEQUENCE LISTING

<110> Agency for Science, Technology and Research

<120> Identification of biologically and clinically essential genes and gene pairs, and methods employing the identified genes and gene pairs

<130> PX210369EPA

<150> US 61/158,948
<151> 2009-03-10

<160> 20

<170> PatentIn version 3.4

<210> 1
<211> 15735
<212> DNA
<213> Homo sapiens

<400> 1
ggtctaaagg gctttatgca ctgtctggag ggtggggact ggcgcgggta gaaaacggga 60

tgcctcgggc gtgggggcag gcttttggcc actaggagct ggcggaggtg cagacctaaa 120

ggagcgttcg ctagcagagg cgctgccggt gcggtgtgct acgcgcgccc acctcccggg 180

gaaggaacgg cgaggccggg gaccgtcgcg gagatggatc gcgggccggc agcagtggcg 240

tgcacgctgc tcctggctct cgtcgcctgc ctagcgccgg ccagtggcca agaatgtgac 300

agtgcgcatt ttcgctgtgg aagtgggcat tgcatccctg cagactggag gtgtgatggg 360

accaaagact gttcagatga cgcggatgaa attggctgcg ctgttgtgac ctgccagcag 420

ggctatttca agtgccagag tgagggacaa tgcatcccca actcctgggt gtgtgaccaa 480

gatcaagact gtgatgatgg ctcagatgaa cgtcaagatt gctcacaaag tacatgctca 540

agtcatcaga taacatgctc caatggtcag tgtatcccaa gtgaatacag gtgcgaccac 600

gtcagagact gccccgatgg agctgatgag aatgactgcc agtacccaac atgtgagcag 660

cttacttgtg acaatggggc ctgctataac accagtcaga agtgtgattg gaaagttgat 720

tgcagggact cctcagatga aatcaactgc actgagatat gcttgcacaa tgagttttca 780

tgtggcaatg gagagtgtat ccctcgtgct tatgtctgtg accatgacaa tgattgccaa 840

gacggcagtg acgaacatgc ttgcaactat ccgacctgcg gtggttacca gttcacttgc 900

cccagtggcc gatgcattta tcaaaactgg gtttgtgatg gagaagatga ctgtaaagat 960

aatggagatg aagatggatg tgaaagcggt cctcatgatg ttcataaatg ttccccaaga 1020

gaatggtctt gcccagagtc gggacgatgc atctccattt ataaagtttg tgatgggatt 1080

ttagattgcc caggaagaga agatgaaaac aacactagta ccggaaaata ctgtagtatg 1140

actctgtgct ctgccttgaa ctgccagtac cagtgccatg agacgccgta tggaggagcg 1200

tgtttttgtc ccccaggtta tatcatcaac cacaatgaca gccgtacctg tgttgagttt 1260

gatgattgcc agatatgggg aatttgtgac cagaagtgtg aaagccgacc tggccgtcac 1320

ctgtgccact gtgaagaagg gtatatcttg gagcgtggac agtattgcaa agctaatgat 1380

tcctttggcg aggcctccat tatcttctcc aatggtcggg atttgttaat tggtgatatt 1440

catggaagga gcttccggat cctagtggag tctcagaatc gtggagtggc cgtgggtgtg 1500

gctttccact atcacctgca aagagttttt tggacagaca ccgtgcaaaa taaggttttt 1560

tcagttgaca ttaatggttt aaatatccaa gaggttctca atgtttctgt tgaaacccca 1620

gagaacctgg ctgtggactg ggttaataat aaaatctatc tagtggaaac caaggtcaac 1680

cgcatagata tggtaaattt ggatggaagc tatcgggtta cccttataac tgaaaacttg 1740

gggcatccta gaggaattgc cgtggaccca actgttggtt atttattttt ctcagattgg 1800

gagagccttt ctggggaacc taagctggaa agggcattca tggatggcag caaccgtaaa 1860

gacttggtga aacaaagct gggatggcct gctggggtaa ctctggatat gatatcgaag 1920

cgtgtttact gggttgactc tcggtttgat tacattgaaa ctgtaactta tgatggaatt 1980

caaaggaaga ctgtagttca tggaggctcc ctcattcctc atccctttgg agtaagctta 2040

tttgaaggtc aggtgttctt tacagattgg acaaagatgg ccgtgctgaa ggcaaacaag 2100

ttcacagaga ccaacccaca agtgtactac caggcttccc tgaggcccta tggagtgact 2160

gtttaccatt ccctcagaca gccctatgct accaatccgt gtaaagataa caatgggggc 2220

tgtgagcagg tctgtgtcct cagccacaga acagataatg atggtttggg tttccgttgc 2280

aagtgcacat tcggcttcca actggataca gatgagcgcc actgcattgc tgttcagaat 2340

ttcctcattt tttcatccca agttgctatt cgtgggatcc cgttcacctt gtctacccag 2400

gaagatgtca tggttccagt ttcggggaat ccttctttct ttgtcgggat tgattttgac 2460

gcccaggaca gcactatctt tttttcagat atgtcaaaac acatgatttt taagcaaaag 2520

attgatggca caggaagaga aattctcgca gctaacaggg tggaaaatgt tgaaagtttg 2580

gcttttgatt ggatttcaaa gaatctctat tggacagact ctcattacaa gagtatcagt 2640

gtcatgaggc tagctgataa aacgagacgc acagtagttc agtatttaaa taacccacgg 2700

tcggtggtag ttcatccttt tgccgggtat ctattcttca ctgattggtt ccgtcctgct 2760

aaaattatga gagcatggag tgacggatct cacctcttgc ctgtaataaa cactactctt 2820

ggatggccca atggcttggc catcgattgg gctgcttcac gattgtactg ggtagatgcc 2880

tattttgata aaattgagca cagcaccttt gatggtttag acagaagaag actgggccat 2940

atagagcaga tgacacatcc gtttggactt gccatctttg gagagcattt attttttact 3000

gactggagac tgggtgccat tattcgagtc aggaaagcag atggtggaga aatgacagtt 3060

atccgaagtg gcattgctta catactgcat ttgaaatcgt atgatgtcaa catccagact 3120
ggttctaacg cctgtaatca acccacgcat cctaacggtg actgcagcca cttctgcttc 3180
ccggtgccaa atttccagcg agtgtgtggg tgcccttatg gaatgaggct ggcttccaat 3240
cacttgacat gcgaggggga cccaaccaat gaaccaccca cagagcagtg tggcttattt 3300
tccttcccct gtaaaaatgg cagatgtgtg cccaattact atctctgtga tggagtcgat 3360
gattgtcatg ataacagtga tgagcaacta tgtggcacac ttaataatac ctgttcatct 3420
tcggcgttca cctgtggcca tggggagtgc attcctgcac actggcgctg tgacaaacgc 3480
aacgactgtg tggatggcag tgatgagcac aactgcccca cccacgcacc tgcttcctgc 3540
cttgacaccc aatacacctg tgataatcac cagtgtatct caaagaactg ggtctgtgac 3600
acagacaatg attgtgggga tggatctgat gaaaagaact gcaattcgac agagacatgc 3660
caacctagtc agtttaattg ccccaatcat cgatgtattg acctatcgtt tgtctgtgat 3720
ggtgacaagg attgtgttga tggatctgat gaggttggtt gtgtattaaa ctgtactgct 3780
tctcaattca agtgtgccag tggggataaa tgtattggcg tcacaaatcg ttgtgatggt 3840
gtttttgatt gcagtgacaa ctcggatgaa gcaggctgtc caaccaggcc tcctggtatg 3900
tgccactcag atgaatttca gtgccaagaa gatggtatct gcatcccgaa cttctgggaa 3960
tgtgatgggc atccagactg cctctatgga tctgatgagc acaatgcctg tgtccccaag 4020
acttgccctt catcatattt ccactgtgac aacggaaact gcatccacag ggcatggctc 4080
tgtgatcggg acaatgactg cggggatatg agtgatgaga aggactgccc tactcagccc 4140
tttcgctgtc ctagttggca atggcagtgt cttggccata acatctgtgt gaatctgagt 4200
gtagtgtgtg atggcatctt tgactgcccc aatgggacag atgagtcccc actttgcaat 4260
gggaacagct gctcagattt caatggtggt tgtactcacg agtgtgttca agagcccttt 4320
ggggctaaat gcctatgtcc attgggattc ttacttgcca atgattctaa gacctgtgaa 4380
gacatagatg aatgtgatat tctaggctct tgtagccagc actgttacaa tatgagaggt 4440
tctttccggt gctcgtgtga tacaggctac atgttagaaa gtgatgggag gacttgcaaa 4500
gttacagcat ctgagagtct gctgttactt gtggcaagtc agaacaaaat tattgccgac 4560
agtgtcacct cccaggtcca caatatctat tcattggtcg agaatggttc ttacattgta 4620
gctgttgatt ttgattcaat tagtggtcgt atcttttggt ctgatgcaac tcagggtaaa 4680
acctggagtg cgtttcaaaa tggaacggac agaagagtgg tatttgacag tagcatcatc 4740
ttgactgaaa ctattgcaat agattgggta ggtcgtaatc tttactggac agactatgct 4800
ctggaaacaa ttgaagtctc caaaattgat gggagccaca ggactgtgct gattagtaaa 4860
aacctaacaa atccaagagg actagcatta gatcccagaa tgaatgagca tctactgttc 4920
tggtctgact ggggccacca ccctcgcatc gagcgagcca gcatggacgg cagcatgcgc 4980

actgtcattg tccaggacaa gatcttctgg ccctgcggct taactattga ctaccccaac 5040
agactgctct acttcatgga ctcctatctt gattacatgg acttttgtga ttataatgga 5100
caccatcgga gacaggtgat agccagtgat ttgattatac ggcaccccta tgccctaact 5160
ctctttgaag actctgtgta ctggactgac cgtgctactc gtcgggttat gcgagccaac 5220
aagtggcatg gagggaacca gtcagttgta atgtataata ttcaatggcc ccttgggatt 5280
gttgcggttc atccttcgaa acaaccaaat tccgtgaatc catgtgcctt ttcccgctgc 5340
agccatctct gcctgctttc ctcacagggg cctcattttt actcctgtgt ttgtccttca 5400
ggatggagtc tgtctcctga tctcctgaat tgcttgagag atgatcaacc tttcttaata 5460
actgtaaggc aacatataat ttttggaatc tcccttaatc ctgaggtgaa gagcaatgat 5520
gctatggtcc ccatagcagg gatacagaat ggtttagatg ttgaatttga tgatgctgag 5580
caatacatct attgggttga aaatccaggt gaaattcaca gagtgaagac agatggcacc 5640
aacaggacag tatttgcttc tatatctatg gtggggcctt ctatgaacct ggccttagat 5700
tggatttcaa gaaaccttta ttctaccaat cctagaactc agtcaatcga ggttttgaca 5760
ctccacggag atatcagata cagaaaaaca ttgattgcca atgatgggac agctcttgga 5820
gttggctttc caattggcat aactgttgat cctgctcgtg ggaagctgta ctggtcagac 5880
caaggaactg acagtggggt tcctgccaag atcgccagtg ctaacatgga tggcacatct 5940
gtgaaaactc tctttactgg gaacctcgaa cacctggagt gtgtcactct tgacatcgaa 6000
gagcagaaac tctactgggc agtcactgga agaggagtga ttgaaagagg aaacgtggat 6060
ggaacagatc gaatgatcct ggtacaccag ctttcccacc cctggggaat tgcagtccat 6120
gattctttcc tttattatac tgatgaacag tatgaggtca ttgaaagagt tgataaggcc 6180
actggggcca acaaaatagt cttgagagat aatgttccaa atctgagggg tcttcaagtt 6240
tatcacagac gcaatgccgc cgaatcctca aatggctgta gcaacaacat gaatgcctgt 6300
cagcagattt gcctgcctgt accaggagga ttgttttcct gcgcctgtgc cactggattt 6360
aaactcaatc ctgataatcg gtcctgctct ccatataact ctttcattgt tgtttcaatg 6420
ctgtctgcaa tcagaggctt tagcttggaa ttgtcagatc attcagaaac catggtgccg 6480
gtggcaggcc aaggacgaaa cgcactgcat gtggatgtgg atgtgtcctc tggctttatt 6540
tattggtgtg attttagcag ctcagtggca tctgataatg cgatccgtag aattaaacca 6600
gatggatctt ctctgatgaa cattgtgaca catggaatag gagaaaatgg agtccggggt 6660
attgcagtgg attgggtagc aggaaatctt tatttcacca atgcctttgt ttctgaaaca 6720
ctgatagaag ttctgcggat caatactact taccgccgtg ttcttcttaa agtcacagtg 6780
gacatgccta ggcatattgt tgtagatccc aagaacagat acctcttctg ggctgactat 6840

gggcagagac caaagattga gcgttctttc cttgactgta ccaatcgaac agtgcttgtg 6900
tcagagggca ttgtcacacc acggggcttg gcagtggacc gaagtgatgg ctacgtttat 6960
tgggttgatg attctttaga tataattgca aggattcgta tcaatggaga gaactctgaa 7020
gtgattcgtt atggcagtcg ttacccaact ccttatggca tcactgtttt tgaaaattct 7080
atcatatggg tagataggaa tttgaaaaag atcttccaag ccagcaagga accagagaac 7140
acagagccac ccacagtgat aagagacaat atcaactggc taagagatgt gaccatcttt 7200
gacaagcaag tccagccccg gtcaccagca gaggtcaaca acaacccttg cttggaaaac 7260
aatggtgggt gctctcatct ctgctttgct ctgcctggat tgcacacccc aaaatgtgac 7320
tgtgcctttg ggaccctgca aagtgatggc aagaattgtg ccatttcaac agaaaatttc 7380
ctcatctttg ccttgtctaa ttccttgaga agcttacact tggaccctga aaaccatagc 7440
ccacctttcc aaacaataaa tgtggaaaga actgtcatgt ctctagacta tgacagtgta 7500
agtgatagaa tctacttcac acaaaattta gcctctggag ttggacagat ttcctatgcc 7560
accctgtctt cagggatcca tactccaact gtcattgctt caggtatagg gactgctgat 7620
ggcattgcct ttgactggat tactagaaga atttattaca gtgactacct caaccagatg 7680
attaattcca tggctgaaga tgggtctaac cgcactgtga tagcccgcgt tccaaaacca 7740
agagcaattg tgttagatcc ctgccaaggg tacctgtact gggctgactg ggatacacat 7800
gccaaaatcg agagagccac attgggagga aacttccgcg tacccattgt gaacagcagt 7860
ctggtcatgc ccagtgggct gactctggac tatgaagagg accttctcta ctgggtggat 7920
gctagtctgc agaggattga acgcagcact ctgacgggcg tggatcgtga agtcattgtc 7980
aatgcagccg ttcatgcttt tggcttgact ctctatggcc agtatattta ctggactgac 8040
ttgtacacac aaagaattta ccgagctaac aaatatgacg ggtcaggtca gattgcaatg 8100
accacaaatt tgctctccca gcccagggga atcaacactg ttgtgaagaa ccagaaacaa 8160
cagtgtaaca atccttgtga acagtttaat gggggctgca gccatatctg tgcaccaggt 8220
ccaaatggtg ccgagtgcca gtgtccacat gagggcaact ggtatttggc caacaacagg 8280
aagcactgca ttgtggacaa tggtgaacga tgtggtgcat cttccttcac ctgctccaat 8340
gggcgctgca tctcggaaga gtggaagtgt gataatgaca acgactgtgg ggatggcagt 8400
gatgagatgg aaagtgtctg tgcacttcac acctgctcac cgacagcctt cacctgtgcc 8460
aatgggcgat gtgtccaata ctcttaccgc tgtgattact acaatgactg tggtgatggc 8520
agtgatgagg cagggtgcct gttcagggac tgcaatgcca ccacggagtt tatgtgcaat 8580
aacagaaggt gcatacctcg tgagtttatc tgcaatggtg tagacaactg ccatgataat 8640
aacacttcag atgagaaaaa ttgccctgat cgcacttgcc agtctggata cacaaaatgt 8700
cataattcaa atatttgtat tcctcgcgtt tatttgtgtg acggagacaa tgactgtgga 8760

gataacagtg atgaaaaccc tacttattgc accactcaca cgtgcagcag cagtgagttc 8820
caatgcgcat ctgggcgctg tattcctcaa cattggtatt gtgatcaaga aacagattgt 8880
tttgatgcct ctgatgaacc tgcctcttgt ggtcactctg agcgaacatg cctagctgat 8940
gagttcaagt gtgatggtgg gaggtgcatc ccaagcgaat ggatctgtga cggtgataat 9000
gactgtgggg atatgagtga cgaggataaa aggcaccagt gtcagaatca aaactgctcg 9060
gattccgagt ttctctgtgt aaatgacaga cctccggaca ggaggtgcat tccccagtct 9120
tgggtctgtg atggcgatgt ggattgtact gacggctacg atgagaatca gaattgcacc 9180
aggagaactt gctctgaaaa tgaattcacc tgtggttacg gactgtgtat cccaaagata 9240
ttcaggtgtg accggcacaa tgactgtggt gactatagcg acgagagggg ctgcttatac 9300
cagacttgcc aacagaatca gtttacctgt cagaacgggc gctgcattag taaaaccttc 9360
gtctgtgatg aggataatga ctgtggagac ggatctgatg agctgatgca cctgtgccac 9420
accccagaac ccacgtgtcc acctcacgag ttcaagtgtg acaatgggcg ctgcatcgag 9480
atgatgaaac tctgcaacca cctagatgac tgtttggaca acagcgatga gaaaggctgt 9540
ggcattaatg aatgccatga cccttcaatc agtggctgcg atcacaactg cacagacacc 9600
ttaaccagtt tctattgttc ctgtcgtcct ggttacaagc tcatgtctga caagcggact 9660
tgtgttgata ttgatgaatg cacagagatg ccttttgtct gtagccagaa gtgtgagaat 9720
gtaataggct cctacatctg taagtgtgcc ccaggctacc tccgagaacc agatggaaag 9780
acctgccggc aaaacagtaa catcgaaccc tatctcattt ttagcaaccg ttactatttg 9840
agaaatttaa ctatagatgg ctatttttac tccctcatct tggaaggact ggacaatgtt 9900
gtggcattag attttgaccg agtagagaag agattgtatt ggattgatac acagaggcaa 9960
gtcattgaga gaatgtttct gaataagaca aacaaggaga caatcataaa ccacagacta 10020
ccagctgcag aaagtctggc tgtagactgg gtttccagaa agctctactg gttggatgcc 10080
cgcctggatg gcctctttgt ctctgacctc aatggtggac accgccgcat gctggcccag 10140
cactgtgtgg atgccaacaa caccttctgc tttgataatc ccagaggact tgcccttcac 10200
cctcaatatg ggtacctcta ctgggcagac tggggtcacc gcgcatacat tgggagagta 10260
ggcatggatg gaaccaacaa gtctgtgata atctccacca agttagagtg gcctaatggc 10320
atcaccattg attacaccaa tgatctactc tactgggcag atgcccacct gggttacata 10380
gagtactctg atttggaggg ccaccatcga cacacggtgt atgatggggc actgcctcac 10440
cctttcgcta ttaccatttt tgaagacact atttattgga cagattggaa tacaaggaca 10500
gtggaaaagg gaaacaaata tgatggatca aatagacaga cactggtgaa cacaacacac 10560
agaccatttg acatccatgt gtaccatcca tataggcagc ccattgtgag caatccctgt 10620

ggtaccaaca atggtggctg ttctcatctc tgcctcatca agccaggagg aaaagggttc 10680
acttgcgagt gtccagatga cttccgcacc cttcagctga gtggcagcac ctactgcatg 10740
cccatgtgct ccagcaccca gttcctgtgc gctaacaatg aaaagtgcat tcctatctgg 10800
tggaaatgtg atggacagaa agactgctca gatggctctg atgaactggc cctttgcccg 10860
cagcgcttct gccgactggg acagttccag tgcagtgacg gcaactgcac cagcccgcag 10920
actttatgca atgctcacca aaattgccct gatgggtctg atgaagaccg tcttctttgt 10980
gagaatcacc actgtgactc caatgaatgg cagtgcgcca acaaacgttg catcccagaa 11040
tcctggcagt gtgacacatt taacgactgt gaggataact cagatgaaga cagttcccac 11100
tgtgccagca ggacctgccg gccgggccag tttcggtgtg ctaatggccg ctgcatcccg 11160
caggcctgga agtgtgatgt ggataatgat tgtggagacc actcggatga gcccattgaa 11220
gaatgcatga gctctgccca tctctgtgac aacttcacag aattcagctg caaaacaaat 11280
taccgctgca tcccaaagtg ggccgtgtgc aatggtgtag atgactgcag ggacaacagt 11340
gatgagcaag gctgtgagga gaggacatgc catcctgtgg gggatttccg ctgtaaaaat 11400
caccactgca tccctcttcg ttggcagtgt gatgggcaaa atgactgtgg agataactca 11460
gatgaggaaa actgtgctcc ccgggagtgc acagagagcg agtttcgatg tgtcaatcag 11520
cagtgcattc cctcgcgatg gatctgtgac cattacaacg actgtgggga caactcagat 11580
gaacgggact gtgagatgag gacctgccat cctgaatatt ttcagtgtac aagtggacat 11640
tgtgtacaca gtgaactgaa atgcgatgga tccgctgact gtttggatgc gtctgatgaa 11700
gctgattgtc ccacacgctt tcctgatggt gcatactgcc aggctactat gttcgaatgc 11760
aaaaaccatg tttgtatccc gccatattgg aaatgtgatg gcgatgatga ctgtggcgat 11820
ggttcagatg aagaacttca cctgtgcttg gatgttccct gtaattcacc aaaccgtttc 11880
cggtgtgaca acaatcgctg catttatagt catgaggtgt gcaatggtgt ggatgactgt 11940
ggagatggaa ctgatgagac agaggagcac tgtagaaaac cgacccctaa accttgtaca 12000
gaatatgaat ataagtgtgg caatgggcat tgcattccac atgacaatgt gtgtgatgat 12060
gccgatgact gtggtgactg gtccgatgaa ctgggttgca ataaaggaaa agaaagaaca 12120
tgtgctgaaa atatatgcga gcaaaattgt acccaattaa atgaaggagg atttatctgc 12180
tcctgtacag ctgggttcga aaccaatgtt tttgacagaa cctcctgtct agatatcaat 12240
gaatgtgaac aatttgggac ttgtccccag cactgcagaa ataccaaagg aagttatgag 12300
tgtgtctgtg ctgatggctt cacgtctatg agtgaccgcc ctggaaaacg atgtgcagct 12360
gagggtagct ctcctttgtt gctactgcct gacaatgtcc gaattcgaaa atataatctc 12420
tcatctgaga ggttctcaga gtatcttcaa gatgaggaat atatccaagc tgttgattat 12480
gattgggatc ccaaggacat aggcctcagt gttgtgtatt acactgtgcg aggggagggc 12540

tctaggtttg gtgctatcaa acgtgcctac atccccaact ttgaatccgg ccgcaataat 12600

cttgtgcagg aagttgacct gaaactgaaa tacgtaatgc agccagatgg aatagcagtg 12660

gactgggttg gaaggcatat ttactggtca gatgtcaaga ataaacgcat tgaggtggct 12720

aaacttgatg gaaggtacag aaagtggctg atttccactg acctggacca accagctgct 12780

attgctgtga atcccaaact agggcttatg ttctggactg actggggaaa ggaacctaaa 12840

atcgagtctg cctggatgaa tggagaggac cgcaacatcc tggttttcga ggaccttggt 12900

tggccaactg gcctttctat cgattatttg aacaatgacc gaatctactg gagtgacttc 12960

aaggaggacg ttattgaaac cataaaatat gatgggactg ataggagagt cattgcaaag 13020

gaagcaatga acccttacag cctggacatc tttgaagacc agttatactg gatatctaag 13080

gaaaagggag aagtatggaa acaaaataaa tttgggcaag gaaagaaaga gaaaacgctg 13140

gtagtgaacc cttggctcac tcaagttcga atctttcatc aactcagata caataagtca 13200

gtgcccaacc tttgcaaaca gatctgcagc cacctctgcc ttctgagacc tggaggatac 13260

agctgtgcct gtccccaagg ctccagcttt atagagggga gcaccactga gtgtgatgca 13320

gccatcgaac tgcctatcaa cctgcccccc ccatgcaggt gcatgcacgg aggaaattgc 13380

tattttgatg agactgacct ccccaaatgc aagtgtccta gcggctacac cggaaaatat 13440

tgtgaaatgg cgttttcaaa aggcatctct ccaggaacaa ccgcagtagc tgtgctgttg 13500

acaatcctct tgatcgtcgt aattggagct ctggcaattg caggattctt ccactataga 13560

aggaccggct ccctttttgcc tgctctgccc aagctgccaa gcttaagcag tctcgtcaag 13620

ccctctgaaa atgggaatgg ggtgaccttc agatcagggg cagatcttaa catggatatt 13680

ggagtgtctg gttttggacc tgagactgct attgacaggt caatggcaat gagtgaagac 13740

tttgtcatgg aaatggggaa gcagcccata atatttgaaa acccaatgta ctcagccaga 13800

gacagtgctg tcaaagtggt tcagccaatc caggtgactg tatctgaaaa tgtggataat 13860

aagaattatg gaagtcccat aaacccttct gagatagttc cagagacaaa cccaacttca 13920

ccagctgctg atggaactca ggtgacaaaa tggaatctct tcaaacgaaa atctaaacaa 13980

actaccaact ttgaaaatcc aatctatgca cagatggaga acgagcaaaa ggaaagtgtt 14040

gctgcgacac cacctccatc accttcgctc cctgctaagc ctaagcctcc ttcgagaaga 14100

gacccaactc caacctattc tgcaacagaa gacactttta aagacaccgc aaatcttgtt 14160

aaagaagact ctgaagtata gctataccag ctatttaggg aataattaga aacacacttt 14220

tgcacatata tttttacaa acagatgaaa aaagttaaca ttcagtactt tatgaaaaaa 14280

atatattttt ccctgtttgc ctatagttgg aggtatcctg tgtgtctttt tttacttatg 14340

ccgtctcata tttttacaaa taattatcac aatgtactat atgtatatct ttgcactgaa 14400

gttgtctgaa ggtaatacta taaatatatt gtatatttgt aaattttgga aagattatcc 14460

tgttactgaa tttgctaata aagatgtctg ctgatttggt tggtgatcat tatagtaaat 14520

gatccaacaa gaaaaggaat tgactgggga cctttagccg tgtctaaaga agaggcacca 14580

ctcatatttc ctataaaatt atctaggaaa ggaatccagg ccccgctctt gggtccattt 14640

ttacacatta gcacttaatt aatgttcaat attacatgtc aatttgatta atggctatgt 14700

tgatagggc cactatgtgt tgtatagaca tctggacttg actgtagact cctcagataa 14760

tacagaaggt aggaaaagca attcagtttg gcccttctgt gtgttggcat tgtctaacca 14820

gaactctctg tttcatgtgt gttctctcac tagctgccaa gacaacattt ttatttgtga 14880

tgtctatgag gaaatcccat atcattaagt gccagtgtcc tgcattgagt ttgtggttaa 14940

ttaaatgagc tcttctgctg atggaccctg gagcaatttc tcccctcacc tgacattcaa 15000

ggtggtcacc tgccctagta gttggagctc agtagctgaa tttctgaaac caaatctgtg 15060

tcttcataaa ataaggtgca aaaaaaaaaa ataccagtta agtaaagcct caactgggtt 15120

tttgtttcta tgaaaatatc attataatca ctatttattt cctaagttga acctgaatag 15180

aaagggaaac cattcttatt aagcttttta ttaggccctg tggctaaatg tgtacattta 15240

tattagaatg tactgtacag tccagatctt ttctttaatt cttattggtt tttttttttt 15300

tttttttttt agagatggag tcttgctata ttgccaaggc tgatcttgaa gtcctgggct 15360

caagtgatcc tcccacctca gcctcctgag tggttggggt tacgggcgtg agccactgtg 15420

cctggcttcc agctctcctc ttaaatagtg ggtatagtct gcacaacagg aaccatggca 15480

ggaatataca ctttcccata gcaaatagca tacctgactc tctgtgctaa tattgcacat 15540

ttgttaaaca atgaatgaat ggatggatgg atggatggat gaatgaatga aacatatact 15600

actgattatt ttattccaga gttctcaaaa tatttgttgc tgatattttg agtgctgact 15660

gtaattactt tgattagata aacaactgga aataatgctg ctgaaaaagt tctaataaat 15720

gtgtatttta tcaga 15735

<210> 2
<211> 4138
<212> DNA
<213> Homo sapiens

<400> 2
gggcgccgga gctgcggctg ctgtagttgt cctagccggt gctggggcgg cggggtggcg 60

gagcggcggg cgggcgggag ggctggcggg gcgaacgtct gggagacgtc tgaaagacca 120

acgagacttt ggagaccaga gacgcgcctg gggggacctg gggcttgggg cgtgcgagat 180

ttcccttgca ttcgctggga gctcgcgcag ggatcgtccc atggccgggg ctcggagccg 240

cgacccttgg ggggcctccg ggatttgcta ccttttggc tccctgctcg tcgaactgct 300

```
cttctcacgg gctgtcgcct tcaatctgga cgtgatgggt gccttgcgca aggagggcga     360
gccaggcagc ctcttcggct tctctgtggc cctgcaccgg cagttgcagc cccgacccca     420
gagctggctg ctggtgggtg ctccccaggc cctggctctt cctgggcagc aggcgaatcg     480
cactggaggc ctcttcgctt gcccgttgag cctggaggag actgactgct acagagtgga     540
catcgaccag ggagctgata tgcaaaagga aagcaaggag aaccagtggt tgggagtcag     600
tgttcggagc caggggcctg gggcaagat tgttacctgt gcacaccgat atgaggcaag     660
gcagcgagtg gaccagatcc tggagacgcg ggatatgatt ggtcgctgct ttgtgctcag     720
ccaggacctg gccatccggg atgagttgga tggtggggaa tggaagttct gtgagggacg     780
cccccaaggc catgaacaat ttgggttctg ccagcagggc acagctgccg ccttctcccc     840
tgatagccac tacctcctct ttggggcccc aggaacctat aattggaagg ggttgctttt     900
tgtgaccaac attgatagct cagaccccga ccagctggtg tataaaactt tggaccctgc     960
tgaccggctc ccaggaccag ccggagactt ggccctcaat agctacttag gcttctctat    1020
tgactcgggg aaaggtctgg tgcgtgcaga agagctgagc tttgtggctg gagccccccg    1080
cgccaaccac aagggtgctg tggtcatcct gcgcaaggac agcgccagtc gcctggtgcc    1140
cgaggttatg ctgtctgggg agcgcctgac ctccggcttt ggctactcac tggctgtggc    1200
tgacctcaac agtgatggct ggccagacct gatagtgggt gccccctact tctttgagcg    1260
ccaagaagag ctgggggggtg ctgtgtatgt gtacttgaac cagggggggtc actgggctgg    1320
gatctcccct ctccggctct gcggctcccc tgactccatg ttcgggatca gcctggctgt    1380
cctgggggac ctcaaccaag atggctttcc agatattgca gtgggtgccc cctttgatgg    1440
tgatgggaaa gtcttcatct accatgggag cagcctgggg gttgtcgcca aaccttcaca    1500
ggtgctggag ggcgaggctg tgggcatcaa gagcttcggc tactccctgt caggcagctt    1560
ggatatggat gggaaccaat accctgacct gctggtgggc tccctggctg acaccgcagt    1620
gctcttcagg gccagaccca tcctccatgt ctcccatgag gtctctattg ctccacgaag    1680
catcgacctg gagcagccca actgtgctgg cggccactcg gtctgtgtgg acctaagggt    1740
ctgtttcagc tacattgcag tccccagcag ctatagccct actgtggccc tggactatgt    1800
gttagatgcg gacacagacc ggaggctccg ggccaggtt ccccgtgtga cgttcctgag     1860
ccgtaacctg gaagaaccca agcaccaggc ctcgggcacc gtgtggctga agcaccagca    1920
tgaccgagtc tgtggagacg ccatgttcca gctccaggaa aatgtcaaag acaagcttcg    1980
ggccattgta gtgaccttgt cctacagtct ccagacccct cggctccggc gacaggctcc    2040
tggccagggg ctgcctccag tggcccccat cctcaatgcc caccagccca gcacccagcg    2100
ggcagagatc cacttcctga agcaaggctg tggtgaagac aagatctgcc agagcaatct    2160
gcagctggtc cgcgcccgct tctgtacccg ggtcagcgac acggaattcc aacctctgcc    2220
```

catggatgtg gatggaacaa cagccctgtt tgcactgagt gggcagccag tcattggcct 2280

ggagctgatg gtcaccaacc tgccatcgga cccagcccag ccccaggctg atggggatga 2340

tgcccatgaa gcccagctcc tggtcatgct tcctgactca ctgcactact caggggtccg 2400

ggccctggac cctgcggaga agccactctg cctgtccaat gagaatgcct cccatgttga 2460

gtgtgagctg gggaacccca tgaagagagg tgcccaggtc accttctacc tcatccttag 2520

cacctccggg atcagcattg agaccacgga actggaggta gagctgctgt tggccacgat 2580

cagtgagcag gagctgcatc cagtctctgc acgagcccgt gtcttcattg agctgccact 2640

gtccattgca ggaatggcca ttccccagca actcttcttc tctggtgtgg tgaggggcga 2700

gagagccatg cagtctgagc gggatgtggg cagcaaggtc aagtatgagg tcacggtttc 2760

caaccaaggc cagtcgctca gaaccctggg ctctgccttc ctcaacatca tgtggcctca 2820

tgagattgcc aatgggaagt ggttgctgta cccaatgcag gttgagctgg agggcgggca 2880

ggggcctggg cagaaagggc tttgctctcc caggcccaac atcctccacc tggatgtgga 2940

cagtagggat aggaggcggc gggagctgga gccacctgag cagcaggagc ctggtgagcg 3000

gcaggagccc agcatgtcct ggtggccagt gtcctctgct gagaagaaga aaaacatcac 3060

cctggactgc gcccggggca cggccaactg tgtggtgttc agctgcccac tctacagctt 3120

tgaccgcgcg gctgtgctgc atgtctgggg ccgtctctgg aacagcacct ttctggagga 3180

gtactcagct gtgaagtccc tggaagtgat tgtccgggcc aacatcacag tgaagtcctc 3240

cataaagaac ttgatgctcc gagatgcctc cacagtgatc ccagtgatgg tatacttgga 3300

ccccatggct gtggtggcag aaggagtgcc ctggtgggtc atcctcctgg ctgtactggc 3360

tgggctgctg gtgctagcac tgctggtgct gctcctgtgg aagatgggat tcttcaaacg 3420

ggcgaagcac cccgaggcca ccgtgcccca gtaccatgcg gtgaagattc ctcgggaaga 3480

ccgacagcag ttcaaggagg agaagacggg caccatcctg aggaacaact ggggcagccc 3540

ccggcgggag ggcccggatg cacaccccat cctggctgct gacgggcatc ccgagctggg 3600

ccccgatggg catccagggc caggcaccgc ctaggttccc atgtcccagc ctggcctgtg 3660

gctgccctcc atcccttccc cagagatggc tccttgggat gaagagggta gagtgggctg 3720

ctggtgtcgc atcaagattt ggcaggatcg gcttcctcag ggcacagac ctctcccacc 3780

cacaagaact cctcccaccc aacttcccct tagagtgctg tgagatgaga gtgggtaaat 3840

cagggacagg gccatggggt agggtgagaa gggcaggggt gtcctgatgc aaaggtgggg 3900

agaagggatc ctaatccctt cctctcccat tcaccctgtg taacaggacc ccaaggacct 3960

gcctccccgg aagtgcctta acctagaggg tcggggagga ggttgtgtca ctgactcagg 4020

ctgctccttc tctagtttcc cctctcatct gaccttagtt tgctgccatc agtctagtgg 4080

tttcgtggtt tcgtctattt attaaaaaat atttgagaac aaaaaaaaaa aaaaaaa 4138

<210> 3
<211> 2811
<212> DNA
<213> Homo sapiens

<400> 3
ccatttcaat agtcgcggga tacttgaact gcaagaacag ccgccgctcc ggcgggctgc 60

tcgctgcatc tctgggcgtc tttggctcgc cacgctgggc agtgcctgcc tgcgcctttc 120

gcaacctcct cggccctgcg tggtctcgag ctgggtgagc gagcgggcgg gctggtaggc 180

tggcctgggc tgcgaccggc ggctacgact attctttggc cgggtcggtg cgagtggtcg 240

gctgggcaga gtgcacgctg cttggcgccg caggctgatc ccgccgtcca ctcccgggag 300

cagtgatgtt gggcaactct gcgccggggc ctgcgacccg cgaggcgggc tcggcgctgc 360

tagcattgca gcagacggcg ctccaagagg accaggagaa tatcaacccg gaaaaggcag 420

cgcccgtcca acaaccgcgg acccgggccg cgctggcggt actgaagtcc gggaacccgc 480

ggggtctagc gcagcagcag aggccgaaga cgagacgggt tgcacccctt aaggatcttc 540

ctgtaaatga tgagcatgtc accgttcctc cttggaaagc aaacagtaaa cagcctgcgt 600

tcaccattca tgtggatgaa gcagaaaaag aagctcagaa gaagccagct gaatctcaaa 660

aaatagagcg tgaagatgcc ctggctttta attcagccat tagtttacct ggacccagaa 720

aaccattggt ccctcttgat tatccaatgg atggtagttt tgagtcacca catactatgg 780

acatgtcaat tgtattagaa gatgaaaagc cagtgagtgt taatgaagta ccagactacc 840

atgaggatat tcacacatac cttagggaaa tggaggttaa atgtaaacct aaagtgggtt 900

acatgaagaa acagccagac atcactaaca gtatgagagc tatcctcgtg gactggttag 960

ttgaagtagg agaagaatat aaactacaga atgagaccct gcatttggct gtgaactaca 1020

ttgataggtt cctgtcttcc atgtcagtgc tgagaggaaa acttcagctt gtgggcactg 1080

ctgctatgct gttagcctca aagtttgaag aaatataccc cccagaagta gcagagtttg 1140

tgtacattac agatgatacc tacaccaaga aacaagttct gagaatggag catctagttt 1200

tgaaagtcct tacttttgac ttagctgctc caacagtaaa tcagtttctt acccaatact 1260

ttctgcatca gcagcctgca aactgcaaag ttgaaagttt agcaatgttt ttgggagaat 1320

taagtttgat agatgctgac ccatacctca agtatttgcc atcagttatt gctggagctg 1380

cctttcattt agcactctac acagtcacgg gacaaagctg gcctgaatca ttaatacgaa 1440

agactggata taccctggaa agtcttaagc cttgtctcat ggaccttcac cagacctacc 1500

tcaaagcacc acagcatgca caacagtcaa taagagaaaa gtacaaaaat tcaaagtatc 1560

atggtgtttc tctcctcaac ccaccagaga cactaaatct gtaacaatga aagactgcct 1620

ttgttttcta agatgtaaat cactcaaagt atatggtgta cagtttttaa cttaggtttt 1680

aattttacaa tcatttctga atacagaagt tgtggccaag tacaaattat ggtatctatt 1740

actttttaaa tggttttaat ttgtatatct tttgtatatg tatctgtctt agatatttgg 1800

ctaattttaa gtggttttgt taaagtatta atgatgccag ctgtcaggat aataaattga 1860

tttggaaaac tttgcaagtc aaatttaact tcttcaggat tttgcttagt aaagaagttt 1920

acttggttta ctatataatg ggaagtgaaa agccttcctc taaaattaaa gtaggtttag 1980

gaaaacagac cctcaaattc tgacattcat tttcctaagc aactggatca atttgctgac 2040

ttgggcataa tctaatctaa gcatatctga atacagtatt cagagataga tacagtagag 2100

attccccaga ctttttcgct ctttgtaaaa cctgtttgtt taggttttgc gaggtaaact 2160

caacagaggt tgggagtgga agagggtggg aagcttatat gcaaattaac agacgagaaa 2220

tgctccagaa ggtttattat tttaaagcac attaaaaaca aaaaactatt tttaaaatcc 2280

tgctagattt tataatggat ttgtgaataa aaaataccca gggttctcag aatggaataa 2340

atatcccttt taatagttat atatacagat atacaactgt tagctttaat tggcagctct 2400

cttctttttt cttcttttca ctggcttttt acttggtgct ttttcttgtt ttgcactggt 2460

ggtctgtgtt ctgtgaataa agcaaagtaa gaatttacta agagtatgtt aagttttgga 2520

ttattgaaat aagaggcatt tcttagtttt ccagtaggat ctaaaatgtg tcagctatga 2580

gtaagactgg catccaagaa gtttatatta tagatttagg tcctaatttt tataaatcac 2640

aaggtaaaaa aatcacagaa cagatggatc tctaatgaaa aagggatgtc tttttgttta 2700

tagtcatgtg gcaagatgag agtaaaacca gagagcaaac ctctataagt gttgagtata 2760

tgtatacatt tgaaataaac cagaaatttg ttaccttaaa aaaaaaaaaa a 2811

<210> 4
<211> 12701
<212> DNA
<213> Homo sapiens

<400> 4
cctcccgcct cagttcgcgc cgcgcctcgg cttggaacgc aggagcgccg gctccgggag 60

cccgagcgga gccagccgcg cgcacagcca gcggccgcgc cggcgatgcg gggccacccc 120

gcgcccgccc cagtcccggc cccggccccc gcgggaaggg gctgagctgc ccgccgccgc 180

ccggatggcg agcctcgccg cgctcgccct cagcctgctc ctgaggctgc agctgccgcc 240

actgcccggc gcccgggctc agagcgccgc aggtggctgt tcctttgatg agcactacag 300

caactgtggt tatagtgtgg ctctagggac caatgggttc acctgggagc agattaacac 360

atgggagaaa ccaatgctgg accaggcagt gcccacagga tctttcatga tggtgaacag 420

ctctgggaga gcctctggcc agaaggccca ccttctcctg ccaaccctga aggagaatga 480

cacccactgc atcgacttcc attactactt ctccagccgt gacaggtcca gcccaggggc 540
cttgaacgtc tacgtgaagg tgaatggtgg cccccaaggg aaccctgtgt ggaatgtgtc 600
cggggtcgtc actgagggct gggtgaaggc agagctcgcc atcagcactt tctggccaca 660
tttctatcag gtgatatttg aatccgtctc attgaagggt catcctggct acatcgccgt 720
ggacgaggtc cgggtccttg ctcatccatg cagaaaagca cctcattttc tgcgactcca 780
aaacgtggag gtgaatgtgg ggcagaatgc cacatttcag tgcattgctg gtgggaagtg 840
gtctcagcat gacaagcttt ggctccagca atggaatggc agggacacgg ccctgatggt 900
cacccgtgtg gtcaaccaca ggcgcttctc agccacagtc agtgtggcag acactgccca 960
gcggagcgtc agcaagtacc gctgtgtgat ccgctctgat ggtgggtctg gtgtgtccaa 1020
ctacgcggag ctgatcgtga aagagcctcc cacgcccatt gctcccccag agctgctggc 1080
tgtgggggcc acatacctgt ggatcaagcc aaatgccaac tccatcatcg gggatggccc 1140
catcatcctg aaggaagtgg aatatcgcac caccacaggc acgtgggcag agacccacat 1200
agtcgactct cccaactata agctgtggca tctggacccc gatgttgagt atgagatccg 1260
agtgctcctc acacgaccag gtgagggggg tacgggaccg ccagggcctc ccctcaccac 1320
caggaccaag tgtgcagatc cggtacatgg cccacagaac gtggaaatcg tagacatcag 1380
agcccggcag ctgaccctgc agtgggagcc cttcggctac gcggtgaccc gctgccatag 1440
ctacaacctc accgtgcagt accagtatgt gttcaaccag cagcagtacg aggccgagga 1500
ggtcatccag acctcctccc actacaccct gcgaggcctg cgcccottca tgaccatccg 1560
gctgcgactc ttgctgtcta accccgaggg ccgaatggag agcgaggagc tggtggtgca 1620
gactgaggaa gacgttccag gagctgttcc tctagaatcc atccaagggg ggccctttga 1680
ggagaagatc tacatccagt ggaaacctcc caatgagacc aatggggtca tcacgctcta 1740
cgagatcaac tacaaggctg tcggctcgct ggacccaagt gctgacctct cgagccagag 1800
ggggaaagtg ttcaagctcc ggaatgaaac ccaccacctc tttgtgggtc tgtacccagg 1860
gaccacctat tccttcacca tcaaggccag cacagcaaag ggctttgggc cccctgtcac 1920
cactcggatt gccaccaaaa tttcagctcc atccatgcct gagtacgaca cagacacccc 1980
attgaatgag acagacacga ccatcacagt gatgctgaaa cccgctcagt cccggggagc 2040
tcctgtcagt gtttatcagc tggttgtcaa ggaggagcga cttcagaagt cacggagggc 2100
agctgacatt attgagtgct tttcggtgcc cgtgagctat cggaatgcct ccagcctcga 2160
ttctctacac tactttgctg ctgagttgaa gcctgccaac ctgcctgtca cccagccatt 2220
tacagtgggt gacaataaga catacaatgg ctactggaac cctcctctct ctcccctgaa 2280
aagctacagc atctacttcc aggcactcag caaagccaat ggagagacca aaatcaactg 2340
tgttcgtctg gctacaaaag caccaatggg cagcgcccag gtgaccccgg ggactccact 2400

ctgcctcctc accacaggtg cctccaccca gaattctaac actgtggagc cagagaagca 2460
ggtggacaac accgtgaaga tggctggcgt gatcgctggc ctcctcatgt tcatcatcat 2520
tctcctgggc gtgatgctca ccatcaaaag gagaagaaat gcttattcct actcctatta 2580
cttgaagctg gccaagaagc agaaggagac ccagagtgga gcccagaggg agatggggcc 2640
tgtggcctct gccgacaaac ccaccaccaa gctcagcgcc agccgcaatg atgaaggctt 2700
ctcttctagt tctcaggacg tcaacggatt cacagatggc agccgcgggg agctttccca 2760
gcccaccctc acgatccaga ctcatcccta ccgcacctgt gaccctgtgg agatgagcta 2820
cccccgggac cagttccaac ccgccatccg ggtggctgac ttgctgcagc acatcacgca 2880
gatgaagaga ggccagggct acgggttcaa ggaggaatac gaggccttac cagaggggca 2940
gacagcttcg tgggacacag ccaaggagga tgaaaaccgc aataagaatc gatatgggaa 3000
catcatatcc tacgaccatt cccgggtgag gctgctggtg ctggatggag acccgcactc 3060
tgactacatc aatgccaact acattgacgg ataccatcga cctcggcact acattgcgac 3120
tcaaggtccg atgcaggaga ctgtaaagga cttttggaga atgatctggc aggagaactc 3180
cgccagcatc gtcatggtca caaacctggt ggaagtgggc agggtgaaat gtgtgcgata 3240
ctggccagat gacacggagg tctacggaga cattaaagtc accctgattg aaacagagcc 3300
cctggcagaa tacgtcatac gcaccttcac agtccagaag aaaggctacc atgagatccg 3360
ggagctccgc ctcttccact tcaccagctg gcctgaccac ggcgttccct gctatgccac 3420
tggccttctg ggcttcgtcc gccaggtcaa gttcctcaac cccccggaag ctgggcccat 3480
agtggtccac tgcagtgctg gggctgggcg gactggctgc ttcattgcca ttgacaccat 3540
gcttgacatg gccgagaatg aaggggtggt ggacatcttc aactgcgtgc gtgagctccg 3600
ggcccaaagg gtcaacctgg tacagacaga ggagcaatat gtgtttgtgc acgatgccat 3660
cctggaagcg tgcctctgtg gcaacactgc catccctgtg tgtgagttcc gttctctcta 3720
ctacaatatc agcaggctgg acccccagac aaactccagc caaatcaaag atgaatttca 3780
gaccctcaac attgtgacac cccgtgtgcg gcccgaggac tgcagcattg ggctcctgcc 3840
ccggaaccat gataagaatc gaagtatgga cgtgctgcct ctggaccgct gcctgccctt 3900
ccttatctca gtggacggag aatccagcaa ttacatcaac gcagcactga tggatagcca 3960
caagcagcct gccgccttcg tggtcaccca gcaccctcta cccaacaccg tggcagactt 4020
ctggaggctg gtgttcgatt acaactgctc ctctgtggtg atgctgaatg agatggacac 4080
tgcccagttc tgtatgcagt actggcctga gaagacctcc gggtgctatg ggcccatcca 4140
ggtggagttc gtctccgcag acatcgacga ggacatcatc cacagaatat tccgcatctg 4200
taacatggcc cggccacagg atggttatcg tatagtccag cacctccagt acattggctg 4260

gcctgcctac cgggacacgc ccccctccaa gcgctctctg ctcaaagtgg tccgacgact 4320
ggagaagtgg caggagcagt atgacgggag ggagggacgt actgtggtcc actgcctaaa 4380
tgggggaggc cgtagtggaa ccttctgtgc catctgcagt gtgtgtgaga tgatccagca 4440
gcaaaacatc attgacgtgt tccacatcgt gaaaacactg cgtaacaaca aatccaacat 4500
ggtggagacc ctggaacagt ataaatttgt atacgaggtg gcactggaat atttaagctc 4560
cttttagctc aatgggatgg ggaacctgcc ggagtccaga ggctgctgtg accaagcccc 4620
cttttgtgtg aatggcagta actgggctca ggagctctga ggtggcaccc tgcctgactc 4680
caaggagaag actggtggcc ctgtgttcca cggggggctc tgcaccttct gaggggtctc 4740
ctgttgccgt gggagatgct gctccaaaag gcccaggctt ccttttcaac ctaaccagcc 4800
acagccaagg gcccaagcag aagtacaccc acaagcaagg ccttggattt ctggctccca 4860
gaccacctgc ttttgttctg agtttgtgga tctcttggca agccaactgt gcaggtgctg 4920
gggagtggga ggctcccctg ccctccttct ccttaggagt ggaggagatg tgtgttctgc 4980
tcctctacgt catggaaaag attgaggctc ttgggggtca ctgctctgct gccccctgca 5040
acctccttca ggggcctctg gcaccagaca tttgcagtct ggaccagtgt gaccttacga 5100
tgttccctag gccacaagag aggcccccca tcctcacacc taacctgcat ggggcttcgc 5160
ccacaaccat tctgtacccc ttccccagcc tgggccttga ccgtccagca ttcactggcc 5220
ggccagctgt gtccacagca gtttttgata aaggtgttct ttgctttttt gtgtggtcag 5280
tgggaggggg tggaactgca gggaacttct ctgctcctcc ttgtctttgt aaaaagggac 5340
cacctccctg gggcagggct tgggctgacc tgtaggatgt aacccctgtg tttctttggt 5400
ggtagctttc tttggaagag acaaacaaga taagatttga ttattttcca aagtgtatgt 5460
gaaaagaaac tttcttttgg agggtgtaaa atcttagtct cttatgtcaa aaagaagggg 5520
gcgggggagt ttgagtatgt acctctaaga caaatctctc gggcctttta tttttttcctg 5580
gcaatgtcct taaaagctcc caccctggga cagcatgcca ctgagcaagg agagatgggt 5640
gagcctgaag atggtccctt tggtttctgg ggcaaataga gcaccagctt tgtgcataat 5700
ttggatgtcc aaatttgaac tccttcctaa agaaacccag cagccacctt gaaaaaggcc 5760
attgtggagc ccattatact ttgatttaaa ataggccaag agaatcaggc ctggagatct 5820
agggtcttgt ccaaagtgtg agtgagtcaa tgagagggaa ccaacatttg ctaagtctct 5880
actgtatgcc agggatcatg cttggcactt tccataggac atttcacaca gtccttagaa 5940
cccccaggag agagctactg acttgttatc atctccattt gatcatctcc tccaatgagg 6000
aaacccacgc accttcctta gtaatgaaat cctgggttcc aaaggggcag gtaatggcaa 6060
tgagacttct ccgtgctgtt ttcttcatct tctctaagcc aagcaattat tttatggagg 6120
gaaaataagg ccagaaactt ctgagcagat aactccacaa atggaaattt agtactttct 6180

tcctgatgcc agttcttctg ggaagcgcag aatttcagat atattttagt aacacattcc 6240
cagctcccca ggaaagccag tctcatctaa tttcttagtc agtaaaaaca attccctgtt 6300
ccttcaggct atgaatggac cagccaggga aactctcgac cttgatctct agccagtgct 6360
taggcccaat atctgacagc ctcaggtggg ctgggaccta ggaagctcca tcttgaaggc 6420
tggtctagcc ccagacaggg catgaggggc agagaattca agaaggtaca gctttggccc 6480
tcaagagccc actgtatgct ggggaaatgg aaccatggtg cagtagtgtg gagtggatga 6540
gtgttccatg agcctaggag caagaaagtc tcttcggcct cgggcttcct ggagaagggg 6600
acgtccattc ctgctgggtc ttaacaagca taaaaaggaa aaaaaggaaa ctcaggcaaa 6660
gggatccata tgtgcaatgg caaagaaatg tgaaaaggca ttgggagaag cagtctgggg 6720
gaggccagcc cagtgcgggc acagcacaac acggggagca gcaagagatg agccagggtc 6780
caggagacag atgcccatcg cgagtacaga ctttgtccta ttggcaacaa ggagtccatg 6840
gagctttaga gagatgcact cagcttcgtg ttggccaaga ctccttctgg gccaatgggg 6900
ctgcctcttt tcctttcatc agacactgtg aaaacattcc cttaagcgtg cactttttaa 6960
tatcacatct atttgtctgt ctgctcattg ttttgttgct ggaactaaat atgcaatgga 7020
tcatgagact cagattctat gagaaaccca gggtctctgc tttaccacgg agcagggtca 7080
ccaacccaga tctccaggcc catgaggatg gaacatgaaa ggagccgaca aaagttgctt 7140
ccattggcat gggctctgga gctgtccaga agtccaggga caccagactt gatcaaggaa 7200
gggctgtcac tttagaggtt caaaaggaag tgcctcaaag caaaggcaag caaaggaacc 7260
ccacgatgaa cttgctcttt tcctttgatg agcctctccc caggtgtatt tcagcagacc 7320
ccggggaccc acccccactg ggcctgctgg cctccctcgg ctccagccca atgccccagc 7380
tggccttccc cagcctgcaa ggagcctgta gcatggcaaa tctgcctgct gtatgctatt 7440
ttcttagatc ttggtacatc cagacaggat gagggtggag ggagagctat ttaacacaaa 7500
tcctaagatt tttttctgct caggaagggg tgaaatagct ggcagataca aaagacagtg 7560
gcttttatca ttttaaatgg taggaattta aggtgtgact tcagggagaa acaaacttgc 7620
aaaaaaaaaa aatctcaggc catgttgggg taacccagca agggccagtg atgatttccc 7680
ccagctcatc cccttatttt cccacaaccc aaccattctc taaagcagga cagtgaatag 7740
gtcttaggcc agtgcacaca ggaagaaatt gaggcttatg gatggggatg acttccctaa 7800
gatcccatgg gacaaggatg tggcaaggct tggatgagat ggggcaccag tgcccaggaa 7860
tttgaacatt ttcctttacc caggaaatct ccggagccaa caccaccacc cccaggggt 7920
ctccccaccc caccccattt acagggtgag ctcagcctgt catgagcaga ggaaaatatt 7980
attaatgctc tctgagtctt tacaacagga gctcttacct catagatgtg ggctctgttt 8040

ggggaagatg caaggaagta atgagaagcc caggaaattt ctccacctgt gtttatggcc 8100
taaatagctt caggatgtat cttagctgca ctccaacatt gcatcctttc tggggtgaag 8160
aatctgggcc aaccaggggt ccttgggcct ctagaaggcc acagtaggcc tctctttgtg 8220
ggaatggaag gggacagttt gcttttagtg ctggccctct ctgtgggtgt ggcctgcaaa 8280
ggaaccaaca gaccctatgc tggggactct aacatgtgag ctcattaaat tcttccagca 8340
ttctaaagga gggtttgtga ttgtcaccat ttactgatga ggaaactaag gctcctaggg 8400
gagaaatcac ttgcccacag ttccacagct agtgagtgaa tgaaccagga tttaaaccgg 8460
ttttttctca ctacagagac aatatttttc caccattgta tctcacattt ttcccaggag 8520
gttacccata acagaagaga ctagagtgga acagatacgt cagtggataa agctcaaagc 8580
aaacaacagt aagcttaaaa ttccttcata gtctcatgtt ttacgttcac aattcatgca 8640
aaatttgcat tccactttct gatttagcct tgttggtttt aatatgactc tatgaatatt 8700
tcaaaaaaaa atgtgctctg ttcctcatgt tgttctgttc tgttcacccc gctatgacgg 8760
accctaggtc agctggtctt cagcttgacc ctagaattga ctctaggagc agtgaccctg 8820
ctgcctccca gagccagtta taggctcaag atcaagacca actgaccttc tcctaggcag 8880
ctcctttggt gtgtgggtgc tctgacctca ctgttcatga ggggacctca actaaggcat 8940
cttccagttg ggtgctggaa ggaacccatt aactcacact agaatgatga ggatttgctc 9000
atctggcgtg gagaaggatg agcccacaaa accctaaagg gaaaagagaa gctggacaca 9060
gctgtactca gcagattcct gaatgctagg ctggaaagtg gtgcctgttg tccaagtgga 9120
gtcacatggt tgctaatgtg ggcaagtctg aggacacact tcatgagcag ctggggtctg 9180
gaaggctcct cactttaccc tagccacaca taattactgg gtgcctacag cacctagcac 9240
cttggagggg gcactattag gaaatcgaga ttactatggc acaattaatt cctgggtaag 9300
gcatggggtt gtggtggaca gagctcagtc tttagtttga acgaaaacat acatacatga 9360
aaaacataca tgaaaaaagg accctcatca acattagaag ggtagattt ggagcacttt 9420
aggcaggaaa acaggaacgc aaggccagga aactggaacc cagtgaatac tcagaaccga 9480
ggatgcagat gacttattta gcaaaatggt cacttctgtg acatagctgg agaaaggatg 9540
ggtaacagct tgccagagcc acttggaaca agggcaaatc tcagtgtctg ggcaaaaga 9600
tgatgcattt ccctctgacc catcatgttt attcatcctc cactccccat tgccacacta 9660
gctcttgctg taagtcctca ccaggatcta catttcctcg tcgctggtgg gaacccctta 9720
gagtacatag aggtatcagt ccagtaagac tgctctacac aacagaagtg aggcccaggg 9780
agtagcagcc aggcccttat cctgttacct ctgcaggagt gactgcccaa cccagatcca 9840
gagacattga aggaaatgat aattccttgg tacctcactg ccttgggaca aaatgaagaa 9900
agccaccctt ccttaggctg cagcttgcca ctcctgggct gggtaaacag gtcatcagca 9960

ccaagctcaa ccaggagtaa cactctggaa gacatgggtg agcccaagag gaagcatgaa 10020
caggacgctg ttcctaagtc atgtcaacag gttgtgctgg gccaggatcc ccagggaaaa 10080
aaatggtcaa cccaactgga gggtaggtta gaagaaaaaa aacataaacg tggatagtca 10140
tgtcatctca aatccctgac ttggcttccc cattacttaa cagtctgagc tccttcttag 10200
cctgtgacca gcttcaaatc acagccaagt aaaacaagga aataggaaaa gtaaatccaa 10260
ctagaagaga caagctgaga ttcagatttg tttactcctc ccatgcaaag tttccctgtt 10320
ggaggttttc catgtataca tgtctagaag tgatagaatg caaggccttg gctttgtctt 10380
gcagggatct gcctttgagg tcatagactg aacagcaggg agagaggtta gtggtggagt 10440
gtggggggag ctgttctagc tccagtttct tctgacacat ttttcaggat catggatctg 10500
atcctccgaa gcacagcaga gatatctaag ccatatttgt gcacatgagc agactcttct 10560
agtttttttag taaccaggga tgggcttttg catggcactg actatagaga tgtcttgtag 10620
agatcaagcc agtcttttgc atcccacctg cccacctcca gaagagatgg gaaaaggtca 10680
tcaaagggca ttcaccaact gaaatccact catgaatgtt aggtctctaa aaggaggcat 10740
caacactcac aatggtagcc tccaaaccta gcatcccacc tatctaagag ctcaggggtg 10800
gtccactggg gcagatacaa gggaagtgca agggctcagg atgaaagaaa atctattggg 10860
aagagtttta ggggcttgat cattatgggg cttccttcta tatctgagaa ctgctctggg 10920
tggtgagatg tggactctga tccttaattg gaatgttcgg agaatgagtg tctggtggcc 10980
ttgaagtgtt ggacagaaaa gtatcagtat aaaagcctgg agctcagggt aattaatgta 11040
gttcatggtt ccttagtgag caggactctt ggatgtggag gagaaagggt cataggaagt 11100
aaaccaccaa aattacaaaa ttgagtctct gtacaattac ttcagtgcct ttgggcttat 11160
gaatacaaat cagtgggcct tctctatgat ggtccaacaa actctcagtg tccaccctgt 11220
ccctgtatct cccatggaag atgaataatg tcaggtgttc tttgggtcaa aggccccagg 11280
gcagtctgga ggcttagagg gcagagtggt gtcattccat gtaaagttag gcttctgagg 11340
ggtcaggcag aatatggtgt ccatatcttc catagctctg cagattcttg gatgaagtca 11400
agcacagttt gctagaccca ggtcactcct ctgagtataa ctaggaccca tgagtgaaac 11460
ttaatagctg taaggaagaa cctgctgtct gccagagagg ataagctgcc catctcagca 11520
gctgtctaaa agaaggcagg tgtctcttta aagggaagag aagcattggt gaaatggatt 11580
tcaggtcact tccattccag atgggtgaga tcttgtggag ctgggatcat gtttgaactc 11640
attcatacct gtagagcacg aatccaagta gattgtgttt ggtctgtaca ggctgaagcc 11700
ccctgctctc ccacccaagt gcccccactg agcaggccaa catgctgttg tggccacata 11760
tactgggctg atccaggctg gttatcacca aacagcaaac catagggaac agctgctttg 11820

ccatagaccc aatacccatg tagatctctc atgagagcag ccataactca gacccactga 11880

ccaacagggc catgagtgac agccagaacc agtgaaggtc caagtaggac acagagcagg 11940

gcttttctta ccatacacat tatctccaga ggttatttct accccactcc ctattcaagg 12000

cctgttggag cacactgcaa aagcaaaagc acagtaactc aatttacaca tgattataat 12060

catttccagt gcacacattt catcaccagg tggatcctga gctagcccat gtaaatccgg 12120

gttaacccat attggtaatc atactcaaaa gcacttttca ccctacattc tactagccaa 12180

tcaaagacaa agagttgtgg cctctaccat tgccttggct tctggacacc ctcacaagct 12240

atcccaaggt tcccgctcaa ctccagggag gctgacatct tcacatccac tgggcatata 12300

atattgcatg agaccaaagt ctccacactc tttgcagcct cctccatgaa tcccaatggc 12360

ctgcacttgt acagtttggg tgtttgatag ataaagcacg tatgagaaga gaaaacaaaa 12420

taaatcaact ttttaaaaaa gccagcactg tgctgtcaat gttttttttt tcttttcaat 12480

tctagctcag aaaagcagaa ggtaaataat gtcaggtcaa tgaatatcag atatattttt 12540

tgactgtaca ttacagtgaa gtgtaatctt tttacacctg caagtccatc ttatttattc 12600

ttgtaaatgt tccctgacaa tgtttgtaat atggctgtgt taaaaaatct atacaataaa 12660

gctgtgaccc tgagattcat gttttcctaa gataaaaaaa a 12701

<210> 5
<211> 822
<212> DNA
<213> Homo sapiens

<220>
<221> misc_feature
<222> (566)..(566)
<223> n is a, c, g, or t

<400> 5
aaaaaaaaaa gtcctgtgga aatcatatag acaaacattt gcaaagctgc tactgccatt 60

gtaccagtgt taaactgtgt tctaccttgc atcttttact gatttttatg acagatttta 120

tattggtaac cattcgagaa ctctgtaagt gctatggctt ccttaaacta cgatttatca 180

tatgctccca gtgtttactt tgagactgaa tggcaaccag agaatgtaaa caaccaaggt 240

gcatctggtt atgttttaaa ataaagatta ataaaagtta aggtaaaagg tctgtgtctg 300

aacctatgca tttttttcacc tctgaggagg atactaatac ctaatgagaa aagctgaaat 360

gtgtgggccg cgggtggcgg gtgcgctaca tggcgctaat ggatactctc agacagcttg 420

aggggagggc gcgcaggcag gtcagaaatc gagcctggag gctcggagaa gaattcggag 480

ttacccaggg gcgtgggacc ctaagcgagt ggagtggaac acccttgatt ctcgtgagtg 540

gagaggtgtc accaaaaata ttgagnccgg ggcggactat taggacagaa ctaccatgat 600

catatccacg aggtgctaaa ctggtgcgac aagggtctga aggcgcagta aatcagaggc 660

gtggtaacac cccgctaagg agtgggccgt aagtgtcctg gcgcctggga tgtccagaga 720

agattcgggg gggccaataa gggcaagtaa cccaggattc tggcaggcaa cactaacttc 780

agttcgaacc gggttccccg gtggcggtta caacacaagc cc 822

<210> 6
<211> 567
<212> DNA
<213> Homo sapiens

<400> 6
ccgggcgcgt tccgttggcg gcggattcga acgttcggac tgaggttttt ctgcctgaag 60

aagcgtcata cggaccggat tgttttcgct ggcccagtgt ccccggagct tgtgtgcgat 120

acagagagca cctcggaagc tgaggcagct ggtacttgac agagaggatg gcgctgtcga 180

ccatagtctc ccagaggaag cagataaagc ggaaggctcc ccgtggcttt ctaaagcgag 240

tcttcaagcg aaagaagcct caacttcgtc tggagaaaag tggtgactta ttggtccatc 300

tgaactgttt actgtttgtt catcgattag cagaagagtc caggacaaac gcttgtgcga 360

gtaaatgtag agtcattaac aaggagcatg tactggccgc agcaaaggta attctaaaga 420

agagcagagg ttagaagtca aagaacatat tcttgaaagt tatgatgcat tcttttgggt 480

ggtaacagat cataaagaca ttttttacac atcagttaat atgggattat taaatattgg 540

ctataagtga aaaaaaaaaa aaaaaaa 567

<210> 7
<211> 2501
<212> DNA
<213> Homo sapiens

<400> 7
cgaaaagatt cttaggaacg ccgtaccagc cgcgtctctc aggacagcag gcccctgtcc 60

ttctgtcggg cgccgctcag ccgtgccctc cgcccctcag gttctttttc taattccaaa 120

taaacttgca agaggactat gaaagattat gatgaacttc tcaaatatta tgaattacat 180

gaaactattg ggacaggtgg ctttgcaaag gtcaaacttg cctgccatat ccttactgga 240

gagatggtag ctataaaaat catggataaa aacacactag ggagtgattt gccccggatc 300

aaaacggaga ttgaggcctt gaagaacctg agacatcagc atatatgtca actctaccat 360

gtgctagaga cagccaacaa aatattcatg gttcttgagt actgccctgg aggagagctg 420

tttgactata taatttccca ggatcgcctg tcagaagagg agacccgggt tgtcttccgt 480

cagatagtat ctgctgttgc ttatgtgcac agccagggct atgctcacag ggacctcaag 540

ccagaaaatt tgctgtttga tgaatatcat aaattaaagc tgattgactt tggtctctgt 600

gcaaaaccca agggtaacaa ggattaccat ctacagacat gctgtgggag tctggcttat 660

gcagcacctg agttaataca aggcaaatca tatcttggat cagaggcaga tgtttggagc 720
atgggcatac tgttatatgt tcttatgtgt ggatttctac catttgatga tgataatgta 780
atggctttat acaagaagat tatgagagga aaatatgatg ttcccaagtg gctctctccc 840
agtagcattc tgcttcttca acaaatgctg caggtggacc caaagaaacg gatttctatg 900
aaaaatctat tgaaccatcc ctggatcatg caagattaca actatcctgt tgagtggcaa 960
agcaagaatc cttttattca cctcgatgat gattgcgtaa cagaactttc tgtacatcac 1020
agaaacaaca ggcaaacaat ggaggattta atttcactgt ggcagtatga tcacctcacg 1080
gctacctatc ttctgcttct agccaagaag gctcggggaa aaccagttcg tttaaggctt 1140
tcttctttct cctgtggaca agccagtgct accccattca cagacatcaa gtcaaataat 1200
tggagtctgg aagatgtgac cgcaagtgat aaaaattatg tggcgggatt aatagactat 1260
gattggtgtg aagatgattt atcaacaggt gctgctactc cccgaacatc acagtttacc 1320
aagtactgga cagaatcaaa tggggtggaa tctaaatcat taactccagc cttatgcaga 1380
acacctgcaa ataaattaaa gaacaaagaa aatgtatata ctcctaagtc tgctgtaaag 1440
aatgaagagt actttatgtt tcctgagcca aagactccag ttaataagaa ccagcataag 1500
agagaaatac tcactacgcc aaatcgttac actacaccct caaaagctag aaaccagtgc 1560
ctgaaagaaa ctccaattaa aataccagta aattcaacag gaacagacaa gttaatgaca 1620
ggtgtcatta gccctgagag gcggtgccgc tcagtggaat tggatctcaa ccaagcacat 1680
atggaggaga ctccaaaaag aaagggagcc aaagtgtttg ggagccttga aaggggggttg 1740
gataaggtta tcactgtgct caccaggagc aaaaggaagg gttctgccag agacgggccc 1800
agaagactaa agcttcacta taatgtgact acaactagat tagtgaatcc agatcaactg 1860
ttgaatgaaa taatgtctat tcttccaaag aagcatgttg actttgtaca aaagggttat 1920
acactgaagt gtcaaacaca gtcagatttt gggaaagtga caatgcaatt tgaattagaa 1980
gtgtgccagc ttcaaaaacc cgatgtggtg ggtatcagga ggcagcggct taagggcgat 2040
gcctgggttt acaaaagatt agtggaagac atcctatcta gctgcaaggt ataattgatg 2100
gattcttcca tcctgccgga tgagtgtggg tgtgatacag cctacataaa gactgttatg 2160
atcgctttga ttttaaagtt cattggaact accaacttgt ttctaaagag ctatcttaag 2220
accaatatct ctttgttttt aaacaaaaga tattattttg tgtatgaatc taaatcaagc 2280
ccatctgtca ttatgttact gtcttttta atcatgtggt tttgtatatt aataattgtt 2340
gactttctta gattcacttc catatgtgaa tgtaagctct taactatgtc tctttgtaat 2400
gtgtaatttc tttctgaaat aaaaccattt gtgaatataa aaaaaaaaaa aaaaaaaaaa 2460
aaaaaaaaaa aaaaaaaaaa aaaaaaaaaa aaaaaaaaaa a 2501

<210> 8
<211> 728
<212> DNA
<213> Homo sapiens

<400> 8
agggctttct gtatccctag gtttcttgcc ttgatgtact ggagcaatca gatcacacgg 60

cggcttggag aaacccaggg accatgggcg cctccaggct ctataccctg gtgctggtcc 120

tgcagcctca gcgagttctc ctgggcatga aaaagcgagg cttcggggcc ggccggtgga 180

atggctttgg gggcaaagtg caagaaggag agaccatcga ggatggggct aggagggagc 240

tgcaggagga gagcggtctg acagtggacg ccctgcacaa ggtgggccag atcgtgtttg 300

agttcgtggg cgagcctgag ctcatggacg tgcatgtctt ctgcacagac agcatccagg 360

ggacccccgt ggagagcgac gaaatgcgcc catgctggtt ccagctggat cagatcccct 420

tcaaggacat gtggcccgac gacagctact ggtttccact cctgcttcag aagaagaaat 480

tccacgggta cttcaagttc cagggtcagg acaccatcct ggactacaca ctccgcgagg 540

tggacacggt ctagcgggag cccagggcag cccctgggca ggagacgtgg ctgctgaaca 600

gccgcaaacc atcttcacct gggggcattg agtggcgcag agccgggttt catctggaat 660

taactggatg gaagggaaaa taaagctatc tagcggtgaa aaaaaaaaaa aaaaaaaaaa 720

aaaaaaaa 728

<210> 9
<211> 818
<212> DNA
<213> Homo sapiens

<400> 9
agtcctgtgt ccgggccccg aggcacagcc agggcaccag gtggagcacc agctacgcgt 60

ggcgcagcgc agcgtcccta gcaccgagcc tcccgcagcc gccgagatgc tgcgaacaga 120

gagctgccgc cccaggtcgc ccgccggaca ggtggccgcg gcgtccccgc tcctgctgct 180

gctgctgctg ctcgcctggt gcgcgggcgc ctgccgaggt gctccaatat tacctcaagg 240

attacagcct gaacaacagc tacagttgtg gaatgagata gatgatactt gttcgtcttt 300

tctgtccatt gattctcagc ctcaggcatc caacgcactg gaggagcttt gctttatgat 360

tatgggaatg ctaccaaagc ctcaggaaca agatgaaaaa gataatacta aaaggttctt 420

atttcattat tcgaagacac agaagttggg caagtcaaat gttgtgtcgt cagttgtgca 480

tccgttgctg cagctcgttc ctcacctgca tgagagaaga atgaagagat tcagagtgga 540

cgaagaattc caaagtccct ttgcaagtca aagtcgagga tattttttat tcaggccacg 600

gaatggaaga aggtcagcag ggttcattta aaatggatgc cagctaattt tccacagagc 660

aatgctatgg aatacaaaat gtactgacat tttgttttct tctgaaaaaa atccttgcta 720

aatgtactct gttgaaaatc cctgtgttgt caatgttctc agttgtaaca atgttgtaaa 780

tgttcaattt gttgaaaatt aaaaaatcta aaaataaa 818

<210> 10
<211> 2748
<212> DNA
<213> Homo sapiens

<400> 10
agcgggtgcg gggcgggacc ggcccggcct atatattggg ttggcgccgg cgccagctga 60

gccgagcggt agctggtctg gcgaggtttt atacacctga aagaagagaa tgtcaagacg 120

aagtagccgt ttacaagcta agcagcagcc ccagcccagc cagacggaat ccccccaaga 180

agcccagata atccaggcca agaagaggaa aactacccag gatgtcaaaa aaagaagaga 240

ggaggtcacc aagaaacatc agtatgaaat taggaattgt tggccacctg tattatctgg 300

ggggatcagt ccttgcatta tcattgaaac acctcacaaa gaaataggaa caagtgattt 360

ctccagattt acaaattaca gatttaaaaa tctttttatt aatccttcac ctttgcctga 420

tttaagctgg ggatgttcaa aagaagtctg gctaaacatg ttaaaaaagg agagcagata 480

tgttcatgac aaacattttg aagttctgca ttctgacttg gaaccacaga tgaggtccat 540

acttctagac tggcttttag aggtatgtga agtatacaca cttcataggg aaacatttta 600

tcttgcacaa gactttttttg atagatttat gttgacacaa aaggatataa ataaaaatat 660

gcttcaactc attggaatta cctcattatt cattgcttcc aaacttgagg aaatctatgc 720

tcctaaactc caagagtttg cttacgtcac tgatggtgct tgcagtgaag aggatatctt 780

aaggatggaa ctcattatat taaaggcttt aaaatgggaa ctttgtcctg taacaatcat 840

ctcctggcta aatctctttc tccaagttga tgctcttaaa gatgctccta aagttcttct 900

acctcagtat tctcaggaaa cattcattca aatagctcag cttttagatc tgtgtattct 960

agccattgat tcattagagt tccagtacag aatactgact gctgctgcct tgtgccattt 1020

tacctccatt gaagtggtta agaaagcctc aggtttggag tgggacagta tttcagaatg 1080

tgtagattgg atggtacctt ttgtcaatgt agtaaaaagt actagtccag tgaagctgaa 1140

gacttttaag aagattccta tggaagacag acataatatc cagacacata caaactattt 1200

ggctatgctg gaggaagtaa attacataaa caccttcaga aaagggggac agttgtcacc 1260

agtgtgcaat ggaggcatta tgacaccacc gaagagcact gaaaaaccac caggaaaaca 1320

ctaaagaaga taactaagca aacaagttgg aattcaccaa gattgggtag aactggtatc 1380

actgaactac taaagtttta cagaaagtag tgctgtgatt gattgcccta gccaattcac 1440

aagttacact gccattctga ttttaaaact tacaattggc actaaagaat acatttaatt 1500

atttcctatg ttagctgtta aagaaacagc aggacttgtt tacaaagatg tcttcattcc 1560

caaggttact ggatagaagc caaccacagt ctataccata gcaatgtttt tcctttaatc 1620

cagtgttact gtgtttatct tgataaacta ggaattttgt cactggagtt ttggactgga 1680

taagtgctac cttaaagggt atactaagtg atacagtact ttgaatctag ttgttagatt 1740

ctcaaaattc ctacactctt gactagtgca atttggttct tgaaaattaa atttaaactt 1800

gtttacaaag gtttagtttt gtaataaggt gactaattta tctatagctg ctatagcaag 1860

ctattataaa acttgaattt ctacaaatgg tgaaatttaa tgtttttttaa actagtttat 1920

ttgccttgcc ataacacatt ttttaactaa taaggcttag atgaacatgg tgttcaacct 1980

gtgctctaaa cagtgggagt accaaagaaa ttataaacaa gataaatgct gtggctcctt 2040

cctaactggg gctttcttga catgtaggtt gcttggtaat aacctttttg tatatcacaa 2100

tttgggtgaa aaacttaagt accctttcaa actatttata tgaggaagtc actttactac 2160

tctaagatat ccctaaggaa tttttttttt taatttagtg tgactaaggc tttatttatg 2220

tttgtgaaac tgttaaggtc ctttctaaat tcctccattg tgagataagg acagtgtcaa 2280

agtgataaag cttaacactt gacctaaact tctattttct taaggaagaa gagtattaaa 2340

tatatactga ctcctagaaa tctatttatt aaaaaaagac atgaaaactt gctgtacata 2400

ggctagctat ttctaaatat tttaaattag cttttctaaa aaaaaaatcc agcctcataa 2460

agtagattag aaaactagat tgctagttta ttttgttatc agatatgtga atctcttctc 2520

cctttgaaga aactatacat ttattgttac ggtatgaagt cttctgtata gtttgttttt 2580

aaactaatat ttgtttcagt attttgtctg aaaagaaaac accactaatt gtgtacatat 2640

gtattatata aacttaacct tttaatactg tttattttta gcccattgtt taaaaaataa 2700

aagttaaaaa aatttaactg cttaaaagta aaaaaaaaaa aaaaaaa 2748

<210> 11
<211> 8630
<212> DNA
<213> Homo sapiens

<400> 11
taaatttaaa ggcggggcgg cctgtgagcc ctgaagtgcc ggccgcggag ggtcctggcc 60

attttcctgg gaccagttca gcctgatagg atggcggagg aaggagccgt ggccgtctgc 120

gtgcgagtgc ggccgctgaa cagcagagaa gaatcacttg gagaaactgc ccaagtttac 180

tggaaaactg acaataatgt catttatcaa gttgatggaa gtaaatcctt caattttgat 240

cgtgtctttc atggtaatga aactaccaaa aatgtgtatg aagaaatagc agcaccaatc 300

atcgattctg ccatacaagg ctacaatggt actatatttg cctatggaca gactgcttca 360

ggaaaaacat ataccatgat gggttcagaa gatcatttgg gagttatacc cagggcaatt 420

catgacattt tccaaaaaat taagaagttt cctgataggg aatttctctt acgtgtatct 480

tacatggaaa tatacaatga aaccattaca gatttactct gtggcactca aaaaatgaaa 540
cctttaatta ttcgagaaga tgtcaatagg aatgtgtatg ttgctgatct cacagaagaa 600
gttgtatata catcagaaat ggctttgaaa tggattacaa agggagaaaa gagcaggcat 660
tatggagaaa caaaaatgaa tcaaagaagc agtcgttctc ataccatctt taggatgatt 720
ttggaaagca gagagaaggg tgaaccttct aattgtgaag gatctgttaa ggtatcccat 780
ttgaatttgg ttgatcttgc aggcagtgaa agagctgctc aaacaggcgc tgcaggtgtg 840
cggctcaagg aaggctgtaa tataaatcga agcttattta ttttgggaca agtgatcaag 900
aaacttagtg atggacaagt tggtggtttc ataaattatc gagatagcaa gttaacacga 960
attctccaga attccttggg aggaaatgca aagacacgta ttatctgcac aattactcca 1020
gtatcttttg atgaaacact tactgctctc cagtttgcca gtactgctaa atatatgaag 1080
aatactcctt atgttaatga ggtatcaact gatgaagctc tcctgaaaag gtatagaaaa 1140
gaaataatgg atcttaaaaa acaattagag gaggtttctt tagagacgcg ggctcaggca 1200
atggaaaaag accaattggc ccaacttttg gaagaaaaag atttgcttca gaaagtacag 1260
aatgagaaaa ttgaaaactt aacacggatg ctggtgacct cttcttccct cacgttgcaa 1320
caggaattaa aggctaaaag aaaacgaaga gttacttggt gccttggcaa aattaacaaa 1380
atgaagaact caaactatgc agatcaattt aatataccaa caaatataac aacaaaaaca 1440
cataagcttt ctataaattt attacgagaa attgatgaat ctgtctgttc agagtctgat 1500
gttttcagta acactcttga tacattaagt gagatagaat ggaatccagc aacaaagcta 1560
ctaaatcagg agaatataga aagtgagttg aactcacttc gtgctgacta tgataatctg 1620
gtattagact atgaacaact acgaacagaa aaagaagaaa tggaattgaa attaaaagaa 1680
aagaatgatt tggatgaatt tgaggctcta gaaagaaaaa ctaaaaaaga tcaagagatg 1740
caactaattc atgaaatttc gaacttaaag aatttagtta agcatgcaga agtatataat 1800
caagatcttg agaatgaact cagttcaaaa gtagagctgc ttagagaaaa ggaagaccag 1860
attaagaagc tacaggaata catagactct caaaagctag aaaatataaa aatggacttg 1920
tcatactcat tggaaagcat tgaagaccca aaacaaatga agcagactct gtttgatgct 1980
gaaactgtag cccttgatgc caagagagaa tcagcctttc ttagaagtga aaatctggag 2040
ctgaaggaga aaatgaaaga acttgcaact acatacaagc aaatggaaaa tgatattcag 2100
ttatatcaaa gccagttgga ggcaaaaaag aaaatgcaag ttgatctgga gaaagaatta 2160
caatctgctt ttaatgagat aacaaaactc acctccctta tagatggcaa agttccaaaa 2220
gatttgctct gtaatttgga attggaagga aagattactg atcttcagaa agaactaaat 2280
aaagaagttg aagaaaatga agctttgcgg gaagaagtca ttttgctttc agaattgaaa 2340

tctttacctt ctgaagtaga aaggctgagg aaagagatac aagacaaatc tgaagagctc 2400
catataataa catcagaaaa agataaattg ttttctgaag tagttcataa ggagagtaga 2460
gttcaaggtt tacttgaaga aattgggaaa acaaaagatg acctagcaac tacacagtcg 2520
aattataaaa gcactgatca agaattccaa aatttcaaaa cccttcatat ggactttgag 2580
caaaagtata agatggtcct tgaggagaat gagagaatga atcaggaaat agttaatctc 2640
tctaaagaag cccaaaaatt tgattcgagt ttgggtgctt tgaagaccga gctttcttac 2700
aagacccaag aacttcagga gaaaacacgt gaggttcaag aaagactaaa tgagatggaa 2760
cagctgaagg aacaattaga aaatagagat tctacgctgc aaactgtaga aagggagaaa 2820
acactgatta ctgagaaact gcagcaaact ttagaagaag taaaaacttt aactcaagaa 2880
aaagatgatc taaaacaact ccaagaaagc ttgcaaattg agagggacca actcaaaagt 2940
gatattcacg atactgttaa catgaatata gatactcaag aacaattacg aaatgctctt 3000
gagtctctga aacaacatca agaaacaatt aatacactaa aatcgaaaat ttctgaggaa 3060
gtttccagga atttgcatat ggaggaaaat acaggagaaa ctaaagatga atttcagcaa 3120
aagatggttg gcatagataa aaaacaggat ttggaagcta aaaataccca aacactaact 3180
gcagatgtta aggataatga gataattgag caacaaagga agatattttc tttaatacag 3240
gagaaaaatg aactccaaca aatgttagag agtgttatag cagaaaagga acaattgaag 3300
actgacctaa aggaaaatat tgaaatgacc attgaaaacc aggaagaatt aagacttctt 3360
ggggatgaac ttaaaaagca acaagagata gttgcacaag aaaagaacca tgccataaag 3420
aaagaaggag agctttctag gacctgtgac agactggcag aagttgaaga aaaactaaag 3480
gaaaagagcc agcaactcca agaaaaacag caacaacttc ttaatgtaca agaagagatg 3540
agtgagatgc agaaaaagat taatgaaata gagaatttaa agaatgaatt aaagaacaaa 3600
gaattgacat tggaacatat ggaaacagag aggcttgagt tggctcagaa acttaatgaa 3660
aattatgagg aagtgaaatc tataaccaaa gaaagaaaag ttctaaagga attacagaag 3720
tcatttgaaa cagagagaga ccaccttaga ggatatataa gagaaattga agctacaggc 3780
ctacaaacca aagaagaact aaaaattgct catattcacc taaaagaaca ccaagaaact 3840
attgatgaac taagaagaag cgtatctgag aagacagctc aaataataaa tactcaggac 3900
ttagaaaaat cccataccaa attacaagaa gagatcccag tgcttcatga ggaacaagag 3960
ttactgccta atgtgaaaga agtcagtgag actcaggaaa caatgaatga actggagtta 4020
ttaacagaac agtccacaac caaggactca acaacactgg caagaataga aatggaaagg 4080
ctcaggttga atgaaaaatt tcaagaaagt caggaagaga taaaatctct aaccaaggaa 4140
agagacaacc ttaaaacgat aaaagaagcc cttgaagtta aacatgacca gctgaaagaa 4200
catattagag aaactttggc taaaatccag gagtctcaaa gcaaacaaga acagtcctta 4260

aatatgaaag aaaaagacaa tgaaactacc aaaatcgtga gtgagatgga gcaattcaaa 4320
cccaaagatt cagcactact aaggatagaa atagaaatgc tcggattgtc caaaagactt 4380
caagaaagtc atgatgaaat gaaatctgta gctaaggaga aagatgacct acagaggctg 4440
caagaagttc ttcaatctga aagtgaccag ctcaaagaaa acataaaaga aattgtagct 4500
aaacacctgg aaactgaaga ggaacttaaa gttgctcatt gttgcctgaa agaacaagag 4560
gaaactatta atgagttaag agtgaatctt tcagagaagg aaactgaaat atcaaccatt 4620
caaaagcagt tagaagcaat caatgataaa ttacagaaca agatccaaga gatttatgag 4680
aaagaggaac aatttaatat aaaacaaatt agtgaggttc aggaaaaagt gaatgaactg 4740
aaacaattca aggagcatcg caaagccaag gattcagcac tacaaagtat agaaagtaag 4800
atgctcgagt tgaccaacag acttcaagaa agtcaagaag aaatacaaat tatgattaag 4860
gaaaaagagg aaatgaaaag agtacaggag gcccttcaga tagagagaga ccaactgaaa 4920
gaaaacacta aagaaattgt agctaaaatg aaagaatctc aagaaaaaga atatcagttt 4980
cttaagatga cagctgtcaa tgagactcag gagaaaatgt gtgaaataga acacttgaag 5040
gagcaatttg agacccagaa gttaaacctg gaaaacatag aaacggagaa tataaggttg 5100
actcagatac tacatgaaaa ccttgaagaa atgagatctg taacaaaaga aagagatgac 5160
cttaggagtg tggaggagac tctcaaagta gagagagacc agctcaagga aaaccttaga 5220
gaaactataa ctagagacct agaaaaacaa gaggagctaa aaattgttca catgcatctg 5280
aaggagcacc aagaaactat tgataaacta agagggattg tttcagagaa aacaaatgaa 5340
atatcaaata tgcaaaagga cttagaacac tcaaatgatg ccttaaaagc acaggatctg 5400
aaaatacaag aggaactaag aattgctcac atgcatctga aagagcagca ggaaactatt 5460
gacaaactca gaggaattgt ttctgagaag acagataaac tatcaaatat gcaaaaagat 5520
ttagaaaatt caaatgctaa attacaagaa aagattcaag aacttaaggc aaatgaacat 5580
caacttatta cgttaaaaaa agatgtcaat gagacacaga aaaaagtgtc tgaaatggag 5640
caactaaaga aacaaataaa agaccaaagc ttaactctga gtaaattaga aatagagaat 5700
ttaaatttgg ctcagaaact tcatgaaaac cttgaagaaa tgaaatctgt aatgaaagaa 5760
agagataatc taagaagagt agaggagaca ctcaaactgg agagagacca actcaaggaa 5820
agcctgcaag aaaccaaagc tagagatctg gaaatacaac aggaactaaa aactgctcgt 5880
atgctatcaa aagaacacaa agaaactgtt gataaactta gagaaaaaat ttcagaaaag 5940
acaattcaaa tttcagacat tcaaaaggat ttagataaat caaaagatga attacagaaa 6000
aagatccaag aacttcagaa aaaagaactt caactgctta gagtgaaaga agatgtcaat 6060
atgagtcata aaaaaattaa tgaaatggaa cagttgaaga agcaatttga ggcccaaaac 6120

ttatctatgc aaagtgtgag aatggataac ttccagttga ctaagaaact tcatgaaagc 6180
cttgaagaaa taagaattgt agctaaagaa agagatgagc taaggaggat aaaagaatct 6240
ctcaaaatgg aaagggacca attcatagca accttaaggg aaatgatagc tagagaccga 6300
cagaaccacc aagtaaaacc tgaaaaaagg ttactaagtg atggacaaca gcaccttacg 6360
gaaagcctga gagaaaagtg ctctagaata aaagagcttt tgaagagata ctcagagatg 6420
gatgatcatt atgagtgctt gaatagattg tctcttgact tggagaagga aattgaattc 6480
caaaaagagc tttcaatgag agttaaagca aacctctcac ttccctattt acaaaccaaa 6540
cacattgaaa aactttttac tgcaaaccag agatgctcca tggaattcca cagaatcatg 6600
aagaaactga agtatgtgtt aagctatgtt acaaaaataa aagaagaaca acatgaatcc 6660
atcaataaat ttgaaatgga ttttattgat gaagtggaaa agcaaaagga attgctaatt 6720
aaaatacagc accttcaaca agattgtgat gtaccatcca gagaattaag ggatctcaaa 6780
ttgaaccaga atatggatct acatattgag gaaattctca aagatttctc agaaagtgag 6840
ttccctagca taaagactga atttcaacaa gtactaagta ataggaaaga aatgacacag 6900
tttttggaag agtggttaaa tactcgtttt gatatagaaa agcttaaaaa tggcatccag 6960
aaagaaaatg ataggatttg tcaagtgaat aacttcttta ataacagaat aattgccata 7020
atgaatgaat caacagagtt tgaggaaaga agtgctacca tatccaaaga gtgggaacag 7080
gacctgaaat cactgaaaga gaaaaatgaa aaactattta aaaactacca aacattgaag 7140
acttccttgg catctggtgc ccaggttaat cctaccacac aagacaataa gaatcctcat 7200
gttacatcaa gagctacaca gttaaccaca gagaaaattc gagagctgga aaattcactg 7260
catgaagcta aagaaagtgc tatgcataag gaaagcaaga ttataaagat gcagaaagaa 7320
cttgaggtga ctaatgacat aatagcaaaa cttcaagcca aagttcatga atcaaataaa 7380
tgccttgaaa aaacaaaaga gacaattcaa gtacttcagg acaaagttgc tttaggagct 7440
aagccatata aagaagaaat tgaagatctc aaaatgaagc ttgtgaaaat agacctagag 7500
aaaatgaaaa atgccaaaga atttgaaaag gaaatcagtg ctacaaaagc cactgtagaa 7560
tatcaaaagg aagttataag gctattgaga gaaaatctca gaagaagtca acaggcccaa 7620
gatacctcag tgatatcaga acatactgat cctcagcctt caaataaacc cttaacttgt 7680
ggaggtggca gcggcattgt acaaaacaca aaagctctta ttttgaaaag tgaacatata 7740
aggctagaaa aagaaatttc taagttaaag cagcaaaatg aacagctaat aaaacaaaag 7800
aatgaattgt taagcaataa tcagcatctt tccaatgagg tcaaaacttg gaaggaaaga 7860
acccttaaaa gagaggctca caaacaagta acttgtgaga attctccaaa gtctcctaaa 7920
gtgactggaa cagcttctaa aaagaaacaa attacaccct ctcaatgcaa ggaacggaat 7980
ttacaagatc ctgtgccaaa ggaatcacca aaatcttgtt tttttgatag ccgatcaaag 8040

tctttaccat cacctcatcc agttcgctat tttgataact caagtttagg cctttgtcca 8100

gaggtgcaaa atgcaggagc agagagtgtg gattctcagc caggtccttg gcacgcctcc 8160

tcaggcaagg atgtgcctga gtgcaaaact cagtagactc ctctttgtca cttctctgga 8220

gatccagcat tccttatttg gaaatgactt tgtttatgtg tctatccctg gtaatgatgt 8280

tgtagtgcag cttaatttca attcagtctt tactttgcca ctagagttga aagataaggg 8340

aacaggaaat gaatgcattg tggtaattta gaatggtgat agcaatacct tcttcttgca 8400

tatggtaata cttttaaaag ttgaattgtt ttatttattt gtatattttg taaagaataa 8460

agttattgaa agaaatgtaa agttatctac atgacttagc atattccaaa gcataataca 8520

tacattaata taaaacatca ttttattaac aaaattgtaa atgtttttaa taccttacac 8580

attcaataaa tgtttagtag ttctgaatca ccaaaaaaaa aaaaaaaaaa 8630

<210> 12
<211> 2319
<212> DNA
<213> Homo sapiens

<400> 12
gtggagtttg aattgggtgg cggttgactg tagagccgct ctctctcact ggcacagcga 60

ggttttgctc agcccttgtc tcgggaccgc agcctccgcc gagcgccatg gctcctagga 120

agggcagtag tcgggtggcc aagaccaact ccttacggag gcggaagctc gcctcctttc 180

tgaaagactt cgaccgtgaa gtggaaatac gaatcaagca aattgagtca gacaggcaga 240

acctcctcaa ggaggtggat aacctctaca acatcgagat cctgcggctc cccaaggctc 300

tgcgcgagat gaactggctt gactacttcg cccttggagg aaacaaacag gccctggaag 360

aggcggcaac agctgacctg gatatcaccg aaataaacaa actaacagca gaagctattc 420

agacacccct gaaatctgcc aaaacacgaa aggtaataca ggtagatgaa atgatagtgg 480

aagaggaaga agaagaagaa aatgaacgta agaatcttca aactgcaaga gtcaaaaggt 540

gtcctccatc caagaagaga actcagtcca tacaaggaaa aggaaaaggg aaaaggtcaa 600

gccgtgctaa cactgttacc ccagccgtgg gccgattgga ggtgtccatg gtcaaaccaa 660

ctccaggcct gacacccagg tttgactcaa gggtcttcaa gacccctggc ctgcgtactc 720

cagcagcagg agagcggatt tacaacatct cagggaatgg cagccctctt gctgacagca 780

aagagatctt cctcactgtg ccagtgggcg gcggagagag cctgcgatta ttggccagtg 840

acttgcagag gcacagtatt gcccagctgg atccagaggc cttgggaaac attaagaagc 900

tctccaaccg tctcgcccaa atctgcagca gcatacggac ccacaaatga gacaccaaag 960

ttgacaggat ggacttttaa tgggcacttc tgggaccctg aagagacttc ttcccttcag 1020

gcttattgtt tgagtgtgaa gttccagagc aaggagccat gttcctctaa gggaattcag 1080

gaattcagac gtgctagtcc cacaccagtt aggtagagct gtctgttcac cctcccatcc 1140

cagctgatcc cagtcactgc ttgctggggc catgccatgg aagcttccca tcagtctccc 1200

agctgaatcc tccctgctct ctgagctgct gccttttgcc tcctgcaact caacatcctc 1260

ttcaccctgc cctgcctgca gttgaggggg cgaagaagaa ccctgtgttc tcaggaagac 1320

tgcctccacc accgctaccc agagaacctc tgcatctggc atttctgctc tctatgcttg 1380

agaccgggag gtttaggctc agataagtga gctctgggcc atgagagggt aggtccagaa 1440

ggtgggggga actgtacaga tcagcagagc aggacagttg gcagcagtga cctcagtagg 1500

gaacatgtcc gtctaccctc tcgcactcat gacacctccc cctaccagcc ctcctcttcc 1560

tcctcctcct cctcctgtgg gaggtggtca gtgggactta gggatctttc acctgctgtg 1620

cccagtagtt ctgaagtctg cttgtggagc agtgttttat gtttatccct gtttactgaa 1680

gaccaaatac tggtttggag acaacttcca tgtcttgctc ttctacctcc ctagttagtg 1740

gaaatttgga taagggaact gtagggccca gattctggag gttttatgtc attggccaca 1800

gaataactgt ctctaagcta tccatggtcc agtggtccct gccaagtctg tagacttcag 1860

agagcacttc tctcttatgg ggttcatggg aacaggggtg ggtgtgactt gcttggtggc 1920

ctcattccat gtgtgcctgt gcctggggca tggactttgt taagcagagt cagcagtgag 1980

gtcctcattc tccagccagc ctctctgccc tggagaatca tgtgctatgt tctaagaatt 2040

tgagaactag agtcctcatc cccaggcttg aaggcacatg gctttctcat gtagggctct 2100

ctgtggtatt tgttattatt ttgcaacaag accattttag taaaacagtc ctgttcaagt 2160

tgtattcttt taagttcttt tattctcctt tccctgagat ttttgtatat attgttctga 2220

gtaatggtat ctttgagctg attgttctaa tcagagctgg tacctacttt caataaattc 2280

tggttttgtg ttttcttttg taaaaaaaaa aaaaaaaaa 2319

<210> 13
<211> 1720
<212> DNA
<213> Homo sapiens

<400> 13
gtctctcctg tctgaaggcc agagcaggct gctaggcctg gggccaccac tgcccctggg 60

tgctacaccc agtgtgctgg gtcactggga acttcctgaa gtggtgtcac ctgaactggg 120

cccccaagga tggggtgcgg gcagtaccgc aggaagagga gcagcccctg tgaagattga 180

gagctgccag aggctctgtg attggctgcg gcacgatgac ccgcgcacgg attggctgct 240

tcgggccggg gggccgggcc cgggggacag aatccgcccc cgaaccttca aagagggtac 300

cccccggcag gagctggcag acccaggagg tgcgacagac ccgcggggca aacggactgg 360

ggccaagagc cgggagcgcg ggcgcaaagg caccagggcc cgcccagggc gccgcgcagc 420

```
acggccttgg gggttctgcg ggccttcggg tgcgcgtctc gcctctagcc atggggtccg     480

cagcgttgga gatcctgggc ctggtgctgt gcctggtggg ctgggggggt ctgatcctgg     540

cgtgcgggct gcccatgtgg caggtgaccg ccttcctgga ccacaacatc gtgacggcgc     600

agaccacctg gaaggggctg tggatgtcgt gcgtggtgca gagcaccggg cacatgcagt     660

gcaaagtgta cgactcggtg ctggctctga gcaccgaggt gcaggcggcg cgggcgctca     720

ccgtgagcgc cgtgctgctg gcgttcgttg cgctcttcgt gaccctggcg ggcgcgcagt     780

gcaccacctg cgtggccccg ggcccggcca aggcgcgtgt ggccctcacg ggaggcgtgc     840

tctacctgtt ttgcgggctg ctggcgctcg tgccactctg ctggttcgcc aacattgtcg     900

tccgcgagtt ttacgacccg tctgtgcccg tgtcgcagaa gtacgagctg ggcgcagcgc     960

tgtacatcgg ctgggcggcc accgcgctgc tcatggtagg cggctgcctc ttgtgctgcg    1020

gcgcctgggt ctgcaccggc cgtcccgacc tcagcttccc cgtgaagtac tcagcgccgc    1080

ggcggcccac ggccaccggc gactacgaca agaagaacta cgtctgaggg cgctgggcac    1140

ggccgggccc ctcctgccag ccacgcctgc gaggcgttgg ataagcctgg ggagccccgc    1200

atggaccgcg gcttccgccg ggtagcgcgg cgcgcaggct cctcggaacg tccggctctg    1260

cgccccgacg cggctcctgg atccgctcct gcctgcgccc gcagctgacc ttctcctgcc    1320

actagcccgg ccctgccctt aacagacgga atgaagtttc cttttctgtg cgcggcgctg    1380

tttccatagg cagagcgggt gtcagactga ggatttcgct tcccctccaa gacgctgggg    1440

gtcttggctg ctgccttact tcccagaggc tcctgctgac ttcggagggg cggatgcaga    1500

gcccagggcc cccaccggaa gatgtgtaca gctggtcttt actccatcgg cagggcccga    1560

gcccagggac cagtgacttg gcctggacct cccggtctca ctccagcatc tccccaggca    1620

aggcttgtgg gcaccggagc ttgagagagg gcgggagtgg gaaggctaag aatctgctta    1680

gtaaatggtt tgaactctct ccaaaaaaaa aaaaaaaaaa                          1720


<210>  14
<211>  1582
<212>  DNA
<213>  Homo sapiens

<400>  14
gcttaggctg agccgtggcc gccacagccc atcgtaatgc cgcatggtgc ttggcactcc      60

agagagccaa taggaatgaa agaattcatt tgaatcggcc aatgccggcg ggttaggggg     120

cgggggttga aaaccctata aaggcgtcga tcggccggac aggcggcagc ggcggctcct     180

gcagcggtgg tcggctgttg ggtgtggagt ttcccagcgc ccctcgggtc cgaccctttg     240

agcgttctgc tccggcgcca gcctacctcg ctcctcggcg ccatgaccac aaccaccacc     300

ttcaagggag tcgaccccaa cagcaggaat agctcccgag ttttgcggcc tccaggtggt     360
```

```
ggatccaatt tttcattagg ttttgatgaa ccaacagaac aacctgtgag gaagaacaaa     420
atggcctcta atatctttgg gacacctgaa gaaaatcaag cttcttgggc caagtcagca     480
ggtgccaagt ctagtggtgg cagggaagac ttggagtcat ctggactgca gagaaggaac     540
tcctctgaag caagctccgg agacttctta gatctgaaga aaatgtggac acagacttgc     600
caggcagcct ggggcagagt gaagagaagc ccgtgcctgc tgcgcctgtg cccagcccgg     660
tggccccggc cccagtgcca tccagaagaa atccccctgg cggcaagtcc agcctcgtct     720
tgggttagct ctgactgtcc tgaacgctgt cgttctgtct gtttcctcca tgcttgtgaa     780
ctgcacaact tgagcctgac tgtacatctc ttggatttgt ttcattaaaa agaagcactt     840
tatgtactgc tgtctttttt tttttctttt tgaagaacag gtttctctct gtccttgact     900
cttgggtctg tgggccatgg catgagtgtt ttctagtagt agattggagg gaaagctttg     960
tgacacttag tactgtgttt ttaagaagaa ataatttggt tccagatgtg ttagaggatc    1020
ttttgtactg aggtttttaa cactttactt gggtttacca agcctcaact ggacagacca    1080
taaacagtcc acaggcaccg ttcctgccag gccccaaccc acagggagtc tctccgcaga    1140
gccttcttgg tgttgcccta acttgccagt ggcctttgct cagagcctcc tcctgtgaca    1200
tgtgaacaat gaagaggcct gcgcctcctg ccttgccgcc tgcaaagcaa agaaactgcc    1260
ttttattttt taaccttaaa aagtagccag atagtaacaa gactggctgg ctgatgagca    1320
aagcctttgc tctcacgcag aggaaggctt ggatgtacaa tgaaactgcc tggaactaaa    1380
agcagtgaag caagggaggc aatcacactg aagcgggtct tcctccagga acggggtccc    1440
acaggcgtgt tgttttaaat aacctgatgc tgtgtgcatg atgctggtgc ttgaccatga    1500
aaggaaagtc tcatccttaa aatgtgttgt acttcacaat cctggactgt tgcttcaagt    1560
aaacaatatc cacattttga aa                                             1582
```

<210> 15
<211> 1696
<212> DNA
<213> Homo sapiens

<400> 15

```
gcttaggctg agccgtggcc gccacagccc atcgtaatgc cgcatggtgc ttggcactcc      60
agagagccaa taggaatgaa agaattcatt tgaatcggcc aatgccggcg ggttaggggg     120
cgggggttga aaaccctata aaggcgtcga tcggccggac aggcggcagc ggcggctcct     180
gcagcggtgg tcggctgttg ggtgtggagt ttcccagcgc ccctcgggtc cgaccctttg     240
agcgttctgc tccggcgcca gcctacctcg ctcctcggcg ccatgaccac aaccaccacc     300
ttcaagggag tcgaccccaa cagcaggaat agctcccgag acacggggtc ttgccatgtt     360
gcccaggctg gtcttgaact cctaggctca agtgatgatc ctgccttggc ctcctagggt     420
```

gctgggatta cagagttttg cggcctccag gtggtggatc caatttttca ttaggttttg 480

atgaaccaac agaacaacct gtgaggaaga acaaaatggc ctctaatatc tttgggacac 540

ctgaagaaaa tcaagcttct tgggccaagt cagcaggtgc caagtctagt ggtggcaggg 600

aagacttgga gtcatctgga ctgcagagaa ggaactcctc tgaagcaagc tccggagact 660

tcttagatct gaagggagaa ggtgatattc atgaaaatgt ggacacagac ttgccaggca 720

gcctggggca gagtgaagag aagcccgtgc ctgctgcgcc tgtgcccagc ccggtggccc 780

cggccccagt gccatccaga agaaatcccc ctggcggcaa gtccagcctc gtcttgggtt 840

agctctgact gtcctgaacg ctgtcgttct gtctgtttcc tccatgcttg tgaactgcac 900

aacttgagcc tgactgtaca tctcttggat ttgtttcatt aaaaagaagc actttatgta 960

ctgctgtctt tttttttttt cttttgaaga acaggtttct ctctgtcctt gactcttggg 1020

tctgtgggcc atggcatgag tgttttctag tagtagattg gagggaaagc tttgtgacac 1080

ttagtactgt gtttttaaga agaaataatt tggttccaga tgtgttagag gatcttttgt 1140

actgaggttt ttaacacttt acttgggttt accaagcctc aactggacag accataaaca 1200

gtccacaggc accgttcctg ccaggcccca acccacaggg agtctctccg cagagccttc 1260

ttggtgttgc cctaacttgc cagtggcctt tgctcagagc ctcctcctgt gacatgtgaa 1320

caatgaagag gcctgcgcct cctgccttgc cgcctgcaaa gcaaagaaac tgccttttat 1380

tttttaacct taaaaagtag ccagatagta acaagactgg ctggctgatg agcaaagcct 1440

ttgctctcac gcagaggaag gcttggatgt acaatgaaac tgcctggaac taaaagcagt 1500

gaagcaaggg aggcaatcac actgaagcgg gtcttcctcc aggaacgggg tcccacaggc 1560

gtgttgtttt aaataacctg atgctgtgtg catgatgctg gtgcttgacc atgaaaggaa 1620

agtctcatcc ttaaaatgtg ttgtacttca caatcctgga ctgttgcttc aagtaaacaa 1680

tatccacatt ttgaaa 1696

<210> 16
<211> 1582
<212> DNA
<213> Homo sapiens

<400> 16
gcttaggctg agccgtggcc gccacagccc atcgtaatgc cgcatggtgc ttggcactcc 60

agagagccaa taggaatgaa agaattcatt tgaatcggcc aatgccggcg ggttaggggg 120

cgggggttga aaccctata aaggcgtcga tcggccggac aggcggcagc ggcggctcct 180

gcagcggtgg tcggctgttg ggtgtggagt ttcccagcgc ccctcgggtc cgaccctttg 240

agcgttctgc tccggcgcca gcctacctcg ctcctcggcg ccatgaccac aaccaccacc 300

ttcaagggag tcgaccccaa cagcaggaat agctcccgag ttttgcggcc tccaggtggt 360

ggatccaatt tttcattagg ttttgatgaa ccaacagaac aacctgtgag gaagaacaaa 420

atggcctcta atatctttgg gacacctgaa gaaaatcaag cttcttgggc caagtcagca 480

ggtgccaagt ctagtggtgg cagggaagac ttggagtcat ctggactgca gagaaggaac 540

tcctctgaag caagctccgg agacttctta gatctgaaga aaatgtggac acagacttgc 600

caggcagcct ggggcagagt gaagagaagc ccgtgcctgc tgcgcctgtg cccagcccgg 660

tggccccggc cccagtgcca tccagaagaa atccccctgg cggcaagtcc agcctcgtct 720

tgggttagct ctgactgtcc tgaacgctgt cgttctgtct gtttcctcca tgcttgtgaa 780

ctgcacaact tgagcctgac tgtacatctc ttggatttgt ttcattaaaa agaagcactt 840

tatgtactgc tgtctttttt ttttttcttt tgaagaacag gtttctctct gtccttgact 900

cttgggtctg tgggccatgg catgagtgtt ttctagtagt agattggagg gaaagctttg 960

tgacacttag tactgtgttt ttaagaagaa ataatttggt tccagatgtg ttagaggatc 1020

ttttgtactg aggtttttaa cactttactt gggtttacca agcctcaact ggacagacca 1080

taaacagtcc acaggcaccg ttcctgccag gccccaaccc acagggagtc tctccgcaga 1140

gccttcttgg tgttgcccta acttgccagt ggcctttgct cagagcctcc tcctgtgaca 1200

tgtgaacaat gaagaggcct gcgcctcctg ccttgccgcc tgcaaagcaa agaaactgcc 1260

ttttattttt taaccttaaa aagtagccag atagtaacaa gactggctgg ctgatgagca 1320

aagcctttgc tctcacgcag aggaaggctt ggatgtacaa tgaaactgcc tggaactaaa 1380

agcagtgaag caagggaggc aatcacactg aagcgggtct tcctccagga acggggtccc 1440

acaggcgtgt tgttttaaat aacctgatgc tgtgtgcatg atgctggtgc ttgaccatga 1500

aaggaaagtc tcatccttaa aatgtgttgt acttcacaat cctggactgt tgcttcaagt 1560

aaacaatatc cacattttga aa 1582

<210> 17
<211> 3824
<212> DNA
<213> Homo sapiens

<400> 17
ggaagcgcag agcaggttca aacacagacg gcgggtgaac atggcgtcct cgacttggtc 60

tgagacgtga taggcctgcc ttctggttga agatgtggcg agtgaaaaaa ctgagcctca 120

gcctgtcgcc ttcgccccag acgggaaaac catctatgag aactcctctc cgtgaactta 180

ccctgcagcc cggtgccctc accaactctg gaaaaagatc cccgcttgc tcctcgctga 240

ccccatcact gtgcaagctg gggctgcagg aaggcagcaa caactcatct ccagtggatt 300

ttgtaaataa caagaggaca gacttatctt cagaacattt cagtcattcc tcaaagtggc 360

tagaaacttg tcagcatgaa tcagatgagc agcctctaga tccaattccc caaattagct 420

ctactcctaa aacgtctgag gaagcagtag acccactggg caattatatg gttaaaacca 480
tcgtccttgt accatctcca ctggggcagc aacaagacat gatatttgag gcccgtttag 540
ataccatggc agagacaaac agcatatctt taaatggacc tttgagaaca gacgatctgg 600
tgagagagga ggtggcaccc tgcatgggag acaggttttc agaagttgct gctgtatctg 660
agaaacctat ctttcaggaa tctccgtccc atctcttaga ggagtctcca ccaaatccct 720
gttctgaaca actacattgc tccaaggaaa gcctgagcag tagaactgag gctgtgcgtg 780
aggacttagt accttctgaa agtaacgcct tcttgccttc ctctgttctc tggctttccc 840
cttcaactgc cttggcagca gatttccgtg tcaatcatgt ggacccagag gaggaaattg 900
tagagcatgg agctatggag gaaagagaaa tgaggtttcc cacacatcct aaggagtctg 960
aaacagaaga tcaagcactt gtctcaagtg tggaagatat tctgtccaca tgcctgacac 1020
caaatctagt agaaatggaa tcccaagaag ctccaggccc agcagtagaa gatgttggta 1080
ggattcttgg ctctgataca gagtcttgga tgtccccact ggcctggctg gaaaaaggtg 1140
taaatacctc cgtcatgctg gaaaatctcc gccaaagctt atcccttccc tcgatgcttc 1200
gggatgctgc aattggcact accccttttct ctacttgctc ggtggggact tggtttactc 1260
cttcagcacc acaggaaaag agtacaaaca catcccagac aggcctggtt ggcaccaagc 1320
acagtacttc tgagacagag cagctcctgt gtggccggcc tccagatctg actgccttgt 1380
ctcgacatga cttggaagat aacctgctga gctctcttgt cattctggag gttctctccc 1440
gccagcttcg ggactggaag agccagctgg ctgtccctca cccagaaacc caggacagta 1500
gcacacagac tgacacatct cacagtggga taactaataa acttcagcat cttaaggaga 1560
gccatgagat gggacaggcc ctacagcagg ccagaaatgt catgcaatca tgggtgctta 1620
tctctaaaga gctgatatcc ttgcttcacc tatccctgtt gcatttagaa gaagataaga 1680
ctactgtgag tcaggagtct cggcgtgcag aaacattggt ctgttgctgt tttgatttgc 1740
tgaagaaatt gagggcaaag ctccagagcc tcaaagcaga aagggaggag gcaaggcaca 1800
gagaggaaat ggctctcaga ggcaaggatg cggcagagat agtgttggag gctttctgtg 1860
cacacgccag ccagcgcatc agccagctgg aacaggacct agcatccatg cgggaattca 1920
gaggccttct gaaggatgcc cagacccaac tggtagggct tcatgccaag caagaagagc 1980
tggttcagca gacagtgagt cttacttcta ccttgcaaca agactggagg tccatgcaac 2040
tggattatac aacatggaca gctttgctga gtcggtcccg acaactcaca gagaaactca 2100
cagtcaagag ccagcaagcc ctgcaggaac gtgatgtggc aattgaggaa aagcaggagg 2160
tttctagggt gctggaacaa gtctctgccc agttagagga gtgcaaaggc caaacagaac 2220
aactggagtt ggaaaacagt cgtctagcaa cagatctccg ggctcagttg cagattctgg 2280

ccaacatgga cagccagcta aaagagctac agagtcagca tacccattgt gcccaggacc 2340

tggctatgaa ggatgagtta ctctgccagc ttacccagag caatgaggag caggctgctc 2400

aatggcaaaa ggaagagatg gcactaaaac acatgcaggc agaactgcag cagcaacaag 2460

ctgtcctggc caaagaggtg cgggacctga aagagacctt ggagtttgca gaccaggaga 2520

atcaggttgc tcacctggag ctgggtcagg ttgagtgtca attgaaaacc acactggaag 2580

tgctccggga gcgcagcttg cagtgtgaga acctcaagga cactgtagag aacctaacgg 2640

ctaaactggc cagcaccata gcagataacc aggagcaaga tctggagaaa acacggcagt 2700

actctcaaaa gctagggctg ctgactgagc aactacagag cctgactctc tttctacaga 2760

caaaactaaa ggagaagact gaacaagaga cccttctgct gagtacagcc tgtcctccca 2820

cccaggaaca ccctctgcct aatgacagga ccttcctggg aagcatcttg acagcagtgg 2880

cagatgaaga gccagaatca actcctgtgc ccttgcttgg aagtgacaag agtgctttca 2940

cccgagtagc atcaatggtt tcccttcagc ccgcagagac cccaggcatg gaggagagcc 3000

tggcagaaat gagtattatg actactgagc ttcagagtct ttgttccctg ctacaagagt 3060

ctaaagaaga agccatcagg actctgcagc gaaaaatttg tgagctgcaa gctaggctgc 3120

aggcccagga agaacagcat caggaagtcc agaaggcaaa agaagcagac atagagaagc 3180

tgaaccaggc cttgtgcttg cgctacaaga atgaaaagga gctccaggaa gtgatacagc 3240

agcagaatga gaagatccta gaacagatag acaagagtgg cgagctcata agccttagag 3300

aggaggtgac ccaccttacc cgctcacttc ggcgtgcgga gacagagacc aaagtgctcc 3360

aggaggccct ggcaggccag ctggactcca actgccagcc tatggccacc aattggatcc 3420

aggagaaagt gtggctctct caggaggtgg acaaactgag agtgatgttc ctggagatga 3480

aaaatgagaa ggaaaaactc atgatcaagt tccagagcca tagaaatatc ctagaggaga 3540

accttcggcg ctctgacaag gagttagaaa aactagatga cattgttcag catatttata 3600

agaccctgct ctctattcca gaggtggtga ggggatgcaa agaactacag ggattgctgg 3660

aatttctgag ctaagaaact gaaagccaga atctgcttca cctcttttta cctgcaatac 3720

ccccttaccc caataccaag accaactggc atagagccaa ctgagataaa tgctatttaa 3780

ataaagtgta tttaatgaat ttctccaaaa aaaaaaaaaa aaaa 3824

<210> 18
<211> 4224
<212> DNA
<213> Homo sapiens

<400> 18
gcgaaattca agctccaaac tctaagctcc aagctccaag ctccaagctc caagctccaa 60

actcccgccg gggtaactgg aacccaatcc gagggtcatg gaggcatccc gaaggtttcc 120

ggaagccgag gccttgagcc cagagcaggc tgctcattac ctaagatatg tgaaagaggc 180
caaagaagca actaagaatg gagacctgga agaagcattt aaacttttca atttggcaaa 240
ggacattttt cccaatgaaa aagtgctgag cagaatccaa aaaatacagg aagccttgga 300
ggagttggca gaacagggag atgatgaatt tacagatgtg tgcaactctg gcttgctact 360
ttatcgagaa ctgcacaacc aactctttga gcaccagaag gaaggcatag ctttcctcta 420
tagcctgtat agggatggaa gaaaaggtgg tatattggct gatgatatgg gattagggaa 480
gactgttcaa atcattgctt tcctttccgg tatgtttgat gcatcacttg tgaatcatgt 540
gctgctgatc atgccaacca atcttattaa cacatgggta aaagaattca tcaagtggac 600
tccaggaatg agagtcaaaa cctttcatgg tcctagcaag gatgaacgga ccagaaacct 660
caatcggatt cagcaaagga atggtgttat tatcactaca taccaaatgt taatcaataa 720
ctggcagcaa ctttcaagct ttaggggcca agagtttgtg tgggactatg tcatcctcga 780
tgaagcacat aaaataaaaa cctcatctac taagtcagca atatgtgctc gtgctattcc 840
tgcaagtaat cgcctcctcc tcacaggaac cccaatccag aataatttac aagaactatg 900
gtccctattt gattttgctt gtcaagggtc cctgctggga acattaaaaa cttttaagat 960
ggagtatgaa aatcctatta ctagagcaag agagaaggat gctaccccag gagaaaaagc 1020
cttgggattt aaaatatctg aaaacttaat ggcaatcata aaaccctatt ttctcaggag 1080
gactaaagaa gacgtacaga agaaaaagtc aagcaaccca gaggccagac ttaatgaaaa 1140
gaatccagat gttgatgcca tttgtgaaat gccttccctt tccaggaaaa atgatttaat 1200
tatttggata cgacttgtgc ctttacaaga agaaatatac aggaaatttg tgtctttaga 1260
tcatatcaag gagttgctaa tggagacgcg ctcacctttg gctgagctag gtgtcttaaa 1320
gaagctgtgt gatcatccta ggctgctgtc tgcacgggct tgttgtttgc taaatcttgg 1380
gacattctct gctcaagatg gaaatgaggg ggaagattcc ccagatgtgg accatattga 1440
tcaagtaact gatgacacat tgatggaaga atctggaaaa atgatattcc taatggacct 1500
acttaagagg ctgcgagatg agggacatca aactctggtg ttttctcaat cgaggcaaat 1560
tctaaacatc attgaacgcc tcttaaagaa taggcacttt aagacattgc gaatcgatgg 1620
gacagttact catcttttgg aacgagaaaa aagaattaac ttattccagc aaaataaaga 1680
ttactctgtt tttctgctta ccactcaagt aggtggtgtc ggtttaacat taactgcagc 1740
aactagagtg gtcatttttg accctagctg gaatcctgca actgatgctc aagctgtgga 1800
tagagtttac cgaattggac aaaaagagaa tgttgtggtt tataggctaa tcacttgtgg 1860
gactgtagag gaaaaaatat acagaagaca ggttttcaag gactcattaa taagacaaac 1920
tactggtgaa aaaaagaacc ctttccgata ttttagtaaa caagaattaa gagagctctt 1980
tacaatcgag gatcttcaga actctgtaac ccagctgcag cttcagtctt tgcatgctgc 2040

tcagaggaaa tctgatataa aactagatga acatattgcc tacctgcagt ctttggggat 2100
agctggaatc tcagaccatg atttgatgta cacatgtgat ctgtctgtta aagaagagct 2160
tgatgtggta gaagaatctc actatattca acaaagggtt cagaaagctc aattcctcgt 2220
tgaattcgag tctcaaaata aagagttcct gatggaacaa caaagaacta gaaatgaggg 2280
ggcctggcta agagaacctg tatttccttc ttcaacaaag aagaaatgcc ctaaattgaa 2340
taaaccacag cctcagcctt cacctcttct aagtactcat catactcagg aagaagatat 2400
cagttccaaa atggcaagtg tagtcattga tgatctgccc aaagagggtg agaaacaaga 2460
tctctccagt ataaaggtga atgttaccac cttgcaagat ggtaaaggta caggtagtgc 2520
tgactctata gctactttac caaaggggtt tggaagtgta gaagaacttt gtactaactc 2580
ttcattggga atggaaaaaa gctttgcaac taaaaatgaa gctgtacaaa aagagacatt 2640
acaagagggg cctaagcaag aggcactgca agaggatcct ctggaaagtt ttaattatgt 2700
acttagcaaa tcaaccaaag ctgatattgg gccaaattta gatcaactaa aggatgatga 2760
gattttacgt cattgcaatc cttggcccat tatttccata acaaatgaaa gtcaaaatgc 2820
agaatcaaat gtatccatta ttgaaatagc tgatgacctt tcagcatccc atagtgcact 2880
gcaggatgct caagcaagtg aggccaagtt ggaagaggaa ccttcagcat cttcaccaca 2940
gtatgcatgt gatttcaatc ttttcttgga agactcagca gacaacagac aaaatttttc 3000
cagtcagtct ttagagcatg ttgagaaaga aaatagcttg tgtggctctg cacctaattc 3060
cagagcaggg tttgtgcata gcaaaacatg tctcagttgg gagttttctg agaaagacga 3120
tgaaccagaa gaagtagtag ttaaagcaaa aatcagaagt aaagctagaa ggattgtttc 3180
agatggcgaa gatgaagatg attcttttaa agatacctca agcataaatc cattcaacac 3240
atctctcttt caattctcat ctgtgaaaca atttgatgct tcaactccca aaaatgacat 3300
cagtccacca ggaaggttct tttcatctca aatacccagt agtgtaaata agtctatgaa 3360
ctctagaaga tctctggctt ctaggaggtc tcttattaat atggttttag accacgtgga 3420
ggacatggag gaaagacttg acgacagcag tgaagcaaag ggtcctgaag attatccaga 3480
agaaggggtg gaggaaagca gtggcgaagc ctccaagtat acagaagagg atccttccgg 3540
agaaacactg tcttcagaaa acaagtccag ctggttaatg acgtctaagc ctagtgctct 3600
agctcaagag acctctcttg gtgcccctga gcctttgtct ggtgaacagt tggttggttc 3660
tccccaggat aaggcggcag aggctacaaa tgactatgag actcttgtaa agcgtggaaa 3720
agaactaaaa gagtgtggaa aaatccagga ggccctaaac tgcttagtta aagcgcttga 3780
cataaaaagt gcagatcctg aagttatgct cttgacttta agtttgtata agcaacttaa 3840
taacaattga gaatgtaacc tgtttattgt attttaaagt gaaactgaat atgagggaat 3900

```
ttttgttccc ataattggat tctttgggaa catgaagcat tcaggcttaa ggcaagaaag   3960
atctcaaaaa gcaacttctg ccctgcaacg ccccccactc catagtctgg tattctgagc   4020
actagcttaa tatttcttca cttgaatatt cttatatttt aggcatattc tataaattta   4080
actgtgttgt ttcttggaaa gttttgtaaa attattctgg tcattcttaa ttttactctg   4140
aaagtgatca tctttgtata taacagttca gataagaaaa ttaaagttac ttttctcaag   4200
tgttttcaaa aaaaaaaaaa aaaa                                          4224

<210>  19
<211>  4495
<212>  DNA
<213>  Homo sapiens

<400>  19
gccggcgacg tcacgcggcc gttacggcgc tcaggcgtct cgacgcgcgc gatttaaaac     60
cagctcagga gacgccaagg aaagatggga cctcccggcc cagcactgcc agccacaatg    120
aataactctt cttcagagac gcgaggacac ccccacagtg cctcctctcc ttcagagcgt    180
gtgttcccga tgcccctgcc caggaaggcg cctctcaata ttcctggcac cccagtcctc    240
gaagactttc ctcagaatga cgatgagaag gagcggctgc agcggaggcg ctcgagggtc    300
tttgatctgc agttcagcac tgactcacct cgcttattgg cctccccctc cagcaggagt    360
attgacattt cagctactat ccccaagttt acaaacacgc agattacgga acattactcc    420
acctgtatca aactgtccac tgaaaataaa atcactacca agaatgcttt tggtttgcac    480
ttgattgatt ttatgtcaga gattcttaaa cagaaagaca ccgaaccaac caactttaaa    540
gtggctgcgg gtactctgga tgccagcacc aagatctatg ctgtgcgcgt ggatgccgtc    600
catgccgatg tatacagagt ccttgggggg ctgggcaaag atgcaccgtc tttggaagaa    660
gtagaaggcc atgttgctga tggaagtgct actgaaatgg gaacaaccaa aaaggctgta    720
aagccaaaga agaagcactt acacagaact attgagcaga acataaacaa cctcaatgtc    780
tccgaagcag atcggaagtg tgagattgat cccatgtttc agaagacagc agcctcattt    840
gatgagtgca gcacagcagg ggtgtttctg tccactctcc actgccagga ctacagaagt    900
gaactgctgt ttccctctga tgtccagact ctctccacgg gagaacctct cgagttgcca    960
gagttaggtt gtgtagaaat gacagattta aaagcgccct tgcagcagtg tgcagaagat   1020
cgccagatct gcccttccct ggccgggttc cagtttacac agtgggacag tgaaacacat   1080
aatgagtctg tgtcggccct ggtagacaag tttaagaaga atgaccaggt atttgacatc   1140
aatgctgaag ttgacgagag tgactgtgga gacttccccg atgggtccct gggggatgac   1200
tttgatgcca acgatgaacc tgaccacacc gcagttgggg atcatgaaga gttcaggagc   1260
tggaaggagc cctgccaggt tcagagctgc caggaagaaa tgatttccct tggggatgga   1320
```

gacatcagga ccatgtgccc ccttctgtct atgaaacctg gagaatattc ttatttcagt 1380
cctcggacca tgtcgatgtg ggctggcccg gatcactggc gctttaggcc tcgacgcaaa 1440
caagatgctc cttcccaatc agaaaacaaa aagaagagta caaaaaaaga ttttgaaatt 1500
gactttgaag atgatattga ctttgatgta tattttagaa aaacaaaggc tgctactatt 1560
ctgaccaagt ccactttgga gaaccagaat tggagagcta ccacccttcc tacagatttc 1620
aactacaatg ttgacactct ggtccagctt cacctcaaac caggcaccag gttacttaag 1680
atggcccagg gccatagggt agagactgag cattatgaag aaattgaaga ctatgattac 1740
aacaacccta acgacacctc caacttttgc cctggattac aggctgctga cagtgatgat 1800
gaagatttgg atgacttatt tgtgggacct gttgggaact ctgacctctc accttatcct 1860
tgccatccac ctaagacagc acaacagaat ggtgacactc cagaagccca aggattagac 1920
atcacaacat atggggagtc aaacttggta gctgagcctc agaaggtaaa taaaattgaa 1980
attcactatg ccaagactgc caaaaagatg gacatgaaga aactgaagca gagcatgtgg 2040
agtctgctga cagcgctctc cggaaaggag gcagatgcag aggcaaacca cagggaagct 2100
ggaaaagaag cggccctggc agaagtggct gacgagaaga tgcttagcgg gctcacgaag 2160
gacctgcaga ggagcctgcc ccctgtcatg gctcagaacc tctccatacc tctggctttt 2220
gcctgtctcc tacatttagc caatgaaaag aatctaaaac tggaaggaac agaggacctc 2280
tctgatgttc ttgtgaggca aggagattga gttcactatg gagaagtcag cagcaggagg 2340
cccatccctt actcagttgc cgggacatcc ccagtctcgg gggaagaaga tgccatgggc 2400
ttatacccag gctgtagcca actaccaacg tgcctgtttg tttgttgctc tttccttctc 2460
tccatcatag tctgggtgcc agcgccctga agctccgtgc tcaactgatt aaactttact 2520
gccctatggt gaccatctag gagaggggag ggcagagggg gtgagggtac tattctggat 2580
tgagaaaacc tatatccatt ctttatatca atgtatagtt ttagtctcct aaattgatct 2640
gttattttcc aaactattct cttgtagaaa attttccagt gggcacttaa tggtgccctt 2700
gaagaacttc ctaatccatg tacataaaat acatcatatg tacacttata aatgtatata 2760
gaatgctcaa aaataaaatt cttaataata gaactggcaa aatatttgag tgtccactag 2820
atgagtatca gacctagtcc ttacccttag ggggatgcag tcctggttgt tatccaggat 2880
acacacctgt cagtataagg cagaagatgc ctaagggcca agatggtttg cctcggagga 2940
gaatggaaga gagagattgc tgactggaca ttcagatgca agactgggtc ctgcttaaat 3000
cccaggattc tgctggaggg agctgatagt gatacttgtc ccttctgtac attgcttcat 3060
gtagccttct cagcatccct aggagaaact tactattgtg actctcatgt tggaggagga 3120
aacggacacc caaggtagag gaacttgcaa aagggcagcc ggcaaactgt caggggtggc 3180
ctgagcctgg caatctgcct ccagagtctg ctctcggcca ttgtgctatg tgctacctgg 3240

```
ataggtcata caggctcagc agtgggtgga gagcagtgct cagatttgtc catctccaca   3300
gaatgcagca cacacacaaa tgtacaagtt cttcccctaa cctcagagga ataggggaat   3360
taactttgct tgcaatttgg aacaatatta tagatgttga tccaagtagt tctgttactg   3420
gctggtcctg gatctctgcc agaacacccg tcatcattga ctggctaaat agagatcttg   3480
gatataggcc agaagcagtg aagtatataa ttggaaattg ctcctgataa taacttcctt   3540
cttagccaaa aaccacacaa aacaaaaata atcccctccc cacaggaata tgctttccaa   3600
attgtgtcca aaacattacc tgctctgtta tattgagaag gttagagact tcagagcatg   3660
cttagaaaaa gcagtggtgc cacaggtgag actccacact ctgtcttgct ggggctgaag   3720
cctccatcac tttcccaggc caggttagtg ctgggcttct tgctttcctt ctattcctga   3780
gagtagaact ggctaagccc attccttccc tcagtcagcc ccacttctct atagtgggtt   3840
ctgggggtgg ggggctgaat taccagtaaa actagaaaga ttgggaccaa gtgcagtggc   3900
ccacacctgt aaatcctagc gctttgaaag gaagaggcag gaggattgct tgaagtcagg   3960
agttcaagac cagcctgggc aaaatagaac cccatcttta aaaaaaaagt ttaaaaatta   4020
gccaggtacg gaggtgtgtg cctgtaatcc cagctactca gaaggctgag gtgggataat   4080
cacttgagcc caggagtttg aggctgcagt gagctgtgat cacactactg cattccagcc   4140
aggacaacag agtgagatcc tatctcttaa acaaaaaaaa aaactggcga gttcaatacc   4200
aacttctaca atgaaatccc cttcccccca caaccctgct tctcctaagt ttccctcatt   4260
acatggttgc tgtgggctat gtgtgctgtg gtctgaatgt ttgtgtctaa aattcacatg   4320
ttggtattaa gagatagggc ctttgggagg tgattaggtt atgagggcag atccctcgtg   4380
aatgggatta gtgctcttat aaaagaggcc tgaggaagct tgttcgttcc tcttgccctt   4440
ctgccatgta aggatgcaat gagaaggcac catctgtgag caaggagccc ctcac        4495
```

```
<210>  20
<211>  2554
<212>  DNA
<213>  Homo sapiens

<400>  20
acaaggcagc ctcgctcgag cgcaggccaa tcggctttct agctagaggg tttaactcct     60
atttaaaaag aagaaccttt gaattctaac ggctgagctc ttggaagact tgggtccttg    120
ggtcgcaggt gggagccgac gggtgggtag accgtggggg atatctcagt ggcggacgag    180
gacggcgggg acaaggggcg gctggtcgga gtggcggagc gtcaagtccc ctgtcggttc    240
ctccgtccct gagtgtcctt ggcgctgcct tgtgcccgcc cagcgccttt gcatccgctc    300
ctgggcaccg aggcgccctg taggatactg cttgttactt attacagcta gagggtctca    360
ctccattgcc caggccagag tgcggggata tttgataaga aacttcagtg aaggccgggc    420
```

gcggtggctc atgcccgtaa tcccagcatt ttcggaggcc gaggctggag tgcaatggtg 480
tgatctcagc tcactgcaac ctctgcttcc tgggtttaag tgattctcct gcctcagcct 540
cccgagtagc tgggattaca ggcatcatgg accgatctaa agaaaactgc atttcaggac 600
ctgttaaggc tacagctcca gttggaggtc caaaacgtgt tctcgtgact cagcaatttc 660
cttgtcagaa tccattacct gtaaatagtg gccaggctca gcgggtcttg tgtccttcaa 720
attcttccca gcgcattcct ttgcaagcac aaaagcttgt ctccagtcac aagccggttc 780
agaatcagaa gcagaagcaa ttgcaggcaa ccagtgtacc tcatcctgtc tccaggccac 840
tgaataacac ccaaaagagc aagcagcccc tgccatcggc acctgaaaat aatcctgagg 900
aggaactggc atcaaaacag aaaaatgaag aatcaaaaaa gaggcagtgg gctttggaag 960
actttgaaat tggtcgccct ctgggtaaag gaaagtttgg taatgtttat ttggcaagag 1020
aaaagcaaag caagtttatt ctggctctta aagtgttatt taaagctcag ctggagaaag 1080
ccggagtgga gcatcagctc agaagagaag tagaaataca gtcccacctt cggcatccta 1140
atattcttag actgtatggt tatttccatg atgctaccag agtctaccta attctggaat 1200
atgcaccact tggaacagtt tatagagaac ttcagaaact ttcaaagttt gatgagcaga 1260
gaactgctac ttatataaca gaattggcaa atgccctgtc ttactgtcat tcgaagagag 1320
ttattcatag agacattaag ccagagaact tacttcttgg atcagctgga gagcttaaaa 1380
ttgcagattt tgggtggtca gtacatgctc catcttccag gaggaccact ctctgtggca 1440
ccctggacta cctgcccccct gaaatgattg aaggtcggat gcatgatgag aaggtggatc 1500
tctggagcct tggagttctt tgctatgaat ttttagttgg gaagcctcct tttgaggcaa 1560
acacatacca agagacctac aaaagaatat cacgggttga attcacattc cctgactttg 1620
taacagaggg agccagggac ctcatttcaa gactgttgaa gcataatccc agccagaggc 1680
caatgctcag agaagtactt gaacaccccct ggatcacagc aaattcatca aaaccatcaa 1740
attgccaaaa caaagaatca gctagcaaac agtcttagga atcgtgcagg gggagaaatc 1800
cttgagccag ggctgccata taacctgaca ggaacatgct actgaagttt attttaccat 1860
tgactgctgc cctcaatcta gaacgctaca caagaaatat ttgttttact cagcaggtgt 1920
gccttaacct ccctattcag aaagctccac atcaataaac atgacactct gaagtgaaag 1980
tagccacgag aattgtgcta cttatactgg ttcataatct ggaggcaagg ttcgactgca 2040
gccgccccgt cagcctgtgc taggcatggt gtcttcacag gaggcaaatc cagagcctgg 2100
ctgtggggaa agtgaccact ctgccctgac cccgatcagt taaggagctg tgcaataacc 2160
ttcctagtac ctgagtgagt gtgtaactta ttgggttggc gaagcctggt aaagctgttg 2220
gaatgagtat gtgattcttt ttaagtatga aaataaagat atatgtacag acttgtattt 2280

```
tttctctggt ggcattcctt taggaatgct gtgtgtctgt ccggcacccc ggtaggcctg   2340
attgggtttc tagtcctcct taaccactta tctcccatat gagagtgtga aaaataggaa   2400
cacgtgctct acctccattt agggatttgc ttgggataca gaagaggcca tgtgtctcag   2460
agctgttaag ggcttatttt tttaaaacat tggagtcata gcatgtgtgt aaactttaaa   2520
tatgcaaata aataagtatc tatgtctaaa aaaa                               2554
```